# EUROPEAN PATENT SPECIFICATION

(11) **EP 1 748 062 B1**
(45) Date of publication and mention of the grant of the patent: **13.06.2012**
(21) Application number: 05741287.6
(22) Date of filing: 20.05.2005
(51) Int. Cl.: C12N 1/15, C12N 1/21, C12P 7/46, C12N 15/53, C12N 15/54, C12N 15/55, C12R 1/15

(54) **SUCCINIC ACID-PRODUCING BACTERIUM AND PROCESS FOR PRODUCING SUCCINIC ACID**
BERNSTEINSÄURE PRODUZIERENDES BAKTERIUM UND VERFAHREN ZUR HERSTELLUNG VON BERNSTEINSÄURE
BACTERIE PRODUISANT DE L'ACIDE SUCCINIQUE ET PROCEDE SERVANT A PRODUIRE DE L'ACIDE SUCCINIQUE

(30) Priority: 20.05.2004 JP 2004150672
(43) Date of publication of application: 31.01.2007
(73) Proprietor: Ajinomoto Co., Inc., Tokyo 104-8315 (JP)
(72) Inventor: FUKUI, Keita, c/o AJINOMOTO CO., INC., Kanagawa 2108681 (JP); NAKAMURA, Jun, c/o AJINOMOTO CO., INC., Kanagawa 2108681 (JP); AKIYAMA, Kayo, c/o AJINOMOTO CO., INC., Kanagawa 2108681 (JP); KOJIMA, Hiroyuki, c/o AJINOMOTO CO., INC., Kanagawa 2108681 (JP)
(74) Representative: Vossius & Partner
(86) International application number: PCT/JP2005/009233
(87) International publication number: WO 2005/113745

(56) References cited:
- WO-A1-02/29020
- WO-A1-99/06532
- JP-A- 11 113 588
- JP-A- 11 206 385
- JP-A- 2001 161 386
- JP-A- 2002 291 477
- JP-A- 2002 511 250
- LIN HENRY ET AL: "Genetic reconstruction of the aerobic central metabolism in Escherichia coli for the absolute aerobic production of succinate" BIOTECHNOLOGY AND BIOENGINEERING, vol. 89, no. 2, 20 January 2005 (2005-01-20), pages 148-156, XP009127665 ISSN: 0006-3592
- LIN H ET AL: "Metabolic engineering of aerobic succinate production systems in Escherichia coli to improve process productivity and achieve the maximum theoretical succinate yield" METABOLIC ENGINEERING, ACADEMIC PRESS, US, vol. 7, no. 2, 1 March 2005 (2005-03-01), pages 116-127, XP004801711 ISSN: 1096-7176
- SANCHEZ A M ET AL: "Novel pathway engineering design of the anaerobic central metabolic pathway in Escherichia coli to increase succinate yield and productivity" METABOLIC ENGINEERING, ACADEMIC PRESS, US, vol. 7, no. 3, 1 May 2005 (2005-05-01), pages 229-239, XP004879518 ISSN: 1096-7176
- SHOHEI OKINO ET AL: "Production of organic acids by Corynebacterium glutamicum under oxygen deprivation" APPLIED MICROBIOLOGY AND BIOTECHNOLOGY, SPRINGER, BERLIN, DE, vol. 68, no. 4, 1 September 2005 (2005-09-01), pages 475-480, XP019331935 ISSN: 1432-0614
- INUI MASAYUKI ET AL: "Metabolic analysis of Corynebacterium glutamicum during lactate and succinate productions under oxygen deprivation conditions" JOURNAL OF MOLECULAR MICROBIOLOGY AND BIOTECHNOLOGY, HORIZON SCIENTIFIC PRESS, WYMONDHAM, GB, vol. 7, no. 4, 2004, pages 182-196, XP009070835 ISSN: 1464-1801
- KALINOWSKI J ET AL: 'The complete Corynebacterium glutamicum ATCC 13032 genome sequence and its impact on the production of L-aspartate-derived amino acids and vitamins.' J BIOTECHNOL. vol. 104, no. 1-3, 2003, pages 5 - 25, XP001184752
- DIETER J ET AL: 'Cloning, sequence analysis, expression and inactivation of the Corynebacterium glutamicum pta-ack operon encoding phosphotransacetylase and acetate kinase.' MICROBIOLOGY. vol. 145, 1999, pages 503 - 513, XP002211213
- BOTT M ET AL: "The respiratory chain of Corynebacterium glutamicum", JOURNAL OF BIOTECHNOLOGY, ELSEVIER SCIENCE PUBLISHERS, AMSTERDAM, NL, vol. 104, no. 1-3, 4 September 2003 (2003-09-04), pages 129-153, XP002288114, ISSN: 0168-1656, DOI: DOI:10.1016/S0168-1656(03)00144-5

## Description

### Technical Field

The present invention relates to a method for producing succinic acid as defined in the claims and a coryneform bacterium having a succinic acid-producing ability as defined in the claims.

### Background Art

For production of non-amino organic acids including succinic acid by fermentation, usually, anaerobic bacteria such as those belonging to the genus *Anaerobiospirillum* or *Actinobacillus* are used (Patent Document I or 2, and Non-Patent Document 1). The use of anaerobic bacteria makes the yield of products high, while such bacteria require many nutrients for proliferation and therefore, there is a need for adding a large amount of organic nitrogen sources such as corn steep liquor (CSL) to a medium. The addition of abundant amounts of organic nitrogen sources not only leads to an increase in cost of the medium but also leads to an increase in cost of purification for isolating the product, which is uneconomical.

Furthermore, a method, which comprises culturing aerobic bacteria such as coryneform bacteria under an aerobic condition to proliferate bacterial cells and then collecting and washing the cells to use them as resting bacteria to produce succinic acid without oxygen aeration, has been known (Patent Document 3 and 4). This method is economical because the bacterial cells can grow sufficiently in a simple medium, into which a small amount of organic nitrogen is added for proliferation of bacterial cells, but this method is still to be improved in terms of the amount of generated succinic acid, the concentration thereof, and the production rate thereof per bacterial cells as well as simplification of production process, and the like.

Furthermore, when aerobic bacteria such as coryneform bacteria are cultured under oxygen-limited conditions, organic acids other than a desired substance such as lactic acid and acetic acid are excessively accumulated as by-products, resulting in suppressed growth of bacterial cells and significantly decreased productivity in fermentation. In addition, excessive amounts of counterions to neutralize the organic acids generated as by-products are required, thereby resulting in being uneconomical. To solve such problems, reduction in lactate generated as a by-product has been performed by using a coryneform bacterium having a reduced lactate dehydrogenase activity (Patent Document 5).

### Background Art

However, even if the above-mentioned coryneform bacterium having decreased lactate dehydrogenase activity is used, a large amount of acetic acid is generated as a by-product. As means for solving the problem of reducing acetic acid in a culture medium, there have been known a method of enhancing expression of an acetic acid assimilating gene (aceP) in a bacterium belonging to the genus *Escherichia* (Patent Document 6), a method of enhancing expression of a gene encoding ACE protein in a bacterium belonging to the genus *Escherichia* (Patent Document 7), and the like. Those methods are intended to reduce generation of acetic acid as a by-product by actively assimilating acetic acid released into a culture medium. Meanwhile, as methods of suppressing generation of acetic acid as a by-product by suppressing biosynthesis of acetic acid in a bacterium belonging to the genus *Escherichia,* there is known a method of producing succinic acid using *Escherichia coli* in which phosphoacetyltransferase and lactate dehydrogenase are deficient (patent Document 8).

As enzymes responsible for assimilation of acetic acid in a coryneform bacterium, there have been reported acetate kinase and phosphotransacetylase (Non-Patent Document 2). On the other hand, it is assumed that not only the above-mentioned enzymes but also a plurality of enzymes including pyruvate oxidase (Patent Document 9), acylphosphatase, aldehyde dehydrogenase and acetyl-CoA hydrolase are responsible for generation of acetic acid, but a specific enzyme that contributes to synthesis of acetic acid has not been clarified. Therefore, there has not been known a method of producing succinic acid using a strain of a coryneform bacterium having decreased acetic acid biosynthetic enzyme activity.

Pyruvate oxidase is an enzyme which produces acetic acid from pyruvic acid and water (EC 1.2.2.2), and there have been known a method of producing an L-amino acid using enterobacteria in which pyruvate oxidase is deficient (Patent Document 10), a method of producing D-pantothenic acid using enterobacteria in which pyruvate oxidase is deficient (Patent Document 11), and a method of producing D-pantothenic acid using a coryneform bacterium in which pyruvate oxidase is deficient (Patent Document 12).
A gene sequence of pyruvate oxidase of *Corynebacterium glutamicum* has been identified, and there has been known a method of producing an L-amino acid using a coryneform bacterium which is modified so that the expression of a pyruvate oxidase gene is decreased (Patent Document 13). However, contribution of pyruvate oxidase to succinic acid-biosynthetic system in a coryneform bacterium has been unknown, and no report has been provided on expression analysis of pyruvate oxidase gene under anaerobic conditions.
Patent Document 1: US Patent No. 5,143,833
Patent Document 2: US Patent No. 5,504,004
Patent Document 3: JP11-113588A
Patent Document 4: JP11-196888A
Patent Document 5: JP11-206385A
Patent Document 6: JP06-14781A
Patent Document 7: JP07-67683A
Patent Document 8: WO 99/06532
Patent Document 9: EP 1096013 A
Patent Document 10: WO 02/36797
Patent Document 11: WO 02/072855
Patent Document 12: WO 02/29020
Patent Document 13: EP1108790A

Non-Patent Document 1: International Journal of Systematic Bacteriology, vol. 49, p 207-216, 1999
Non-Patent Document 2: Microbiology. 1999, Feb; 145(Pt 2): 503-13

### DISCLOSURE OF THE INVENTION

An object of the present invention is to provide a coryneform bacterium capable of efficiently producing succinic acid as defined in the claims.

The inventors of the present invention have intensively studied to solve the aforementioned problems, and as a result, they found that generation of acetic acid as a by-product is reduced and a succinic acid is efficiently produced in a coryneform bacterium by decreasing a pyruvate oxidase activity, thereby accomplished the present invention.

That is, the present invention is as follows.
(1) A coryneform bacterium having a succinic acid-producing ability, wherein said bacterium has been modified so that an activity of pyruvate oxidase is decreased and wherein said bacterium has one or more modification selected from the group consisting of:
   (a) activities of one or more of phosphotransacetylase, acetate kinase and acetyl CoA Hydrolase is decreased;
   (b) an activity of lactate dehydrogenase is decreased; and
   (c) an activity of pyruvate carboxylase is increased.
(2) The coryneform bacterium according to (1), wherein the pyruvate oxidase is a protein as described in the following (A) or (B):
   (A) a protein having an amino acid sequence of SEQ ID NO: 49; or
   (B) a protein having an amino acid sequence of SEQ ID NO: 49 including substitution, deletion, insertion, or addition of one or several amino acids, and having a pyruvate oxidase activity.
(3) The coryneform bacterium according to (1) or (2), wherein the pyruvate oxidase activity is decreased by disruption of a pyruvate oxidase gene on a chromosome.
(4) The coryneform bacterium according to (3), wherein the pyruvate oxidase gene is a DNA as described in the following (a) or (b):
   (a) a DNA comprising the nucleotide sequence of nucleotide numbers 996-2732 in SEQ ID NO: 48; or
   (b) a DNA that hybridizes with the nucleotide sequence of nucleotide numbers 996-2732 in SEQ ID NO: 48 or a probe that can be prepared from the nucleotide sequence under stringent conditions, and encodes a protein having a pyruvate oxidase activity.
(5) A method for producing succinic acid, comprising:
   allowing a coryneform bacterium having a succinic acid-producing ability or immunobilized cells or disrupted cells thereof to act on an organic raw material in a reaction liquid containing carbonate ion,
      bicarbonate ion or carbon dioxide to produce and accumulate succinic acid in the reaction liquid; and
   collecting succinic acid from the reaction liquid, wherein said bacterium has been modified so that an activity of pyruvate oxidase is decreased and the generation of acetic acid as a by product is reduced.
(6) The method according to (5), wherein the pyruvate oxidase is a protein as described in the following (A) or (B):
   (A) a protein having an amino acid sequence of SEQ ID NO: 49; or
   (B) a protein having an amino acid sequence of SEQ ID NO: 49 including substitution, deletion, insertion, or addition of one or several amino acids, and having a pyruvate oxidase activity.
(7) The method according to (5) or (6), wherein the pyruvate oxidase activity is decreased by disruption of a pyruvate oxidase gene on a chromosome.
(8) The method according to (7), wherein the pyruvate oxidase gene is a DNA as described in the following (a) or (b):
   (a) a DNA comprising the nucleotide sequence of nucleotide numbers 996-2732 in SEQ ID NO: 48; or
   (b) a DNA that hybridizes with the nucleotide sequence of nucleotide numbers 996-2732 in SEQ ID NO: 48 or a probe that can be prepared from the nucleotide sequence under stringent conditions, and encodes a protein having a pyruvate oxidase activity.
(9) The method according to any one of (5) to (8), wherein said bacterium has been further modified so that activities of one or more of phosphotransacetylase, acetate kinase and acetyl-CoA Hydrolase is decreased.
(10) The method according to any one of (5) to (9), wherein said bacterium has been further modified so that an activity of lactate dehydrogenase is decreased.
(11) The method according to any one of claims (5) to (10), wherein said bacterium has been further modified so that an activity of pyruvate carboxylase is increased.
(12) The production method according to any one of (5) to (11), wherein the bacterium or the immobilized cells or the disrupted cells thereof is allowed to act on the organic raw material under anaerobic conditions.
(13) A method for producing a succinic acid-containing polymer, comprising the steps of producing succinic acid by the method according to any one of (5) or (12) and polymerizing the obtained succinic acid.

### BRIEF DESCRIPTION OF THE DRAWINGS

Fig. 1 shows the procedures for constructing plasmid pBS3.
Fig. 2 shows the procedures for constructing plasmid pBS4S.
Fig. 3 shows the procedures for constructing plasmid pΔldh56-1.
Fig. 4 shows the procedures for constructing plasmid pBS4S:: ΔpoxB.
Fig. 5 is a graph showing ratio of acetic acid as a byproduct in the poxB-disrupted strain and the control strain.
Fig. 6 shows the procedures for constructing plasmid pBS5T::Δack.
Fig. 7 shows the procedures for constructing plasmid pBS5T::Δpta-ack.
Fig. 8 shows the procedures for constructing plasmid pBS5T::ΔpoxB.
Fig. 9 shows the procedures for constructing plasmid pBS4S::Δach.
Fig. 10 shows the procedures for constructing plasmid pBS5T:Δacp.

### DESCRIPTION OF PREFERRED EMBODIMENTS

Hereinafter, embodiments of the present invention will be described in detail.

### <1> Coryneform bacterium to be used in the present invention

In the present invention, the term "coryneform bacterium" includes a bacterium which had been classified as the genus *Brevibacterium* but now classified as the genus *Corynebacterium* (Int. J. Syst. Bacteriol., 41, 255(1981)), and it also includes a bacterium belonging to the genus *Brevibacterium,* which is very closely related to *Corynebacterium.* Examples of such coryneform bacteria include the followings.
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium*
*Corynebacterium melassecola*
*Corynebacterium thermoaminogenes*
*Corynebacterium herculis*
*Brevibacterium divaricatum*
*Brevibacterium flavum*
*Brevibacterium immariophilum*
*Brevibacterium lactofermentum*
*Brevibacterium roseum*
*Brevibacterium saccharolyticum*
*Brevibacterium thiogenitalis*
*Corynebacterium ammoniagenes*
*Brevibacterium album*
*Brevibacterium selinum*
*Microbacterium ammoniaphilum*

In the present invention, the term "succinic acid-producing ability" means an ability to accumulate succinic acid in a medium when the coryneform bacterium of the present invention is cultured. The succinic acid-producing ability may be a feature inherent to a coryneform bacterium or a feature provided by breeding.

To provide the succinic acid-producing ability by breeding, there may be applied methods that have been employed in breeding of coryneform bacteria, which include acquisition of metabolic regulation mutant strains, creation of a recombinant strain having an enhanced biosynthetic enzyme for a desired substance, and the like (Amino Acid Fermentation, Japan Scientific Societies Press, the first edition published on May 30, 1986, p77-100). In these methods, one or two or three or more features such as metabolic regulation mutations and enhancement of biosynthetic enzymes for a desired substance may be provided. Imparting properties such as metabolic regulation mutations and enhancement of biosynthetic enzymes may be combined. An example of succinic acid-producing enzyme includes pyruvate carboxylase as described below.

Particularly preferable specific examples of a coryneform bacterium having a succinic acid-producing ability include *Brevibacterium flavum* MJ233Δldh strain having decreased lactate dehydrogenase activity (JP11-206385A), *Brevibacterium flavum.* MJ233/pPCPYC strain having enhanced activity of pyruvate carboxylase or phosphoenol pyruvate carboxylase (WO 01/27258 and JP11-196887A), *Brevibacterium flavum* MJ-233 (FERM PB-1497), *Brevibacterium flavum* MJ-233 AB-41 (FERM BP-1498), *Brevibacterium ammoniagenes* ATCC6872, *Corynebacterium glutamicum* ATCC31831, and *Brevibacterium lactofermentum* ATCC13869*.* Since *Brevibacterium flavum* may be currently classified as *Corynebacterium glutamicum* (Lielbl, W., Ehrmann, M., Ludwig, W. and Schleifer, K. H., International Journal of Systematic Bacteriology, 1991, vol. 41, p255-260), the aforementioned *Brevibacterium flavum* MJ-233 strain and its mutant MJ-233 AB-41 strain, are defined as the same strains as *Corynebacterium glutamicum* MJ-233 strain and *Corynebacterium glutamicum* MJ-233 AB-41 strain, respectively.

### <2> Construction of the coryneform bacterium of the present invention

The coryneform bacterium of the present invention is a coryneform bacterium that has the above-mentioned succinic acid-producing ability and modified so that pyruvate oxidase activity is decreased and wherein said bacterium further has one or more modifications selected from the group consisting of: (a) activities of one or more of phosphotransacetylase, acetate, kinase and acetyl-CoA hydrolase is decreased; (b) an activity of lactate dehydrogenase is decreased; and (c) an activity of pyruvate carboxylase is increased.
In breeding of the coryneform bacterium of the present invention, there is no preference as to which of the provision of a succinic acid-producing ability and the modification for decreasing pyruvate oxidase (EC 1.2.2.2) activity is performed first.

The term "pyruvate oxidase activity" refers to an activity to catalyze a reaction to generate acetic acid from pyruvic acid and water. The phrase "modified so that pyruvate oxidase activity is decreased" means that pyruvate oxidase activity is decreased as compared to a specific activity of an unmodified strain, for example, a wild-type coryneform bacterium. The pyruvate oxidase activity is preferably decreased to 50% or less per bacterial cell, more preferably 30% or less, further more preferably 10% or less per bacterial cell as compared to an unmodified strain. Herein, examples of a wild-type coryneform bacterium to be used as a control include *Brevibacterium lactofermentum* ATCC13869 (wild-type strain) and *Brevibacterium lactofermentum* Δldh strain (unmodified strain). The pyruvate oxidase activity can be determined according to the method of Chang Y. et al. (Chang Y. and Cronan J. E. JR, J.Bacteriol. 151,1279-1289(1982)).

Examples of pyruvate oxidase having the above-mentioned activity include a protein having an amino acid sequence of SEQ ID NO: 49. In addition, as long as the protein has a pyruvate oxidase activity, it may be a protein having an amino acid sequence of SEQ ID NO: 49 including substitution, deletion, insertion, or addition of one or several amino acids. Here, for example, the term "several" means 2 to 20, preferably 2 to 10, or more preferably 2 to 5.

The phrase "modified so that pyruvate oxidase activity is decreased" includes decrease in the number of molecules of pyruvate oxidase per cell, decrease in the pyruvate oxidase activity per molecule and the like. Specifically, it is achieved by disrupting a gene encoding pyruvate oxidase on a chromosome, modification of an expression regulatory sequence such as promoter, Shine-Dalgarno (SD) sequence, or the like. Examples of the pyruvate oxidase gene on a chromosome include a DNA having the nucleotide sequence of nucleotide numbers 996-2732 in SEQ ID NO: 48. Also, it may be a DNA that hybridizes with a nucleotide sequence of nucleotide numbers 996-2732 in SEQ ID NO: 48 or a probe that can be prepared from the nucleotide sequence under stringent conditions as long as it encodes a protein having the pyruvate oxidase activity. The term "stringent conditions" refers to conditions under which a so-called specific hybrid is formed and non-specific hybrid is not formed. It is difficult to clearly define the conditions by numeric value, but examples thereof include conditions that comprises washing once, preferably twice or three times at salt concentrations corresponding to 1 x SSC, 0.1% SDS, preferably 0.1 x SSC, 0.1% SDS at 60°C.

The pyruvate oxidase gene (hereinafter, referred to as poxB gene) can be obtained by, for example, cloning performed by synthesizing synthetic oligonucleotides based on a sequence of *Corynebacterium glutamicum* registered in GenBank (a complementary strand of 2776766-2778505 of GenBank Accession No. NC_003450), and performing PCR using a chromosome of *Corynebacterium glutamicum* as a template. In addition, there may also be used a sequence of a coryneform bacterium such as *Brevibacterium lactofermentum* having a nucleotide sequence determined by the recent genome project. Chromosomal DNA can be prepared from a bacterium as a DNA donor by, for example, the method of Saito and Miura (H. Saito and K. Miura, Biochem. Biophys. Acta, 72, 619 (1963), Experimental Manual for Biotechnology, edited by. The Society for Biotechnology, Japan, p97-98, Baifukan Co., Ltd., 1992) or the like.

The poxB gene thus prepared or a part thereof can be used for gene disruption. A gene to be used for gene disruption only needs to have homology enough to cause homologous recombination with a poxB gene to be disrupted on a chromosome of a coryneform bacterium (e.g. a gene having the nucleotide sequence of nucleotide numbers 996-2732 in SEQ ID NO: 48), so such a homologous gene may also be used. Here, the homology enough to cause homologous recombination is preferably not less than 70%, more preferably not less than 80%, further more preferably not less than 90%, particularly preferably not less than 95%. Further, DNAs capable of hybridizing with the above-mentioned gene under stringent conditions can cause homologous recombination.
The term "stringent conditions" refers to conditions under which a so-called specific hybrid is formed and non-specific hybrid is not formed. It is difficult to clearly define the conditions by numeric value, but examples thereof include, conditions that comprises washing once, preferably twice or three times at salt concentrations corresponding to 1 x SSC, 0.1% SDS, preferably 0.1 x SSC, 0.1% SDS at 60°C.

For example, by using the above-mentioned gene, a deleted-form of poxB gene, which is modified so as not to produce pyruvate oxidase that normally functions by deleting a partial sequence of the poxB gene, is prepared, and a coryneform bacterium is transformed with a DNA including the gene to cause recombination between the deleted-form gene and a gene on a chromosome, to thereby disrupt the poxB gene on a chromosome. Such a gene disruption by gene substitution using homologous recombination has already been established, and examples thereof include a method using a linear DNA and a method using a plasmid containing a temperature-sensitive replication origin (US Patent No. 6,303,383 or JP05-007491 A). Further, the above-mentioned gene disruption by gene substitution using homologous recombination may also be performed using a non-replicating plasmid in a host.

For example, a poxB gene on a chromosome of a host can be substituted by a deleted-form of poxB gene in accordance with the following procedures. First, a plasmid for recombination is prepared by inserting a temperature-sensitive replication origin, deleted-form of poxB gene, sacB gene encoding levansucrase and marker gene resistant to an antibiotic such as chloramphenicol.

Here, sacB gene encoding levansucrase is a gene which is used for efficiently selecting a strain in which a vector portion has been excised from a chromosome (Schafer, A. et al.,Gene 145 (1994) 69-73). That is, when levansucrase is expressed in a coryneform bacterium, levan generated by assimilation of sucrose acts lethally on the bacterium, so the bacterium cannot grow. Therefore, if a bacterial strain in which a vector carrying levansucrase remains on a chromosome is cultured on a sucrose-containing plate, it cannot grow. As a result, only a bacterial strain from which the vector has been excised can be selected on the sucrose-containing plate.

Genes each having the following sequences can be used as a sacB gene or homologous gene thereof.
*Bacillus subtilis:* sacB GenBank Accession Number X02730 (SEQ ID NO: 41)
*Bacillus amyloliquefaciens:* sacB GenBank Accession Number X52988
*Zymomonas mobilis:* sacB GenBank Accession Number L33402
*Bacillus stearothermophilus:* surB GenBank Accession Number U34874
*Lactobacillus sanfranciscensis:* frfA GenBank Accession Number AJ508391
*Acetobacter xylinus:* IsxA GenBank Accession Number AB034152
*Gluconacetobacter diazotrophicus:* IsdA GenBank Accession Number LA1732

A coryneform bacterium is transformed with the above-mentioned recombinant plasmid. The transformation can be performed in accordance with a transformation method which has been previously reported. Examples of the method include, a method of increasing permeability of a DNA by treating cells of a recipient bacterium with calcium chloride as reported for *Escherichia coli* K-12 (Mandel, M. and Higa, A., J. Mol. Biol., 53, 159 (1970)) and, a method of preparing competent cells using proliferating cells for introduction of DNA as reported for *Bacillus subtilis* (Duncan, C.H., Wilson, G.A and Young, F.E, Gene, 1, 153 (1977)). Alternatively, as reported for *Bacillus subtilis,* actinomycetes and yeasts, a method of introducing a recombinant DNA into cells of a DNA recipient bacterium (Chang. S. and Choen, S.N., Molec. Gen. Genet., 168, 111 (1979); Bibb, M.J., Ward, J.M., and Hopwood, O.A., Nature, 274, 398 (1978); Hinnen, A., Hicks, J.B. and Fink, G.R., Proc. Natl. Acad. Sci. USA, 75 1929 (1978)) may also be applied. In addition, a coryneform bacterium may be transformed by the electric pulse method (Sugimoto et al., JP02-207791A).

Examples of a temperature-sensitive plasmid for a coryneform bacterium include p48K and pSFKT2 (JP2000-262288A), and pHSC4 (France Patent No. 2667875 (1992) and JP05-7491A). These plasmids are autonomously replicable in a coryneform bacterium at least at 25°C, but they are not autonomously replicable at 37°C. *Escherichia coli* AJ12571 having pHSC4 has been deposited with an Accession No. FERM P-11763 at National Institute of Bioscience and Human-Technology, Agency of industrial Science and Technology, Ministry of International Trade and Industry (currently, International Patent Organism Depositary, National Institute of Advanced Industrial Science and Technology) (Central 6, 1-1-1 Higashi, Tsukuba, Ibaraki, 305-5466 Japan) on October 11, 1990, and then transferred to an international deposit under the provisions of Budapest Treaty on August 26, 1991 with an Accession No. FERM BP-3524.

A transformant obtained as described above is cultured at a temperature at which the temperature-sensitive replication origin does not function (25°C), to thereby obtain a strain into which the plasmid has been introduced. The plasmid-introduced strain is cultured at high temperature to excise the temperature-sensitive plasmid, and the bacterial strain is applied onto a plate containing an antibiotic. The temperature-sensitive plasmid cannot replicate at high temperature. Therefore, a bacterial strain from which the plasmid has been excised cannot grow on a plate containing an antibiotic, but a bacterial strain in which recombination has occurred between the poxB gene on the plasmid and the poxB gene on a chromosome appears at a very low frequency.

In the strain obtained by introducing the recombinant DNA into a chromosome as described above, recombination occurs with the poxB gene sequence that is originally present on a chromosome, and two fusion genes of the chromosomal poxB gene and the deleted-form of poxB gene are inserted into a chromosome so that other portions of the recombinant DNA (vector part, temperature-sensitive replication origin and antibiotic-resistance marker) are present between the fusion genes.

Then, in order to leave only the deleted-form of poxB gene on a chromosomal DNA, the gene is eliminated together with the vector portion (the temperature-sensitive replication origin and drug-resistance marker) from the chromosomal. This procedure causes a case where the normal poxB gene remains on the chromosomal DNA and the deleted-form of poxB gene is excised, or to the contrary, a case where the normal poxB gene is excised and the deleted-form of poxB gene remains on chromosomal DNA. In both cases, when culture is performed at a temperature that allows a temperature-sensitive replication origin to function, the cleaved DNA is kept in a cell as a plasmid. Next, when culture is performed at a temperature that does not allow a temperature-sensitive replication origin to function, the poxB gene on the plasmid is eliminated from the cell together with the plasmid. Then, a strain in which the deleted-form of poxB gene remains on the chromosome, is selected by PCR, Southern hybridization, or the like, to thereby yield a strain in which the poxB gene is disrupted.

In the case where a plasmid having no replicability in a coryneform bacterium is used instead of the above-mentioned temperature-sensitive plasmid, gene disruption can also be performed in a similar way. The plasmid having no replicability in a coryneform bacterium is preferably a plasmid having a replicability in *Escherichia coli,* and examples thereof include pHSG299 (Takara Bio Inc.) and pHSG399 (Takara Bio Inc.).

Meanwhile, examples of a method of decreasing an activity of pyruvate oxidase include not only the above-mentioned genetic engineering method but also a method comprising treating a coryneform bacterium with ultraviolet irradiation or with a mutagenesis agent to be generally used for mutation such as N-methyl-N'-nitro-N-nitrosoguanidine (NTG) and nitrous acid, and selecting a bacterial strain having decreased pyruvate oxidase activity.

In the present invention, it is more effective to use a bacterial strain modified so that a lactate dehydrogenase (hereinafter, referred to as LDH) activity is decreased in addition to the above-mentioned pyruvate oxidase activity. The lactate dehydrogenase activity means an activity to catalyze a reaction to generate lactic acid by reducing pyruvic acid using NADH as a coenzyme. The phrase "lactate dehydrogenase activity is decreased" means that LDH activity is decreased as compared to an LDH-unmodified strain. The LDH activity is decreased than an LDH-unmodified strain or a wild-type strain, and it is preferably decreased to 50% or less, more preferably 30% or less, particularly preferably 10% or less per bacterial cell. LDH activity may also be completely eliminated. The decreased LDH activity can be confirmed by determining the LDH activity by the method of L. Kanarek et al. (L. Kanarek and R.L. Hill, J. Biol. Chem. 239, 4202 (1964)). The coryneform bacterium of the present invention can be obtained by preparing a coryneform bacterium having decreased LDH activity and modifying it so that the pyruvate oxidase activity is decreased. However, there is no preference in the order for performing the modification to decrease the LDH activity and the modification to decrease the pyruvate oxidase activity. As an Idh gene, there may be used, for example, a gene having the sequence of SEQ ID NO: 43, and gene disruption may be performed in a similar manner as in the case of the above-mentioned poxB gene.

In the present invention, it is more effective to use a bacterial strain modified so that activity of any one or more of phosphotransacetylase (hereinafter, referred to as PTA), acetate kinase (hereinafter, referred to as ACK), acetyl-CoA hydrolase (ACH) is decreased, in addition to the decrease in pyruvate oxidase activity. It is further effective to use a bacterial strain modified so that activities of two or more of the enzymes are decreased, and is particularly effective to use a bacterial strain modified so that all of the activities are decreased. It is further effective to use a bacterial strain further deficient in acylphosphatase.

In the present invention, the phosphotransacetylase (PTA) activity means an activity to catalyze a reaction to generate acetyl phosphate by transferring phosphate to acetyl-CoA. The acetate kinase (ACK) activity means an activity to catalyze a reaction to generate acetic acid from acetyl phosphate and ADP. The acetyl-CoA hydrolase (ACH) activity means an activity to catalyze a reaction to generate acetic acid from acetyl-CoA and water. The acylphosphatase (ACP) activity means an activity to catalyze a reaction to generate phosphoric acid and acetic acid, or carboxylic acid and phosphoric acid from acetyl phosphate.

Decreasing these activities can be performed by disrupting genes encoding the above-mentioned enzymes, or by modifying expression regulatory sequences such as promoter and Shine-Dalgamo (SD) sequence of genes encoding the enzymes. The disruption of a gene can be performed in the same way as the above-mentioned method of disrupting poxB gene.

As genes encoding the enzymes, there may be used, for example, the following genes of *Corynebacterium glutamicum* registered in GenBank:
pta (phosphoacetyltransferase) gene: NCg12657 of GenBank Accession No. NC_003450 (a complementary strand of nucleotide numbers 2936506-2937891 of NC_003450) (the nucleotide numbers 956-1942 in SEQ ID NO: 45)
ack (acetate kinase) gene: NCg12656 of GenBank Accession No. NC_003450 (a complementary strand of nucleotide numbers 2935313-2936506 of NC_003450) (the nucleotide numbers 1945-3135 in SEQ ID NO: 45).
ach (acetyl-CoA hydrolase) gene: NCgl2480 of GenBank Accession No. NC_003450 (a complementary strand of nucleotide number 2729376-2730884 of NC_003450) (SEQ ID NO: 50)
acp (acylphosphatase) gene: Ncgl1987 of GENEBANK accession NO. NC_003450 (a complementary strand of nucleotide number 2183107-2183391 of NC_003450) (SEQ ID NO: 52)

The phrase "phosphotransacetylase (hereinafter, referred to as PTA) activity is decreased" means that PTA activity is decreased as compared to PTA-unmodified strain. The PTA activity is lower than PTA-unmodified strain or a wild-type strain, and it is preferably decreased to 50% or less, more preferably 30% or less, particularly preferably 10% or less per bacterial cell.. PTA activity may also be completely eliminated. The decreased PTA activity can be confirmed by determining the PTA activity by the method of Klotzsch et al. (Klotzsch H.R., Meth Enzymol. 12, 381-386 (1969)). A coryneform bacterium having decreased activities of POXB and PTA can be obtained by constructing a coryneform bacterium having decreased POXB activity and modifying it so that the PTA activity is decreased. However, there is no preference in the order for performing the modification to decrease PTA activity and the modification to decrease POXB activity.

The phrase "acetate kinase (hereinafter, referred to as ACK) activity is decreased" means that ACK activity is decreased as compared to a wild-type strain or ACK-unmodified strain. The ACK activity is lower than an ACK-unmodified strain or a wild-type strain, and it is preferably decreased to 50% or less, more preferably 30% or less, particularly preferably 10% or less per bacterial cell as compared to an ACK-unmodified strain. ACK activity may also be completely eliminated. The decreased ACK activity can be confirmed by determining the ACK activity by the method of Ramponi et al. (Ramponi G., Meth. Enzymol. 42, 409-426 (1975)). A coryneform bacterium having decreased activities of POXB and ACK can be obtained by constructing a coryneform bacterium having decreased POXB activity and modifying it so that the ACK activity is decreased. However, there is no preference in the order for performing the modification to decrease ACK activity and the modification to decrease POXB activity.

The phrase "acetyl-CoA (hereinafter, referred to as ACH) activity is decreased" means that the activity is lower than an ACH-unmodified strain or a wild-type strain, and the activity is preferably decreased to 50% or less, more preferably 30% or less, particularly preferably 10% or less per bacterial cell as compared to an ACH-unmodified strain. ACH activity may also be completely eliminated. The decreased ACH activity can be confirmed by determining the ACH activity by the method of Gergely, J. et al. (Gergely, J., Hele, P. & Ramkrishnan, C.V. (1952) J. Biol. Chem. 198 p323-334). A coryneform bacterium having decreased activities of ACH and POXB can be obtained by constructing a coryneform bacterium having decreased ACH activity and modifying it so that the POXB activity is decreased. However, there is no preference between the modification to decrease POXB activity and the modification to decrease ACH activity.

The phrase "acylphosphatase (hereinafter, referred to as ACP) activity is decreased" means that ACP activity is decreased as compared to a wild-type strain or an ACP-unmodified strain. The ACP activity is lower than a wild-type strain or an ACP-unmodified strain, and it is preferably decreased to 50% or less per bacterial cell, more desirably 10% or less per bacterial cell as compared to an ACP-unmodified strain. ACP activity may also be completely eliminated. The decreased ACP activity can be confirmed by determining the ACP activity by the same method as for the ACK activity (Ramponi G., Meth. Enzymol. 42, 409-426 (1975)). A coryneform bacterium having decreased activities of POXB and ACP can be obtained by constructing a coryneform bacterium having decreased POXB activity and modifying it so that the ACP activity is decreased However, there is no preference in the order for performing the modification to decrease ACP activity and the modification to decrease POXB activity.

Meanwhile, in the present invention, there may be used a bacterium modified so that an activity of pyruvate carboxylase (hereinafter, referred to as PC) is increased in addition to the decrease in POXB activity The phrase "pyruvate carboxylase activity is increased" means that PC activity is increased as compared to a wild-type strain or an unmodified strain such as a parent strain. The PC activity can be determined by the method of Peters-Wendisch PG et al. (Peters-Wendisch P.G. et al. Microbiology 143, 1095-1103 (1997)).

As a PC gene encoding a PC protein to be used in the method of the present invention, there may be employed a gene whose nucleotide sequence has been determined, or a gene obtained by isolating a DNA fragment that encodes a protein having PC activity from a chromosome of microorganisms, animals, plants, or the like according to the method described below and determining its nucleotide sequence. Further, after determination of the nucleotide sequence, a gene synthesized based on the sequence may be used. For example, there may be used a pyruvate carboxylase gene of *Corynebacterium glutamicum* ATCC13032 (GenBank Accession No. NCg10659 gene: SEQ ID NO: 60). Further, there may also be used PC genes derived from the following organisms.
Human [Biochem. Biophys. Res. Comm., 202, 1009-1014, (1994)]
Mouse [Proc. Natl. Acad. Sci. USA., 90, 1766-1779, (1993)]
Rat [GENE, 165, 331-332, (1995)]
Yeast; *Saccharomyces cerevisiae* [Mol. Gen. Genet., 229, 307-315, (1991)] *Schizosaccharomyces pombe* [DDBJ Accession No.; D78170]
*Bacillus stearothermophilus* [GENE, 191, 47-50, (1997)]
*Rhizobium etli* [J. Bacteriol., 178, 5960-5970, (1996)]

A DNA fragment containing a PC gene can be expressed by inserting the DNA fragment into a suitable expression plasmid such as pUC118 (Takara Bio Inc.), and introducing into a suitable host microorganism such as *Escherichia coli* JM109 (Takara Bio Inc.). The expressed PC gene product, which is pyruvate carboxylase, can be confirmed by determining PC activity by the known method as described above in the transformant, and then comparing the determined PC activity with PC activity of a crude enzyme solution extracted from a non-transformant strain. The DNA fragment containing PC gene is inserted into a suitable plasmid such as a plasmid vector containing at least a gene responsible for replication function of the plasmid in coryneform bacteria, thereby a recombinant plasmid capable of highly expressing PC in coryneform bacteria can be obtained. Here, in the recombinant plasmid, a promoter for expression of PC gene may be a promoter of coryneform bacteria. However, it is not limited to such a promoter, and any promoter can be used as long as it has a nucleotide sequence capable of initiating transcription of PC gene.

Plasmid vectors, into which PC gene can be introduced, are not limited as long as they contain at least a gene responsible for replication function in coryneform bacteria. Specific examples thereof include: plasmid pCRY30 described in JP03-210184A; plasmids pCRY21, pCRY2KE, pCRY2KX, pCRY31, pCRY3KE, and pCRY3KX each described in JP02-72876A and US Patent No. 5,185,262; plasmids pCRY2 and pCRY3 each described in JP01-191686A; pAM330 described in JP58-67679A; pHM1519 described in JP58-77895A; pAJ655, pAJ611, and pAJ 1844 each described in JP58-192900A; pCG1 described in JP57-134500A; pCG2 described in JP58-35197A; and pCGG4 and pCG11 each described in JP57-183799A.

Of those, plasmid vectors used in host-vector system for coryneform bacteria are preferably those having a gene responsible for replication function of the plasmid in coryneform bacteria and a gene responsible for stabilization function of the plasmid in coryneform bacteria. For instance, plasmids pCRY30, pCRY21, pCRY2KE, pCRY2KX, pCRY31, pCRY3KE, and pCRY3KX can be preferably used.

Coryneform bacteria having enhanced PC gene expression can be obtained by transforming a coryneform bacterium, for example, *Brevibacterium lactofermentum* 2256 strain (ATCC13869) with a recombinant vector prepared by inserting PC gene into an appropriate site of a plasmid vector which is replicable in aerobic coryneform bacteria as described above. Transformation can be carried out by, for example, the electric pulse method (Res. Microbiol., Vol. 144; p. 181-185, 1993). PC activity can also be increased by enhancing gene expression by introduction, substitution, amplification or the like of PC gene on a chromosome by a known homologous recombination method. By disrupting the poxB gene in a strain which highly expresses the PC gene, a bacterial strain with enhanced PC activity and decreased pyruvate oxidase activity can be obtained. There is no preference in the order for performing modifications to decrease pyruvate oxidase activity and to enhance PC activity.

Moreover, in the present invention, a bacterium, which has been modified so that activities of pyruvate oxidase, ACH, PTA and ACK are decreased and further modified so that LDH activity is decreased and PC activity is increased, is particularly effectively used for production of a substance, especially for production of succinic acid.

### <3> Production of succinic acid using the bacterium of the present invention

Succinic acid can be efficiently produced by culturing the thus obtained coryneform bacterium in a medium to produce and accumulate succinic acid in the medium and collecting succinic acid from the medium.

Upon use of the above-mentioned bacterium in reaction for producing succinic acid, the bacterium subjected to slant culture on such a solid medium as an agar medium may be used directly for the reaction, but a bacterium obtained by culturing the above-mentioned bacterium in a liquid medium (seed culture) in advance may be preferably used. Succinic acid may be produced by allowing the seed-cultured bacterium to react with an organic material while the bacterium is proliferating in a medium containing the organic raw material. In addition, succinic acid can also be produced by harvesting bacterial cells which has been proliferated and then reacting the bacterial cells with an organic raw material in reaction liquid containing the organic raw material. Further, for the purpose of using an aerobic coryneform bacterium in the method of the present invention, it is preferable to use the aerobic coryneform bacterium for the reaction after culturing the bacterium under a normal aerobic condition. The medium to be used for culture may be any medium normally used for culturing microorganisms. For instance, conventional media, which can be prepared by adding natural nutrient sources such as meat extract, yeast extract and peptone to a composition made of inorganic salts such as ammonium sulfate, potassium phosphate and magnesium sulfate, can be used. In the case of harvesting and using the bacterial cells after culture, the bacterial cells are harvested by centrifugation, membrane separation, or the like, and then used for the reaction.

In the present invention, immobilized bacterial cells which are immobilized on acrylamide, carrageenan or the like or disrupted bacterial cells can be used. Also disclosed are centrifugal supernatant thereof, or fractions obtained by partially purifying the supernatant with an ammonium sulfate treatment or the like.

An organic raw material to be used for the production method of the present invention is not particularly limited as long as it is a carbon source which can be assimilated by the microorganism described herein to produce succinic acid. In general, there is used a fermentable carbohydrate including: a carbohydrate such as galactose, lactose, glucose, fructose, glycerol, sucrose, saccharose, starch and cellulose; polyalcohol such as glycerin, mannitol, xylitol and ribitol. Of those, glucose, fructose and glycerol are preferable, and glucose is particularly preferable.

In addition, a saccharified starch liquid, molasses and the like, which contain any one of the above-mentioned fermentable carbonhydrates, can also be used. Any one of those fermentable carbonhydrates may be used alone or may be used in combination. The concentration at which the above-mentioned organic raw material is used is not particularly limited, but it is advantageous to increase the concentration as high as possible within the range that does not inhibit the production of succinic acid. The reaction is generally performed under the presence of the organic raw material in the range of 5 to 30% (w/v), preferably 10 to 20% (w/v). The organic raw material may be additionally added according to a decrease in the above-mentioned organic raw material when the reaction progresses.

The reaction liquid containing the organic raw material is not particularly limited. The reaction liquid to be used may be water, buffer, medium or the like, but the medium is most preferable. The reaction liquid is preferably one containing a nitrogen source, inorganic salts and the like. Here, the nitrogen source is not particularly limited as long as it can be assimilated by the microorganism described herein to produce succinic acid. Specific examples of the nitrogen source include various organic and inorganic nitrogen compounds such as ammonium salts, nitrate, urea, soybean hydrolysate, casein hydrolysate, peptone, yeast extract, meat extract, and corn steep liquor. Examples of the inorganic salts include various kinds of phosphoric salts, sulfate salts and metal salts of magnesium, potassium, manganese, iron, zinc, and the like. In addition, components that promote growth of bacterial cells including: vitamins such as biotin, pantothenic acid, inositol and nicotinic acid; nucleotides; and amino acids, may be added if necessary. Further, it is preferable that an appropriate amount of a commercially available antifoaming agent is added to the reaction liquid to suppress foaming at the time of reaction.

pH of the reaction liquid can be adjusted by adding sodium carbonate, sodium bicarbonate, potassium carbonate, potassium bicarbonate, magnesium carbonate, sodium hydroxide, calcium hydroxide, magnesium hydroxide, or the like. The pH for the reaction is usually 5 to 10, preferably 6 to 9.5, so the pH of the reaction liquid is adjusted within the above-mentioned range with an alkaline material, carbonate, urea, or the like during the reaction, if necessary.

The medium preferably contains carbonate ion, bicarbonate ion or carbonic acid gas (carbon dioxide). The carbonate ion or bicarbonate ion is supplied from magnesium carbonate, sodium carbonate, sodium bicarbonate, potassium carbonate, or potassium bicarbonate, which can also be used as a neutralizing agent. However, if necessary, the carbonate ion or bicarbonate ion can also be supplied from carbonic acid or bicarbonic acid, or salts thereof, or carbonic acid gas. Specific examples of the salts of carbonic acid or bicarbonic acid include magnesium carbonate, ammonium carbonate, sodium carbonate, potassium carbonate, ammonium bicarbonate, sodium bicarbonate, and potassium bicarbonate. In addition, the carbonate ion or bicarbonate ion is added at a concentration of 0.001 to 5 M, preferably 0.1 to 3 M, and more preferably 1 to 2 M. When carbonic acid gas is contained, the amount of the carbonic acid gas to be contained is 50 mg to 25 g, preferably 100 mg to 15 g, and more preferably 150 mg to 10 g per liter of the liquid.

The optimal temperature at which the bacterium to be used in the reaction grow is generally in the range of 25°C to 35°C. On the other hand, the temperature at the time of reaction is generally in the range of 25°C to 40°C, preferably in the range of 30°C to 37°C. The amount of bacterial cells to be used in the reaction is not particularly limited, but the amount is adjusted in the range of 1 to 700 g/L, preferably 10 to 500 g/L, and more preferably 20 to 400 g/L. The time period of the reaction is preferably 1 to 168 hours, more preferably 3 to 72 hours.

Upon culture of the bacterium, it is necessary to supply oxygen by aeration and agitation. On the other hand, the reaction for producing succinic acid may be performed with aeration and agitation, or may be performed under an anaerobic condition with neither aeration nor supply of oxygen. Here, the term "anaerobic condition" means that the reaction is conducted while keeping the dissolved oxygen low in the liquid. In this case, it is preferable to carry out the reaction at a dissolved oxygen of 0 to 2 ppm, preferably 0 to I ppm, and more preferably 0 to 0.5 ppm. For that purpose, there may be used a method in which a vessel is hermetically sealed to carry out the reaction without aeration; a method in which an inert gas such as a nitrogen gas is supplied to carry out the reaction; a method in which aeration with an inert gas containing carbonic acid gas is performed; and the like.

Succinic acid accumulated in the reaction liquid (culture solution) can be isolated and purified from the reaction liquid according to a conventional procedure. To be specific, succinic acid can be isolated and purified by removing solid materials including bacterial cells through centrifugation, filtration or the like, and desalting the solution with an ion exchange resin or the like, followed by crystallization or column chromatography from the solution.

In the present invention, after production of succinic acid by the method of the present invention as described above, a polymerization reaction is carried out using the obtained succinic acid as a raw material to produce a succinic acid-containing polymer. The succinic acid-containing polymer may be a homopolymer or a coploymer with other polymer raw materials. In recent years, environment-friendly industrial products are on the increase, and polymers prepared by using raw materials of a plant origin have been attracting attention. The succinic acid to be produced in the present invention can be processed into polymers such as polyester and polyamide and then used. Specific examples of the succinic acid-containing polymer include a succinic acid-containing ployester obtained through polymerization between a diol such as butanediol or ethylene glycol and succinic acid, and a succinic acid-containing polyamide obtained through polymerization between a diamine such as hexamethylenediamine and succinic acid.
Further, succinic acid or a composition containing succinic acid which can be obtained by the production method of the present invention can be used as food additives, pharmaceuticals, cosmetics, and the like.

### EXAMPLES

Hereinafter, the present invention will be described in further detail with reference to examples.

### EXAMPLE 1

### <1> Construction of a disruption vector carrying sacB gene

### (A) Construction of pBS3

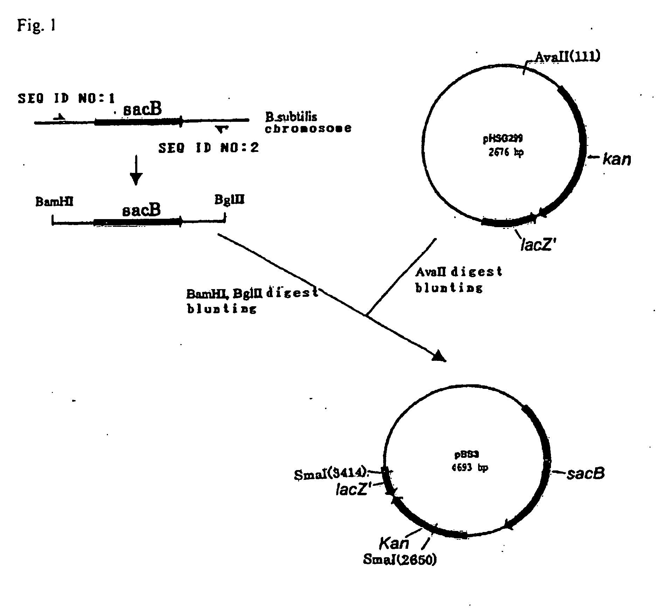

The sacB gene was obtained by PCR using a chromosomal DNA of *Bacillus subtilis* as a template and SEQ ID NOS: 1 and 2 as primers. PCR was carried out using LA Taq (Takara Bio Inc.) in such a way that one cycle of heat-retention at 94°C for 5 minutes was performed and then a cycle of denaturation at 94°C for 30 seconds, annealing at 49°C for 30 seconds and elongation at 72°C for 2 minutes was repeated 25 cycles. The PCR product thus obtained was purified by a conventional procedure and then digested with BgtII and BamHI, followed by blunt-ending. The fragment was inserted into a site of pHSG299 which had been digested with AvaII and blunt-ended. Competent cells of *Escherichia coli JM109* (Takara Bio Inc.) were used for transformation with this DNA and the transformed cells were applied on an LB medium containing 25 µg/ml kanamycin (hereinafter, abbreviated as Km), followed by overnight culture. Subsequently, appeared colonies were picked up, and single colonies were isolated, thereby transformants were obtained. Plasmids were extracted from transformants, and a plasmid into which a PCR product of interest was inserted was named pBS3. Fig. 1 shows the construction procedures of pBS3.

### (B) Construction of pBS4S

SmaI site in the kanamycin resistance gene sequence present on pBS3 was disrupted by crossover PCR-mediated nucleotide substitution causing no amino acid substitution to obtain a plasmid. First, PCR was carried out using pBS3 as a template and synthetic DNAs of SEQ ID NOS: 3 and 4 as primers, thereby amplified product of N-terminal region of the kanamycin resistance gene was obtained. On the other hand, to obtain amplified product of C-terminal region of the Km resistance gene, PCR was carried out using pBS3 as a template and synthetic DNAs of SEQ ID NOS: 5 and 6 as primers. The PCR was carried out using Pyrobest DNA Polymerase (Takara Bio Inc.) in such a way that one cycle of heat-retention at 98°C for 5 minutes was performed and then a cycle of denaturation at 98°C for 10 seconds, annealing at 57°C for 30 seconds and elongation at 72°C for 1 minute was repeated 25 cycles, to thereby yield a PCR product of interest. SEQ ID NOS: 4 and 5 are partially complementary to each other, and the SmaI site in the sequence is disrupted by nucleotide substitution causing no amino acid substitution. Next, to obtain a fragment of a mutant kanamycin resistance gene in which the SmaI site is disrupted, the gene products of the N-terminal and C-terminal regions of the above-mentioned kanamycin resistance gene were mixed at an approximately equimolar concentration, and PCR was carried out using the mixture of the gene products as templates and synthetic DNAs of SEQ ID NOS: 3 and 6 as primers, to thereby yield amplified product of a mutation-introduced Km resistance gene. PCR was carried out using Pyrobest DNA Polymerase (Takara Bio Inc.) in such a way that one cycle of heat-retention at 98°C for 5 minutes was performed and then a cycle of denaturation at 98°C for 10 seconds, annealing at 57°C for 30 seconds and elongation at 72°C for 1.5 minutes was repeated 25 cycles, to thereby yield a PCR product of interest.

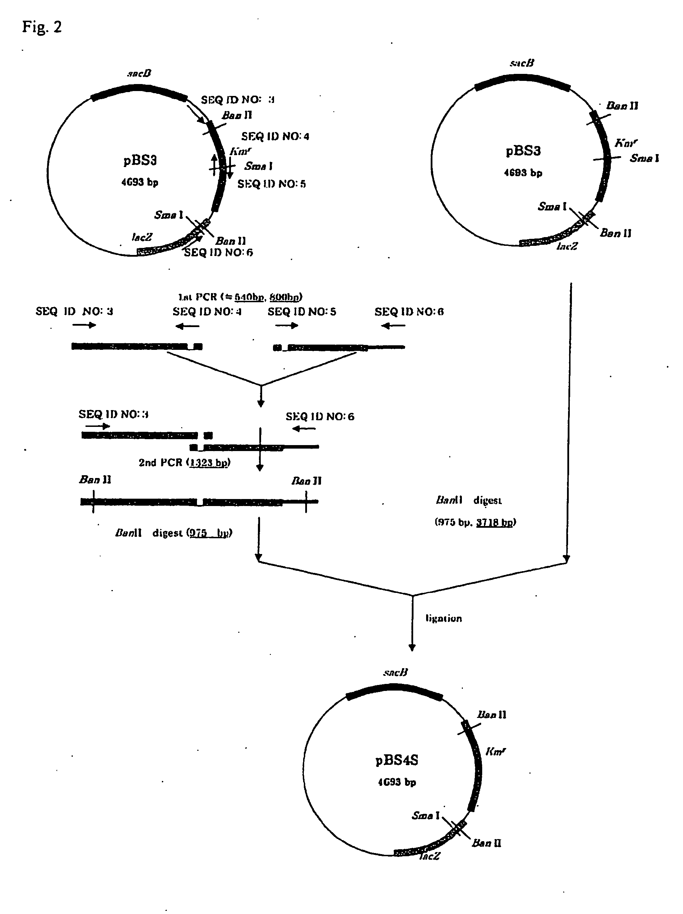

The PCR product was purified by a conventional procedure and then digested with HanII, followed by insertion into BanII site of the above-mentioned pBS3. Competent cells of *Escherichia coli* JM109 (Takara Bio Inc.) were used for transformation with this DNA and transformed cells were applied on an LB medium containing 25 µg/ml of kanamycin, followed by overnight culture. Subsequently, appeared colonies were picked up, and single colonies were isolated, thereby transformants were obtained. Plasmids were extracted from the transformants, and a plasmid into which a PCR product of interest was inserted was named pBS4S. Fig. 2 shows the construction procedures of pBS4S.

### EXAMPLE 2

### <Construction of LDH gene-disrupted strain>

### (A) Cloning of a fragment for disrupting lactate dehydrogenase gene

A fragment of a lactate dehydrogenase gene (hereinafter, abbreviated as ldh gene) derived from *Brevibacterium lactofermentum* 2256 strain in which ORF thereof was deleted was obtained by crossover PCR using as primers synthetic DNAs designed based on the nucleotide sequence (Ncg12810 of GenBank Database Accession No. NC_003450) of the gene of *Corynebacterium glutamicum* ATCC 13032 (GenBank Database Accession No. NC_003450), which has already been disclosed. That is, PCR was carried out by a conventional procedure using a chromosomal DNA of *Brevibacterium lactofermentum* 2256 strain as a template and synthetic DNAs of SEQ ID NOS: 7 and 8 as primers, thereby-amplified product of the N-terminal region of the ldh gene was obtained. On the other hand, to obtain amplified product of the C-terminal region of the ldh gene, PCR was carried out by a conventional procedure using a genomic DNA of *Brevibacterium lactofermentum* 2256 as a template and synthetic DNAs of SEQ ID NOS: 9 and 10 as primers. SEQ ID NO: 8 and 9 are complementary to each other and have structures for deleting the entire sequences of ldh ORF.
*Brevibacterium lactofermentum* 2256 strain is available from the American Type Culture Collection (ATCC) (Address: ATCC, P.O. Box 1549, Manassas, VA 20108, United States of America).

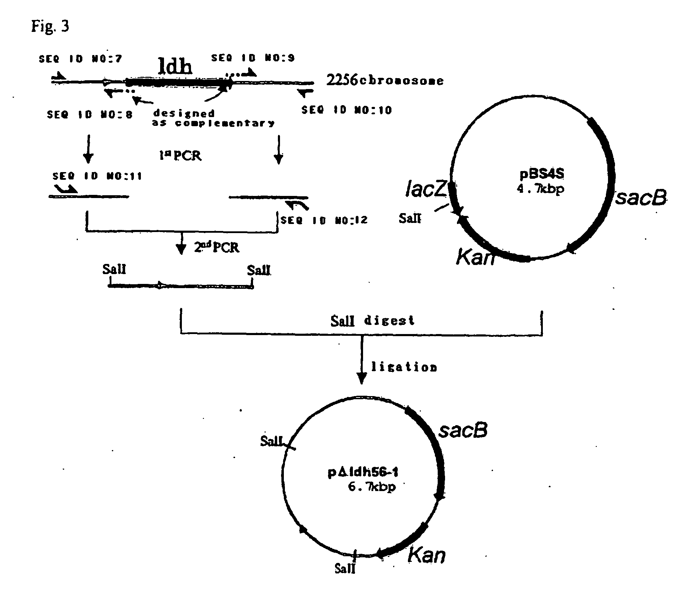

Next, to obtain a fragment of the ldh gene in which its internal sequence is deleted, the above-mentioned gene products of the N-terminal and C-terminal regions of ldh were mixed at an approximately equimolar concentration, and PCR was carried out by a conventional procedure using the mixture of the gene products as templates and synthetic DNAs of SEQ ID NOS: 11 and 12 as primers, to thereby yield amplified product of the mutation-introduced ldh gene. The PCR product thus obtained was purified by a conventional procedure and then digested with SalI, followed by insertion into SalI site of the above-mentioned pBS4S. Competent cells of *Escherichia coli* JM109 (Takara Bio Inc.) were used for transformation with this DNA and transformed cells were applied on an LB medium containing 100 µM of IPTG, 40 µg/ml of X-Gal, and 25 µg/ml of Km, followed by overnight culture. Subsequently, appeared white colonies were picked up, and single colonies were isolated, thereby transformants were obtained. Plasmids were extracted from the transformants, and a plasmid into which a PCR product of interest was inserted was named pΔldh56-1. Fig. 3 shows the construction procedures of the plasmid.

### (B) Preparation of ldh-disrupted strain

The pΔldh56-1 obtained by the above-mentioned (A) does not contain a region that enables autonomous replication in a cell of a coryneform bacterium. Therefore, when a coryneform bacterium is transformed with this plasmid, a strain in which the plasmid is integrated into a chromosome by homologous recombination appears at a very low frequency as a transformant. *Brevibacterium lactofermentum* 2256 strain was transformed using a high concentration of the plasmid pΔldh56-1 by the electric pulse method, and the transformed cells were applied on CM-Dex medium (5 g/L of glucose, 10 g/L of polypeptone, 10 g/L of yeast extract, 1 g/L of KH₂PO₄, 0.4 g/L of MgSO₄ 7H₂O, 0.01 g/L of FeSO₄·7H₂O, 0.01 g/L of MnSO₄·7H₂O, 3 g/L of urea, 1.2 g/L of soybean hydrolysate, pH 7.5 (KOH)) containing 25 µg/ml of kanamycin, followed by culture at 31.5°C for about 30 hours. A strain grown on the medium contains the kanamycin resistance gene and sacB gene which are derived from the plasmid on the genome, as a result of homologous recombination between the ldh gene fragment on the plasmid and the ldh gene on a genome of *Brevibacterium lactofermentum* 2256 strain.

Next, the single cross-over recombinant was cultured at 31.5°C overnight in CM-Dex liquid medium not containing kanamycin, and after suitable dilution, it was applied on 10% sucrose-containing Dex-S 10 medium (100 g/L of sucrose, 10 g/L of polypeptone, 10 g/L of yeast extract, 1 g/L of KH₂PO₄, 0.4 g/L of MgSO₄·7H₂O, 0.01 g/L of FeSO₄·7H₂O, 0.01 g/L of MnSO₄·4H₂O, 3 g/L of urea, 1.2 g/L of soybean hydrolysate, 10 µg/L of biotin, pH 7.5 (KOH)) not containing kanamycin, followed by culture at 31.5°C for about 30 hours. As a result, about 50 strains, which were considered to become sucrose-insensitive due to elimination of the sacB gene by second homologous recombination, were obtained.

The strains thus obtained include: a strain in which ldh gene was replaced by the mutant type derived from pΔldh56-1; and a strain in which ldh gene reverted to the wild type. Whether the ldh gene is the mutant type or the wild type can be confirmed easily by directly subjecting the bacterial strains obtained by culturing on Dex-S10 agar medium to PCR and detecting their ldh gene. In PCR analysis using primers (SEQ ID NOS: 7 and 10) for amplifying ldh gene, a strain which yielded a PCR product having a smaller size than that of a product obtained by PCR using a chromosomal DNA of the 2256 strain as a template was defined as an ldh-disrupted strain and used in the following experiments. As a result of the analysis of the sucrose-insensitive strains by the above-mentioned method, a strain carrying only the mutant type gene was selected and named 2256Δ(ldh) strain. Also, the strain was used as a parent strain for modification in the following examples.

### EXAMPLE 3

### <Construction of pyruvate oxidase gene-disrupted strain>

### (A) Cloning of a fragment for disrupting pyruvate oxidase gene

A fragment of a pyruvate oxidase gene (hereinafter, abbreviated as poxB gene) derived from *Brevibacterium lactofermentum* 2256 strain in which ORF thereof was deleted was obtained by crossover PCR using as primers synthetic DNAs designed based on the nucleotide sequence of the gene of *Corynebacterium glutamicum* ATCC13032 (NCg12521 of GenBank Database Accession No. NC_003450), which has already been disclosed. That is, PCR was carried out by a conventional procedure using a genomic DNA of *Brevibacterium lactofermentum* 2256 strain as a template and synthetic DNAs of SEQ ID NOS: 29 and 30 as primers, thereby amplified product of N-terminal region of the poxB gene was obtained.

On the other hand, to obtain an amplified product of C-terminal region of the poxB gene, PCR was carried out using a genomic DNA of *Brevibacterium lactofermentum* 2256 strain as a template and synthetic DNAs of SEQ ID NOS: 31 and 32 as primers. SEQ ID NOS: 30 and 31 are complementary to each other. PCR was carried out using KOD-plus-(TOYOBO) in such a way that one cycle of heat-retention at 94°C for 2 minutes was performed and then a cycle of denaturation at 94°C for 10 seconds, annealing at 55°C for 30 seconds and elongation at 68°C for 40 seconds was repeated 30 cycles, for the N-terminal and C-terminal regions. Next, to obtain a fragment of a poxB gene in which its internal sequence is deleted, the above-described amplified products of the N-terminal and C-terminal regions of poxB were mixed at an approximately equimolar concentration, and PCR was carried out using the mixture as templates and synthetic DNAs of SEQ ID NOS. 33 and 34 as primers, to thereby yield an amplified product of a mutation-introduced poxB gene. The PCR was carried out using KOD-plus- (TOYOBO) in such a way that one cycle of heat-retention at 94°C for 2 minutes was performed and then a cycle of denaturation at 94°C for 10 seconds, annealing at 55°C for 30 seconds and elongation at 68°C for 70. seconds was repeated 30 cycles, to thereby yield an amplified product of the mutation-introduced poxB gene of interest.

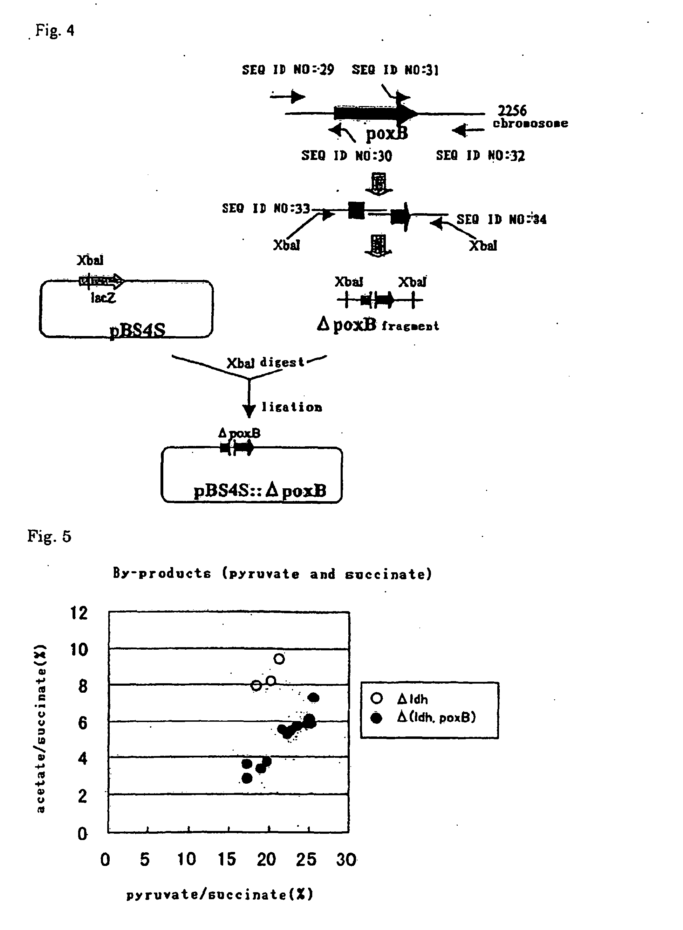

The PCR product thus obtained was purified by a conventional procedure and then digested with XbaI, followed by insertion into XbaI site of the pBS4S constructed in the above-mentioned Example 1(B). Competent cells of *Escherichia coli JM109* (Takara Bio Inc.) were used for transformation with this DNA and transformed cells were applied on an LB medium containing 100 µM of IPTG, 40 µg/ml of X-Gal; and 25 µg/ml of kanamaycin, followed by overnight culture. Subsequently, appeared white colonies were picked up, and single colonies were isolated, thereby transformants were obtained. Plasmids were extracted from the transformants, and a plasmid into which a PCR product of interest was inserted was named pBS4S::ΔpoxB. Fig. 4 shows the construction procedures of the pBS4S::ΔpoxB.

### (B) Preparation of poxB-disrupted strain -

The pBS4S::ΔpoxB obtained in the above-mentioned (A) does not contain a region that enables autonomous replication in a cell of a coryneform bacterium. Therefore, when a coryneform bacterium is transformed with the plasmid, the strain in which the plasmid is integrated into a chromosome by homologous recombination appears at a very low frequency as a transformant. *Brevibacterium lactofermentum* 2256Δ(ldh) strain prepared in Example 2 was transformed using a high concentration of the plasmid pBS4S::ΔpoxB by the electric pulse method, and then applied on CM-Dex medium containing 25 µg/ml of kanamycin, followed by culture at 31.5°C for about 30 hours. The strain grown on the medium contains a kanamycin resistance gene and a sacB gene which are derived from the plasmid on the genome, as a result of homologous recombination between the poxB gene fragment on the plasmid and the poxB gene on a genome of *Brevibacterium lactofermentum* 22566(ldh) strain.

Next, the single crossover recombinant was cultured at 31.5°C overnight in CM-Dex liquid medium not containing kanamycin, and then, after suitable dilution, it was applied on 1.0% sucrose-containing Dex-S 10 medium not containing kanamycin, followed by culture at 31.5°C for about 30 hours.
As a result, about 30 strains, which were considered to become sucrose-insensitive due to elimination of the sacB gene by the second homologous recombination, were obtained.

The thus obtained strains include: a strain in which poxB gene was replaced by the mutant type derived from pBS4S::ΔpoxB; and a strain in which poxB gene reverted to the wild type. Whether the poxB gene is the mutant type or the wild type can be confirmed easily by directly subjecting a bacterial strain obtained through culture on a Dex-S10 agar medium to PCR and detecting the poxB gene. Analysis of the poxB gene by using primers (SEQ ID NOS: 29 and 32) for PCR amplification should result in a DNA fragment of 2.4 kb for the wild type and a DNA fragment of 1.2 kb for the mutant type having the deleted region. As a result of the analysis of the sucrose-insensitive strain by the above-mentioned method, a strain carrying only the mutant type gene was selected and named 2256Δ(ldh, poxB). The strain is also called a poxB-disrupted strain, herein.

### EXAMPLE 4

### <Succinic acid production by the poxB-disrupted strain>

### (A) Evaluation of culture of the poxB-disrupted strain

*Brevibacterium lactofermentum* 2256Δ(ldh) strain and 2256Δ(ldh, poxB) strain were used for culture for producing succinic acid as described below. The bacterial cells of the 2256Δ(ldh) strain and 2256Δ(ldh, poxB) strain obtained by culturing them on a CM-Dex plate medium were inoculated into 3 ml of a seed medium (20 g/L of glucose, 4 g/L of Urea, 14 g/L of (NH₄)₂SO₄, 0.5 g/L of KH₂PO₄, 0.5 g/L of K₂HPO₄ 0.5 gIL of MgSO₄·7H₂O, 0.02 g/L of FeSO₄ ·7H₂O, 0.02 g/L of MnSO₄·7H₂O, 200 µg/L of biotin, 200 µg/L of VB_{I}·HCl, 1 g/L of yeast extract, and 1 g/L of casamino acid: with no pH adjustment; glucose was added after being independently sterilized). Shaking culture was performed in a test tube at 31.5°C for about 16 hours under an aerobic condition.

After that, 3 ml of a main medium A (100 g/L of glucose, 15 g/L of sodium sulfite, and 71.4 g/L of MgCO₃ was added into the tube. For preventing aeration, the succinic acid production culture was carried out while the tube was sealed hermetically with a silicon cap. The culture was performed by shaking at 31.5°C for about 48 hours and terminated before sugar in the medium was exhausted.

After completion of the culture, the accumulation amounts of succinic acid and by-product acetic acid in the medium were analyzed by liquid chromatography after the medium had been suitably diluted. A column obtained by connecting two pieces of Shim-pack SCR-102H (Shimadzu) in series was used, and the sample was eluted at 40°C by using 5 mM p-toluene sulfonic acid. The eluent was neutralized by using 20 mM Bis-Tris aqueous solution containing 5 mM p-toluene sulfonic acid and 100 µM of EDTA. The succinic acid and acetic acid were each measured by determining the electric conductivity by means of CDD-10AD (Shimadzu). The obtained results are shown in Table 1 and Fig. 5.

In the case of 2256Δ(ldh, poxB) strain, the succinic acid production was equal to the parent strain 2256Δ(ldh), but ratio of acetic acid with respect to succinic acid was about one third to two third of the 2256Δ(ldh, poxB) strain. These results indicated that eliminating or decreasing poxB activity is effective for reducing acetic acid under anaerobic conditions.

**Table 1. Production of succinic acid, acetic acid and pyruvic acid by the poxB-disrupted strain**

| | Succinic acid (g/L) | Acetic acid (g/L) | Pyruvic acid (g/L) | Pyruvic acid/ succinic acid (%) | Acetic acid/ succinic acid (%) |
|---|---|---|---|---|---|
| 2256Δldh | 38.5 | 3.6 | 8.2 | 21.4 | 9.4 |
| | 45.1 | 3.6 | 8.2 | 18.3 | 7.9 |
| | 42.2 | 3.5 | 8.5 | 20.1 | 8.2 |
| 2256Δ (ldh, poxB) | 42.0 | 2.3 | 9.6 | 22.8 | 5.4 |
| | 42.4 | 2.2 | 9.5 | 22.4 | 5.2 |
| | 35.7 | 2.6 | 9.1 | 25.5 | 7.3 |
| | 38.4 | 2.3 | 9.6 | 25.1 | 6.1 |
| | 40.9 | 2.3 | 9.7 | 23.7 | 5.7 |
| | 49.1 | 1.9 | 9.7 | 19.8 | 3.8 |
| | 51.3 | 1.5 | 8.8 | 17.2 | 2.9 |
| | 48.3 | 1.6 | 9.2 | 19.0 | 3.4 |
| | 51.2 | 1.9 | 8.8 | 17.2 | 3.6 |
| | 38.4 | 2.2 | 9.7 | 25.2 | 5.8 |
| | 43.8 | 2.4 | 9.5 | 21.8 | 5.5 |
| | 37.9 | 2.2 | 9.4 | 24.9 | 5.9 |

### EXAMPLE 5

### <Construction of poxB, pta, ack-disrupted strain and poxB, pta, ack, ach-disrupted strain

### (5-1) <Construction of acetate kinase gene-disrupted strain>

### (A) Cloning of a fragment for disrupting acetate kinase gene

A fragment of an acetate kinase gene (hereinafter, abbreviated as ack) of *Brevibacterium lactofermentum* 2256 strain in which ORF thereof was deleted was obtained by crossover PCR.using as primers synthetic DNAs designed based on the nucleotide ' sequence of the gene of *Corynebacterium glutamicum* ATCC13032 (NCgl 2656 of GenBank Database Accession No. NC_003450; SEQ ID NO: 45), which has already been disclosed. That is, PCR was carried out using a genomic DNA of *Brevibacterium lactofermentum* 2256 strain as a template and synthetic DNAs of SEQ ID NOS: 13 and 14 as primers, thereby amplified product of N-terminal region of the ack gene was obtained. On the other hand, to obtain amplified product of C-terminal region of the ack gene, PCR was carried out using a genomic DNA of *Brevibacterium lactofermentum* 2256 strain as a template and synthetic DNAs of SEQ ID NOS: 15 and 16 as primers. SEQ ID NOS: 14 and 15 are partially complementary to each other. The PCR was carried out using KOD-plus- (TOYOBO) in such a way that one cycle of heat-retention at 94°C for 2 minutes was performed and then, for the N-terminal region, a cycle of denaturation at 94°C for 10 seconds, annealing at 55°C for 30 seconds and elongation at 68°C for 30 seconds, and for the C-terminal region, a cycle of denaturation at 94°C for 10 seconds, annealing at 55°C for 30 seconds and elongation at 68°C for 2 minutes were repeated 30 cycles, respectively. Next, to obtain a fragment of ack gene in which its internal sequence is deleted, the gene products of the above-mentioned N-terminal and C-terminal regions of ack were mixed at an approximately equimolar concentration, and PCR was carried out using the mixture of the gene products as templates and synthetic DNAs of SEQ ID NOS: 17 and 18 as primers, to thereby yield amplified product of a mutation-introduced ack gene. The PCR was carried out using KOD-plus-(TOYOBO) in such a way that one cycle of heat-retention at 94°C for 2 minutes was performed and then a cycle of denaturation at 94°C for 10 seconds, annealing at 55°C for 30 seconds and elongation at 68°C for 2.5 minutes was repeated 30 cycles, to thereby yield an amplified product of the mutation-introduced ack gene of interest.

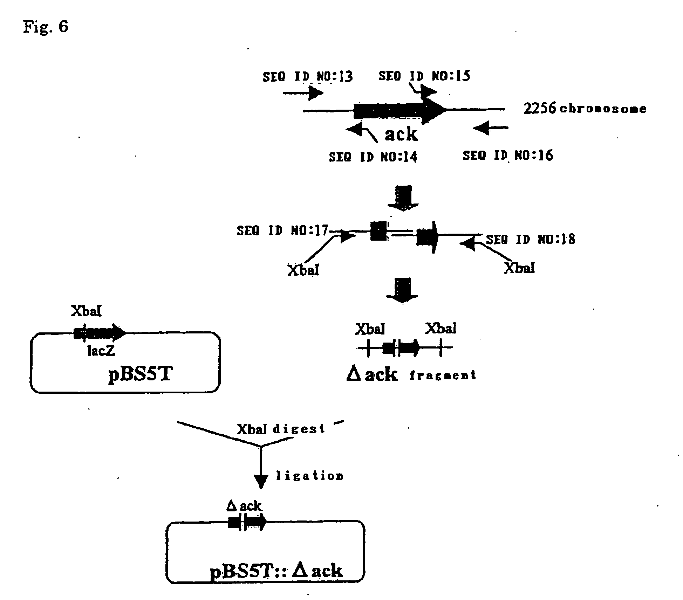

The obtained PCR product was purified by a conventional procedure and then digested with XbaI, followed by insertion into XbaI site of pBS5T constructed in the above-mentioned Example 1 (C). Competent cells of *Escherichia coli JM109* (Takara Bio Inc.) were used for transformation with this DNA and applied on an LB medium containing 100 µM of IPTG, 40 µg/ml of X-Gal, and 25 µg/ml of kanamycin, followed by overnight culture. Subsequently, appeared white colonies were picked up, and single colonies were isolated, thereby transformants were obtained. Plasmids were extracted from the transformants, and a plasmid into which a PCR product of interest was inserted was named pBSST::Δack. Fig. 6 shows the construction procedures of pBS5T::Δack.

### (B) Preparation of ack-disrupted strain

The replication origin for coryneform bacteria in pBSST::Δack obtained in the above-mentioned (A) is temperature-sensitive. That is, the plasmid is autonomously replicable in a cell of a coryneform bacterium at 25°C, but it is not autonomously replicable at 31.5°C (or 34°C). *Brevibacterium lactofermentum* 2256Δ(ldh) strain was transformed using the plasmid by the electric pulse method, and applied on a CM-Dex medium containing 25 µg/ml of kanamycin, followed by culture at 25°C for 2 nights. Appeared colonies were isolated, to thereby yield transformants. The transformants contain the plasmid. The transformants were cultured at 34°C overnight in the CM-Dex medium not containing kanamycin and then, after suitable dilution, it was applied on a CM-Dex medium containing 25 µg/ml of kanamycin, followed by culture at 34°C for about 30 hours. The strain grown on the medium contains a kanamycin resistance gene and a sacB gene which are derived from the plasmid on the genome, as a result of homologous recombination between the ack gene fragment on the plasmid and the ack gene on a genome of *Brevibacterium lactofermentum* 2256Δ(ldh) strain.

Next, the single crossover recombinant was cultured at 31.5°C overnight in a CM-Dex liquid medium not containing kanamycin and, after suitable dilution, it was applied on 10% sucrose-containing Dex-S10 medium not containing kanamycin, followed by culture at 31.5°C for about 30 hours. As a result, about 50 strains, which were considered to become sucrose-insensitive due to elimination of the sacB gene by the second homologous recombination, were obtained.

The thus obtained strains include: a strain in which ack gene was replaced by the mutant type derived from pBSST::Δack; and a strain in which ack gene reverted to the wild type. Whether the ack gene is the mutant type or the wild type can be confirmed easily by directly subjecting a bacterial strain obtained through culturing in a Dex-S10 agar medium to PCR and detecting the ack gene. Analysis of the ack gene by using primers (SEQ ID NOS: 13 and 16) for PCR amplification should result in a DNA fragment of 3.7 kb for the wild type and a DNA fragment of 2.5 kb for the mutant type having the deleted region. As a result of the analysis of the sucrose-insensitive strain by the above-mentioned method, a strain carrying only the mutant type gene was selected and named 2256A(ldh, ack).

### (5-2)<Construction of acetate kinase gene- and phosphotransacetylase gene-disrupted strain>

### (A) Cloning of fragments for disrupting acetate kinase gene and phosphotransacetylase gene

The ORFs of acetate kinase (ack) gene and phosphotransacetylase gene (hereinafter, referred to as pta) of *Brevibacterium lactofermentum* 2256 strain have an operon structure, and the both ORFs can be made deficient simultaneously. These gene fragments were obtained by cross-over PCR using as primers synthetic DNAs designed based on the nucleotide sequences of the genes of *Corynebacterium glutamicum* ATCC 13032 (NCgl2656 and 2657 of GenBank Database Accession No. NC_003450; SEQ ID NO: 45), which have already been disclosed. That is, PCR was carried out using a genomic DNA of *Brevibacterium lactofermentum* 2256 strain as a template and synthetic DNAs of SEQ ID NOS: 19 and 20 as primers, to thereby yield an amplified product of N-terminal region of the pta gene. On the other hand, to yield an amplified product of C-terminal region of the ack gene, PCR was carried out using a genomic DNA of *Brevibacterium lactofermentum* 2256 strain as a template and synthetic DNAs of SEQ ID NOS: 21 and 16 as primers. SEQ ID NOS: 20 and 21 are partially complementary to each other. The PCR was performed by using KOD-plus- (TOYOBO) in such a way that one cycle of heat-retention at 94°C for 2 minutes was performed and then, for the N-terminal region, a cycle of denaturation at 94°C for 10 seconds, annealing at 55°C for 30 seconds and elongation at 68°C for 30 seconds, and for the C-terminal region, a cycle of denaturation at 94°C for 10 seconds, annealing at 55°C for 30 seconds and elongation at 68°C for 2 minutes were each repeated 30 cycles, respectively.

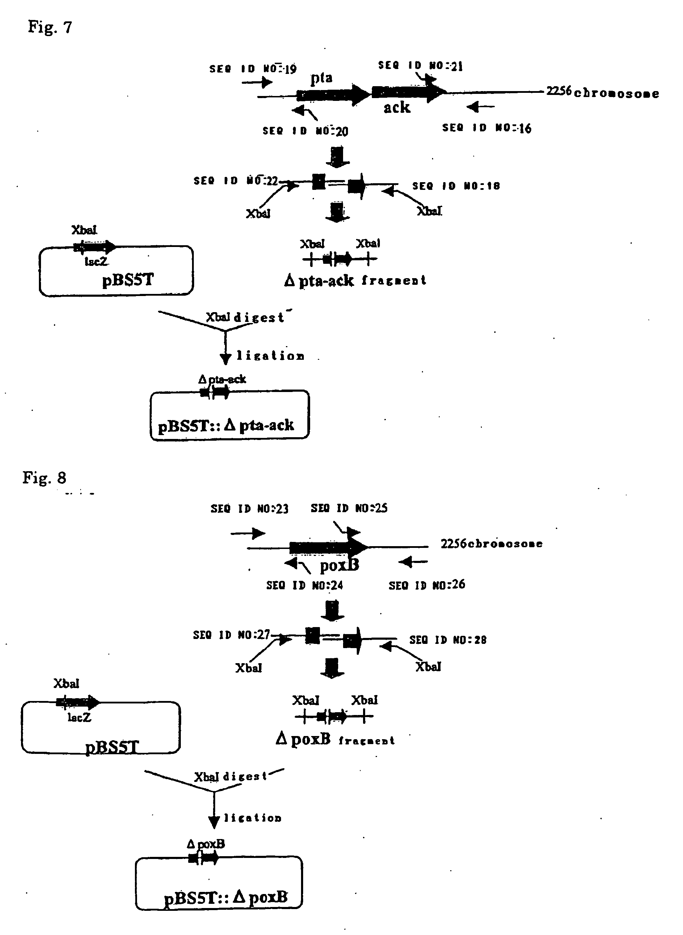

Next, to obtain a fragment of a pta-ack gene in which an internal sequence in pta and ack is deleted, the gene products of the above-mentioned N-terminal region of pta and C-terminal region of ack were mixed at an approximately equimolar concentration, and PCR was carried out using the mixture as templates and synthetic DNAs of SEQ ID NOS: 22 and 18 as primers, to thereby yield amplified product of a mutation-introduced pta-ack gene. The PCR was carried out using KOD-plus- (TOYOBO) in such a way that one cycle of heat-retention at 94°C for 2 minutes was performed and then a cycle of denaturation at 94°C for 10 seconds, annealing at 55°C for 30 seconds and elongation at 68°C for 2.5 minutes was repeated 30 cycles, to thereby yield an amplified product of the mutation-introduced pta-ack gene of interest. The PCR product thus obtained was purified by a conventional procedure and digested with XbaI, followed by insertion into XbaI site of pBS5T. Competent cells of *Escherichia coli* JM109 (Takara Bio Inc.) were used for transformation with this DNA and applied on an LB medium containing 100 µM of IPTG, 40 µg/ml of X-Gal, and 25 µg/ml of kanamycin, followed by overnight culture. Subsequently, appeared white colonies were picked up, and single colonies were isolated, thereby transformants were obtained. Plasmids were extracted from the transformants, and a plasmid into which PCR product of interest was inserted was named pBS5T::Δpta-ack. Fig. 7 shows the construction procedures of pBS5T::Δpta-ack.

### (B) Preparation of pta-ack-disrupted strain

The replication origin for coryneform bacteria in pBS5T::Δpta-ack obtained in the above-mentioned (A) is temperature-sensitive. That is, the plasmid is autonomously replicable in a cell of a coryneform bacterium at 25°C, but it is not autonomously replicable at 31.5°C (or 34°C). *Brevibacterium lactofermentum* 2256Δ(ldh) strain was transformed using the plasmid by the electric pulse method, and applied on a CM-Dex medium containing 25 µg/ml of kanamycin, followed by culture at 25°C for 2 nights. Appeared colonies were isolated, to thereby yield transformants. The transformants have the plasmid. The transformants were cultured at 34°C overnight in a CM-Dex liquid medium not containing kanamycin and then, after suitable dilution, it was applied on a CM-Dex liquid medium containing 25 µg/ml of kanamycin, followed by culture at 34°C for about 30 hours. The strain grown on the medium contains the kanamycin resistance gene and sacB gene which are derived from the plasmid on the genome, as a result of homologous recombination between the pta-ack gene fragment on the plasmid and the pta-ack gene on a genome of *Brevibacterium lactofermentum* 2256Δ(ldh) strain.

Next, the single crossover recombinant was cultured at 31.5°C overnight in CM-Dex liquid medium not containing kanamycin and, after suitably dilution, it was applied on 10% sucrose-containing Dex-S10 medium not containing kanamycin, followed by culture at 31.5°C for about 30 hours. As a result, about 50 strains, which were considered to become sucrose-insensitive due to elimination of the sacB gene by the second homologous recombination, were obtained.

The thus obtained strains include: a strain in which pta and ack genes were replaced by the mutant type derived from pBS5T::Δpta-ack; and a strain in which pta and ack genes reverted to the wild type. Whether the pta-ack gene is the mutant type or the wild type can be confirmed easily by directly subjecting a bacterial strain obtained through culture in a Dex-S10 agar medium to PCR and detecting the pta and ack genes. Analysis of the pta-ack gene by using primers (SEQ ID NOS: 19 and 16) for PCR amplification should result in a DNA fragment of 5.0 kb for the wild type and a DNA fragment of 2.7 kb for the mutant type having a deleted region.

As a result of the analysis of the sucrose-insensitive strains by the above-mentioned method, a strain carrying only the mutant type gene was selected and named 2256A(ldh, pta, ack).

### (5-3)<Construction of acetate kinase gene-, phosphotransacetylase gene-, pyruvate oxidase gene-disrupted strain>

### (A) Cloning of a fragment for disrupting pyruvate oxidase gene

A fragment of a pyruvate oxidase gene (hereinafter, abbreviated as poxB) of *Brevibacterium lactofermentum* 2256 strain in which the ORF thereof was deleted was obtained by crossover PCR using as primers synthetic DNAs designed based on the nucleotide sequence of the gene of *Corynebacterium glutamicum* ATCC13032 (NCgl2521 of GenBank Database Accession No. NC_003450; SEQ ID NO: 48), which has already been disclosed. That is, PCR was carried out using a genomic DNA of *Brevibacterium lactofermentum* 2256 strain as a template and synthetic DNAs of SEQ ID NOS: 23 and 24 as primers, thereby amplified product of N-terminal region of the poxB gene was obtained.

On the other hand, to obtain amplified product of C-terminal region of the poxB gene, PCR was carried out using a genomic DNA of *Brevibacterium lactofermentum* 2256 strain as a template and synthetic DNAs of SEQ ID NOS: 25 and 26 as primers. SEQ ID NOS: 24 and 25 are complementary to each other. The PCR was carried out using KOD-plus-(TOYOBO) in such a way that one cycle of heat-retention at 94°C for 2 minutes was performed and then a cycle of denaturation at 94°C for 10 seconds, annealing at 55°C for 30 seconds and elongation at 68°C for 40 seconds was repeated 30 cycles, for both of the N-terminal region and the C-terminal region. Next, to obtain a fragment of poxB gene in which its internal sequence is deleted, the above-mentioned gene products of the N-terminal and C-terminal regions of poxB were mixed at an approximate equimolar concentration, and PCR was carried out using the mixture as templates and synthetic DNAs of SEQ ID NOS: 27 and 28 as primers, to thereby yield amplified product of a mutation-introduced poxB gene. The PCR was carried out using KOD-plus- (TOYOBO) in such a way that one cycle of heat-retention at 94°C for 2 minutes was performed, and then a cycle of denaturation at 94°C for 10 seconds, annealing at 55°C for 30 seconds and elongation at 68°C for 70 seconds was repeated 30 cycles, to thereby yield an amplified product of the mutation-introduced poxB gene of interest.

The PCR product thus obtained was purified by a conventional procedure and then digested with XbaI, followed by insertion into XbaI site of pBS5T constructed in the above-mentioned Example 1 (C). Competent cells of *Escherichia coli* JM109 (Takara Bio Inc.) were used for transformation with this DNA and applied on an LB medium containing 100 µM of IPTG, 40 µg/ml of X-Gal, and 25 µg/ml of kanamycin, followed by overnight culture. Subsequently, appeared white colonies were picked up, and single colonies were isolated, thereby transformants were obtained. Plasmids were extracted from the transformants, and a plasmid into which a PCR product of interest was inserted was named pBS5T::ΔpoxB. Fig. 8 shows the construction procedures of pBS5T::ΔpoxB.

### (B) Preparation of poxB-disrupted strain

The replication origin for coryneform bacteria in pBS5T::ΔpoxB obtained in the above-mentioned Example 5 (A) is temperature-sensitive. That is, the plasmid is autonomously replicable in a cell of a coryneform bacterium at 25°C, but it is not autonomously replicable at 31.5°C (or 34°C). *Brevibacterium lactofermentum* 2256Δ(ldh, pta, ack) strain was transformed using the plasmid by the electric pulse method, and then applied on a CM-Dex medium containing 25 µg/ml of kanamycin, followed by culture at 25°C for 2 nights. Appeared colonies were isolated, to thereby yield transformants. The transformants should have the plasmid.

The transformants were cultured at 34°C overnight in a CM-Dex liquid medium not containing kanamycin, and after suitable dilution, it was applied on a CM-Dex medium containing 25 µg/ml of kanamycin, followed by culture at 34°C for about 30 hours. In the strain grown on the medium, the kanamycin resistance gene and sacB gene which are derived from the plasmid are inserted into the genome, as a result of homologous recombination between the poxB gene fragment on the plasmid and the poxB gene on a genome of *Brevibacterium lactofermentum* 2256Δ(ldh, pta, ack) strain.
Next, the single crossover recombinant was cultured at 31.5°C overnight in CM-Dex liquid medium not containing kanamycin, and after suitable dilution, it was applied on 10% sucrose-containing Dex-S10 medium not containing kanamycin, followed by culture at 31.5°C for about 30 hours. As a result, about 50 strains, which were considered to become sucrose-insensitive due to elimination of the sacB gene by the second homologous recombination, were obtained.

The thus obtained strains include: a strain in which poxB gene was replaced by the mutant type derived from pBS5T::ΔpoxB; and a strain in which the poxB gene reverted to the wild type. Whether the poxB gene is the mutant type or the wild type can be confirmed easily by directly subjecting a bacterial strain obtained through culture in a Dex-S10 agar medium to PCR and detecting the poxB gene. By analyzing the poxB gene by using primers (SEQ ID NOS: 23 and 26) for PCR amplification, a DNA fragment of 2.4 kb for the wild type and a DNA fragment of 1.2 kb for the mutant type having the deleted region can be detected. As a result of the analysis of the sucrose-insensitive strain by the above-mentioned method, a strain carrying only the mutant type gene was selected and named 2256Δ(ldh, pta, ack, poxB). The strain is also called a pta, ack, poxB-disrupted strain, herein.

### (5-4)<Construction of poxB, pta, ack, ach gene-disrupted strain>

### (A) Cloning of a fragment for disrupting acetyl-CoA hydrolase gene

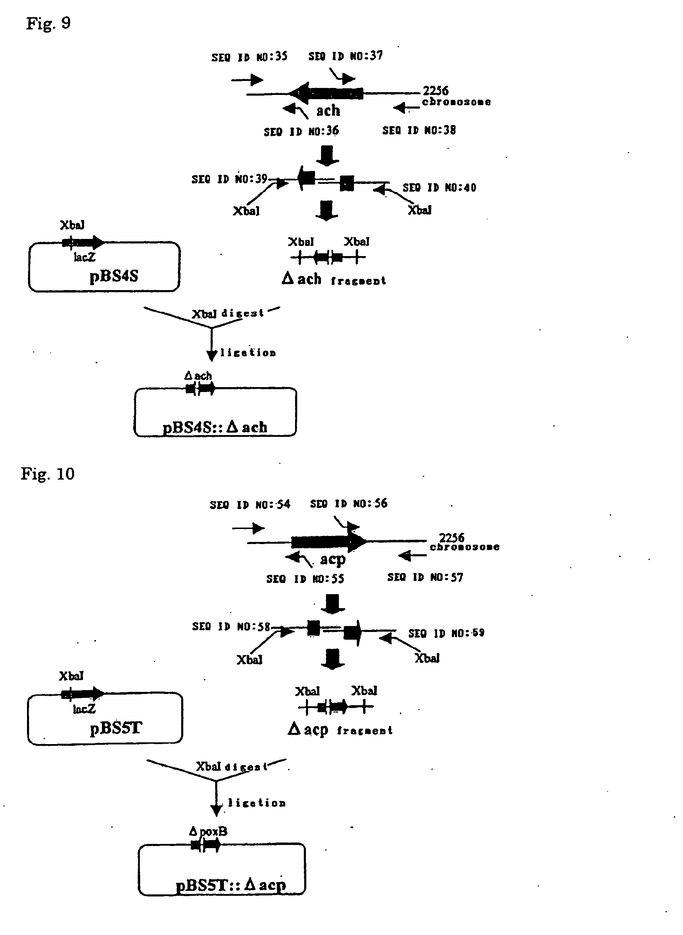

A fragment of an acetyl-CoA hydrolase gene (hereinafter, abbreviated as ach) of *Brevibacterium lactofermentum* 2256 strain in which ORF thereof was deleted was obtained by crossover PCR using as primers synthetic DNAs designed based on the nucleotide sequence of the gene of *Corynebacterium glutamicum* ATCC13032 (NCgl 2480 of GenBank Database Accession No. NC_003450; SEQ ID NO: 50), which has already been disclosed. That is, PCR was carried out using a genomic DNA of *Brevibacterium lactofermentum* 2256 strain as a template and synthetic DNAs of SEQ ID NOS: 35 and 36 as primers, thereby amplified product of C-terminal region of the ach gene was obtained. On the other hand, to obtain amplified product of N-terminal region of the ach gene, PCR was carried out using a genomic DNA of *Brevibacterium lactofermentum* 2256 strain as a template and synthetic DNAs of SEQ ID NOS: 37 and 38 as primers. SEQ ID NOS: 36 and 37 are complementary to each other. The PCR was carried out using KOD -plus- (TOYOBO) in such a way that one cycle of heat-retention at 94°C for 2 minutes was performed and then a cycle of denaturation at 94°C for 10 seconds, annealing at 55°C for 30 seconds and elongation at 68°C for 50 seconds was repeated 30 cycles, for the N-terminal region and the C-terminal region. Next, to obtain a fragment of the ach gene in which its internal sequence is deleted, the above-mentioned gene products of the N-terminal and C-terminal regions of ach were mixed at an approximately equimolar concentration, and PCR was carried out using the mixture as templates and synthetic DNAs of SEQ ID NOS: 39 and 40 as primers, to thereby yield amplified products of a mutation-introduced ach gene. The PCR was carried out using KOD-plus- (TOYOBO) in such a way that one cycle of heat-retention at 94°C for 2 minutes was performed and then a cycle of denaturation at 94°C for 10 seconds, annealing at 55°C for 30 seconds and elongation at 68°C for 90 seconds was repeated 30 cycles, to thereby yield an amplified product of the mutation-introduced ach gene of interest. The PCR product thus obtained was purified by a conventional procedure and digested with XbaI, followed by insertion into XbaI site of pBS4S constructed in the above-mentioned Example 1 (B). Competent cells of *Escherichia coli* JM109 (Takara Bio Inc.) were used for transformation with this DNA and applied on an LB medium containing 100 µM of IPTG, 40 µg/ml of X-Gal, and 25 µg/ml of kanamycin, followed by overnight culture. Subsequently, appeared white colonies were picked up, and single colonies were isolated, thereby transformants were obtained. Plasmids were extracted from the transformants, and a plasmid into which a PCR product of interest was inserted was named pBS4S::Δach. Fig. 9 shows the construction procedures of pBS4S::Δach.

### (B) Preparation of ach-disrupted strain

The pBS4S::Δach obtained in the above-mentioned (A) does not include a region which enables autonomous replication in a cell of a coryneform bacterium, so when a coryneform bacterium is transformed with the plasmid, a strain in which the plasmid is integrated into a chromosome by homologous recombination appears at a very low frequency as a transformant. *Brevibacterium lactofermentum* 2256Δ(ldh, pta, ack, poxB) strain and 2256 strain were transformed by using a high concentration of the plasmid pBS4S::Δach by the electric pulse method, and applied on a CM-Dex medium containing 25 µg/ml kanamycin, followed by culture at 31.5°C for about 30 hours. In the strains grown on the medium, a kanamycin resistance gene and a sacB gene derived from the plasmid are inserted on the genome, as a result of homologous recombination between the ach gene fragment on the plasmid and the ach gene on a genome of each of *Brevibacterium lactofermentum* 2256Δ(ldh) strain, 2256Δ(ldh, pta, ack) strain, 2256Δ(ldh, pta, ack, poxB) strain, and 2256Δ(ldh, pta, ack, poxB, acp) strain.

Next, the single crossover recombinant was cultured at 31.5°C overnight in CM-Dex liquid medium not containing kanamycin and then, after suitable dilution, it was applied on 10% sucrose-containing Dex-S10 medium not containing kanamycin, followed by culture at 31.5°C for about 30 hours. As a result, about 50 strains, which were considered to become sucrose-insensitive due to elimination of the sacB gene by the second homologous recombination, were obtained.

The thus obtained strains include: a strain in which ach gene was replaced by the mutant type derived from pBS4S::Δach; and a strain in which ach gene reverted to the wild type. Whether the ach gene is the mutant type or the wild type can be confirmed easily by directly subjecting a bacterial strain obtained through culture in a Dex-S10 agar medium to PCR and detecting the ach gene. Analysis of the ach gene by using primers (SEQ ID NOS: 35 and 38) for PCR amplification should result in a DNA fragment of 2.9 kb for the wild type and a DNA fragment of 1.4 kb for the mutant type having the deleted region. As a result of the analysis of the sucrose-insensitive strain by the above-mentioned method, a strain carrying only the mutant type gene was selected and the strain obtained from 2256Δ(ldh, pta, ack, poxB) was named 2256Δ(ldh, pta, ack, poxB, ach) strain. The strain is also called an ach, pta, ack, poxB-disrupted strain, herein.

### EXAMPLE 6

### <Construction of acylphosphatase gene-disrupted strain>

### (A) Cloning of a fragment for disrupting acylphosphatase gene

A gene fragment of an acylphosphatase gene (hereinafter, referred to as acp) of *Brevibacterium lactofermentum* 2256 strain in which ORF thereof was deleted was obtained by crossover PCR using as primers synthetic DNAs designed based on a nucleotide sequence (SEQ ID NO. 52), which is obtained as a sequence having high homology to acp gene of *Mycobacterium tuberculos* from a search in a sequence of *Brevibacterium lactofermentum* ATCC13869 strain as identified by genomic analysis. That is, PCR was carried out using a genomic DNA of *Brevibacterium lactofermentum* 2256 strain as a template and synthetic DNAs of SEQ ID NOS: 54 and 55 as primers, thereby an amplified product of C-terminal region of the acp gene was obtained. On the other hand, to obtain an amplified product of N-terminal region of the acp gene, PCR was carried out using a genomic DNA of *Brevibacterium lactofermentum* 2256 strain as a template and synthetic DNAs of SEQ ID NOS. 56 and 57 as primers. SEQ ID NOS: 55 and 56 are complementary to each other. The PCR was carried out using KOD-plus- (TOYOBO) in such a way that one cycle of heat-retention at 94°C for 2 minutes was performed and then a cycle of denaturation at 94°C for 10 seconds, annealing at 55°C for 30 seconds and elongation at 68°C for 35 seconds was repeated 30 cycles for the N-terminal and C-terminal regions. Next, to obtain a fragment of an acp gene with a deletion of an internal sequence, the above-mentioned amplified products of the N-terminal and C-terminal regions of acp were mixed at an approximately equimolar concentration, and PCR was carried out using the mixture as templates and synthetic DNAs of SEQ ID NOS. 58 and 59 as primers, to thereby yield an amplified product of a mutation-introduced acp gene. The PCR was carried out using KOD-plus- (TOYOBO) in such a way that one cycle of heat-retention at 94°C for 2 minutes was performed and then a cycle of denaturation at 94°C for 10 seconds, annealing at 55°C for 30 seconds and elongation at 68°C for 60 seconds was repeated 30 cycles, to thereby obtain an amplified product of the mutation-introduced acp gene of interest.

The PCR product thus obtained was purified by a conventional procedure and then digested with XbaI, followed by insertion into XbaI site of pBS5T constructed in the above-mentioned Example 1 (C). Competent cells of *Escherichia coli* JM109 (Takara Bio Inc.) were used for transformation with this DNA and applied on an LB medium containing 100 µM IPTG, 40 µg/ml X-Gal, and 25 µg/ml kanamycin, followed by overnight culture. Subsequently, appeared white colonies were picked up, and single colonies were isolated, thereby transformants were obtained. Plasmids were extracted from the transformants, and a plasmid into which a PCR product of interest was inserted was named pBS5T::Δacp. Fig. 10 shows the construction procedures of pBS5T::Δacp.

### (B) Preparation of acp-disrupted strain

The pBS5T::Δacp obtained in the above-mentioned (A) does not include a region which enables autonomous replication in a cell of a coryneform bacterium, so when a coryneform bacterium is transformed with the plasmid, a strain in which the plasmid is integrated into a chromosome by homologous recombination appears at a very low frequency as a transformant. *Brevibacterium lactofermentum* 2256Δ(ldh, pta, ack, poxB) strain was transformed by using a high concentration of the plasmid pBS5T::Δacp by the electric pulse method, and applied on a CM-Dex medium containing 25 µg/ml kanamycin, followed by culture at 31.5°C for about 30 hours. In the strain grown on the medium, the kanamycin resistance gene and sacB gene derived from the plasmid are inserted on the genome, as a result of homologous recombination between the acp gene fragment on the plasmid and the acp gene on a genome of *Brevibacterium lactofermentum* 2256Δ(ldh, pta, ack, poxB) strain.

Next, the single crossover recombinant was cultured at 31.5°C overnight in CM-Dex liquid medium not containing kanamycin and, after suitable dilution, it was applied on 10% sucrose-containing Dex-S10 medium not containing kanamycin, followed by culture at 31.5°C for about 30 hours. As a result, about 50 strains, which were considered to become sucrose-insensitive due to elimination of the sacB gene by the second homologous recombination, were obtained.

The thus obtained strains include: a strain in which acp gene was replaced by the mutant type derived from pBS5T::Δacp; and a strain in which acp gene reverted to the wild type. Whether the acp gene is the mutant type or the wild type can be confirmed easily by directly subjecting a bacterial strain obtained through culture in a Dex-S10 agar medium to PCR and detecting the acp gene. Analysis of the acp gene by using primers (SEQ ID NOS: 54 and 57) for PCR amplification should result in a DNA fragment of 1.3 kb for the wild type and a DNA fragment of 1.0 kb for the mutant type having the deleted region. As a result of the analysis of the sucrose-insensitive strain by the above-mentioned method, a strain carrying only the mutant type gene was selected and named 2256Δ(ldh, pta, ack, poxB, acp) strain.

### EXAMPLE 7

### <7-1> Evaluation of culture of ach, pta, ack, poxB-disputed strain and pta, ack, poxB-disputed strain

*Brevibacterium lactofermentum* 2256Δ(ldh) strain, 2256Δ(ldh, pta-ack, poxB) strain, and 2256Δ (ldh, pta-ack, poxB, ach) strain were used for culture for producing succinic acid as described above. The bacterial cells of the 2256Δ(ldh) strain, 2256Δ(ldh, pta-ack, poxB) strain, and 2256Δ (ldh, pta-ack, poxB, ach) strain obtained by culturing them on a CM-Dex plate medium were inoculated into 3 ml of a seed medium B (10 g/L of glucose, 2.5 g/L of (NH₄)₂SO₄, 0.5 g/L of KH₂PO₄, 0.25 g/L of MgSO₄·7H₂O, 2 g/L of urea, 0.01 g/L of FeSO₄·7H₂O, 0.01g/L of MnSO₄·7H₂O, 50 µg/L of biotin, 100 µg/L of VB1·HCl, 15 mg/L of protocatechuic acid, 0.02 mg/L of CuSO₄, and 10 mg/L of CaCl₂, with pH 7.0 (KOH)). Shaking culture was performed in a test tube at 31.5°C for about 15 hours under an aerobic condition.

After that, 3 ml of a main medium B (70 g/L of glucose, 5 g/L of (NH₄)₂SO₄, 2 g/L of KH₂PO₄, 3 g/L of urea, 0.01 g/L of FeSO₄·7H₂O, 0.01 g/L of MnSO₄·7H₂O, 200 µg/L of biotin, 200 µg/L of VB1·HCl, 40 g/L of MOPS, and 50 g/L of MgCO₃, with pH 6.8 (NaOH)) was added into the tube. For preventing aeration, the succinic acid production culture was carried out while the tube was sealed hermetically with a silicon cap. The culture was performed by shaking at 31.5°C for about 24 hours and terminated before sugar in the medium was exhausted. After completion of the culture, the accumulation amounts of succinic acid and by-product acetic acid in the medium were analyzed by liquid chromatography after the medium had been suitably diluted. A column obtained by connecting two pieces of Shim-pack SCR-102H (Shimadzu) in series was used, and the sample was eluted at 40°C by using 5 mM p-toluene sulfonic acid. The eluent was neutralized by using 20 mM Bis-Tris aqueous solution containing 5 mM p-toluene sulfonic acid and 100 µM of EDTA. The succinic acid and acetic acid were each measured by determining the electric conductivity by means of CDD-10AD (Shimadzu). The obtained results are shown in Table 2.

The acetic acid in the 2256Δ(ldh, pta-ack, poxB) strain was drastically decreased as compared to the control 2256Δldh strain, and the acetic acid in the 2256Δ(ldh, pta, ack, ach, poxB) strain was further reduced, that is, reduced by about 40% in comparison with the 2256Δ(ldh, pta, ack, poxB) strain. These results revealed that eliminating or decreasing all or any one of the activities of poxB, pta-ack and ach simultaneously is effective for reducing acetic acid.

**Table 2. Production of succinic acid and acetic acid in the strains in which ACH, PTA, ACK and POXB are disrupted in combination**

| Strains | OD620(×51) | Consumed sugar (g/L) | Yield of succinic acid (%) | Acetic acid (/succinic acid %) |
|---|---|---|---|---|
| 2256Δldh | 0.342 | 31.8 | 57.3 | 11.9 |
| 2256Δ(ldh, pta, ack, poxB) | 0.382 | 36.6 | 56.2 | 8.4 |
| 2256Δ(ldh, pta, ack, poxB, ach) | 0.372 | 39.8 | 56.1 | 3.6 |

### <7-2> Evaluation of culture of the pta, ack, poxB, acp-disrupted strain

*Brevibacterium lactofermentum* 2256(ldh) strain, 2256Δ(ldh, pta, ack, poxB) strain, and 2256Δ(ldh, pta-ack, poxB, acp) strain were used for culture for producing succinic acid as follows. The bacterial cells of the 2256Δ(ldh) strain, 2256Δ(ldh, pta, ack, poxB) strain and 2256Δ(ldh, pta-ack, poxB, acp) strain obtained by culturing them on a CM-Dex plate medium were inoculated into 3 ml of the above-mentioned seed medium B. Shaking culture was performed in a test tube at 31.5°C for about 15 hours under an aerobic condition.
After that, 3 ml of the above-mentioned main medium B was added into the tube. For preventing aeration, the succinic acid production culture was carried out while the tube was sealed hermetically with a silicon cap. The culture was performed by shaking at 31.5°C for about 24 hours and terminated before sugar in the medium had been exhausted.
After completion of the culture, the accumulation amounts of succinic acid and by-product acetic acid in the culture medium were analyzed by liquid chromatography after the culture medium had been suitably diluted. A column obtained by connecting two pieces of Shim-pack SCR-102Hs (Shimadzu) in series was used, and the sample was eluted at 40°C by using 5 mM of p-toluene sulfonic acid. The eluent was neutralized by using 20 mM of Bis-Tris aqueous solution containing 5 mM of p-toluene sulfonic acid and 100 µM of EDTA. The succinic acid and by-product acetic acid were each measured by determining the electric conductivity by means of CDD-10AD (Shimadzu). The obtained results are shown in Table 3.

**Table 3. Production of succinic acid and acetic acid in the strains in which pta, ack, poxB and acp are disrupted in combination**

| Strains | OD620nm(×51) | Consumed sugar (g/L) | Yield of succinic acid (%) | Acetic acid (/succinic acid %) |
|---|---|---|---|---|
| 2256Δldh | 0.333 | 45.9 | 49.2 | 10.4 |
| 2256Δ(ldh, pta, ack, poxB) | 0.307 | 40.7 | 45.2 | 8.9 |
| 2256Δ(ldh, pta, ack, poxB, acp) | 0.341 | 44.3 | 46.0 | 8.8 |

As a result, ratio of acetic acid with respect to succinic acid was slightly reduced by decreasing or eliminating all of the activities of PTA, ACK and POXB, even when ACH activity was not decreased or eliminated as in the above-mentioned (C). On the other hand, decreasing or eliminating ACP activity had little influence on production of acetic acid and succinic acid.

### INDUSTRIAL APPLICABILITY

The present invention is useful for fermentative production of succinic acid. Succinic acid is useful as a raw material for biodegradable polymers, food products, drugs, cosmetics, and the like.

## Claims

1. A method for producing succinic acid, comprising:
allowing a coryneform bacterium having a succinic acid-producing ability or immobilized cells or disrupted cells thereof to act on an organic raw material in a reaction liquid containing carbonate ion, bicarbonate ion or carbon dioxide to produce and accumulate succinic acid in the reaction liquid; and
collecting succinic acid from the reaction liquid,
wherein said bacterium has been modified so that an activity of pyruvate oxidase is decreased and the generation of acetic acid as a by product is reduced.

2. The method according to claim 1, wherein the pyruvate oxidase is a protein as described in the following (A) or (B):
(A) a protein having an amino acid sequence of SEQ ID NO: 49; or
(B) a protein having an amino acid sequence of SEQ ID NO: 49 including substitution, deletion, insertion, or addition of one or several amino acids, and having a pyruvate oxidase activity.

3. The method according to claim 1 or 2, wherein the pyruvate oxidase activity is decreased by disruption of a pyruvate oxidase gene on a chromosome.

4. The method according to claim 3, wherein the pyruvate oxidase gene is a DNA as described in the following (a) or (b):
(a) a DNA comprising the nucleotide sequence of nucleotide numbers 996-2732 in SEQ ID NO: 48; or
(b) a DNA that hybridizes with the nucleotide sequence of nucleotide numbers 996-2732 in SEQ ID NO: 48 or a probe that can be prepared from the nucleotide sequence under stringent conditions, and encodes a protein having a pyruvate oxidase activity.

5. The method according to any one of claims 1 to 4, wherein said bacterium has been further modified so that activities of one or more of phosphotransacetylase, acetate kinase and acetyl-CoA Hydrolase are decreased.

6. The method according to any one of claims 1 to 5, wherein said bacterium has been further modified so that an activity of lactate dehydrogenase is decreased.

7. The method according to any one of claims 1 to 6, wherein said bacterium has been further modified so that an activity of pyruvate carboxylase is increased.

8. The method according to any one of claims 1 to 7, wherein the bacterium or the immobilized cells or the disrupted cells thereof is allowed to act on the organic raw material under anaerobic conditions.

9. A method for producing a succinic acid-containing polymer, comprising the steps of producing succinic acid by the method according to any one of claims 1 to 8 and polymerizing the obtained succinic acid.

10. A coryneform bacterium having a succinic acid-producing ability, wherein said
bacterium has been modified so that an activity of pyruvate oxidase is decreased as compared to Brevibacterium lactofermentum ATCC13869 or the Brevibacterium lactofermentum Δldh strain and, wherein said bacterium further has one or more modification
selected from the group consisting of:
(a) activities of one or more of phosphotransacetylase, acetate kinase and acetyl-CoA Hydrolase is/are decreased;
(b) an activity of lactate dehydrogenase is decreased; and
(c) an activity of pyruvate carboxylase is increased.

11. The coryneform bacterium according to claim 10, wherein the pyruvate oxidase is a protein as described in the following (A) or (B):
(A) a protein having an amino acid sequence of SEQ ID NO: 49; or
(B) a protein having an amino acid sequence of SEQ ID NO: 49 including substitution, deletion, insertion, or addition of one or several amino acids, and having a pyruvate oxidase activity.

12. The coryneform bacterium according to claim 10 or 11, wherein the pyruvate oxidase activity is decreased by disruption of a pyruvate oxidase gene on a chromosome.

13. The coryneform bacterium according to claim 12, wherein the pyruvate oxidase gene is a DNA as described in the following (a) or (b):
(a) a DNA comprising the nucleotide sequence of nucleotide numbers 996-2732 in SEQ ID NO: 48; or
(b) a DNA that hybridizes with the nucleotide sequence of nucleotide numbers 996-2732 in SEQ ID NO: 48 or a probe that can be prepared from the nucleotide sequence under stringent conditions, and encodes a protein having a pyruvate oxidase activity.

## Patentansprüche

1. Verfahren zur Herstellung von Bernsteinsäure, umfassend:
Ermöglichen, dass ein coryneformes Bakterium, das Bernsteinsäure-produzierende Fähigkeit hat, oder immobilisierte Zellen oder aufgebrochene Zellen davon, auf ein organisches Rohmaterial in einer Reaktionsflüssigkeit einwirken, die Carbonationen, Bicarbonationen oder Kohlendioxid enthält, um Bernsteinsäure in der Reaktionsflüssigkeit herzustellen und anzureichern; und
Gewinnen von Bernsteinsäure aus der Reaktionsflüssigkeit,
wobei das Bakterium so modifiziert wurde, dass eine Aktivität von Pyruvatoxidase verringert ist und die Erzeugung von Essigsäure als Nebenprodukt reduziert ist.

2. Verfahren nach Anspruch 1, wobei die Pyruvatoxidase ein wie unter den folgenden (A) oder (B) beschriebenes Protein ist:
(A) ein Protein mit einer Aminosäuresequenz von SEQ ID NO:49; oder
(B) ein Protein mit einer Aminosäuresequenz von SEQ ID NO:49, einschließlich Substitution, Deletion, Insertion oder Addition einer oder mehrerer Aminosäuren, und mit Pyruvatoxidase-Aktivität.

3. Verfahren nach Anspruch 1 oder 2, wobei die Pyruvatoxidase-Aktivität durch Störung eines Pyruvatoxidase-Gens auf einem Chromosom vermindert ist.

4. Verfahren nach Anspruch 3, wobei das Pyruvatoxidase-Gen eine wie unter den folgenden (a) oder (b) beschriebene DNA ist:
(a) eine DNA, umfassend die Nucleotidsequenz der Nucleotide Nummer 996-2732 in SEQ ID NO: 48; oder
(b) eine DNA, die mit der Nucleotidsequenz der Nucleotide Nummer 996-2732 in SEQ ID NO: 48 hybridisiert oder einer Sonde, die aus der Nucleotidsequenz unter stringenten Bedingungen hergestellt werden kann, und die ein Protein mit Pyruvatoxidase-Aktivität codiert.

5. Verfahren nach einem der Ansprüche 1 bis 4, wobei das Bakterium weiter modifiziert wurde, so dass Aktivitäten eines oder mehrerer aus Phosphotransacetylase, Acetatkinase und Acetyl-CoA-Hydrolase verringert sind.

6. Verfahren nach einem der Ansprüche 1 bis 5, wobei das Bakterium weiter modifiziert wurde, so dass eine Aktivität der Lactatdehydrogenase verringert ist.

7. Verfahren nach einem der Ansprüche 1 bis 6, wobei das Bakterium weiter modifiziert wurde, so dass eine Aktivität der Pyruvatcarboxylase erhöht ist.

8. Verfahren nach einem der Ansprüche 1 bis 7, wobei das Bakterium oder die immobilisierten Zellen oder die aufgebrochenen Zellen davon auf ein organisches Rohmaterial unter anaeroben Bedingungen einwirken können.

9. Verfahren zur Herstellung eines Bernsteinsäure enthaltenden Polymers, umfassend die Schritte des Herstellens von Bernsteinsäure durch das Verfahren nach einem der Ansprüche 1 bis 8 und Polymerisieren der erhaltenen Bernsteinsäure.

10. Coryneformes Bakterium, das eine Bernsteinsäure-produzierende Fähigkeit hat, wobei das Bakterium so modifiziert worden ist, dass eine Aktivität der Pyruvatoxidase verringert ist im Vergleich zu *Brevibacterium lactofermentum* ATCC13869 oder dem *Brevibacterium lactofermentum*-Δldh-Stamm, und wobei das Bakterium des Weiteren eine oder mehrere Modifikationen hat, ausgewählt aus der Gruppe bestehend aus:
(a) Aktivitäten einer oder mehrerer aus Phosphotransacetylase, Acetatkinase und Acetyl-CoA-Hydrolase sind verringert;
(b) eine Aktivität von Lactatdehydrogenase ist verringert; und
(c) eine Aktivität von Pyruvatcarboxylase ist erhöht.

11. Coryneformes Bakterium nach Anspruch 10, wobei die Pyruvatoxidase ein wie unter den folgenden (A) oder (B) beschriebenes Protein ist:
(A) ein Protein mit einer Aminosäuresequenz von SEQ ID NO: 49; oder
(B) ein Protein mit einer Aminosäuresequenz von SEQ ID NO:49, einschließlich Substitution, Deletion, Insertion, oder Addition einer oder mehrerer Aminosäuren, und mit Pyruvatoxidase-Aktivität.

12. Coryneformes Bakterium nach Anspruch 10 oder 11, wobei die Pyruvatoxidase-Aktivität durch Störung eines Pyruvatoxidase-Gens auf einem Chromosom vermindert ist.

13. Coryneformes Bakterium nach Anspruch 12, wobei das Pyruvatoxidase-Gen eine wie unter den folgenden (a) oder (b) beschriebene DNA ist:
(a) eine DNA, umfassend die Nucleotidsequenz der Nucleotide Nummer 996-2732 in SEQ ID NO:48; oder
(b) eine DNA, die mit der Nucleotidsequenz der Nucleotide Nummer 996-2732 in SEQ ID NO:48 hybridisiert oder einer Sonde, die aus der Nucleotidsequenz unter stringenten Bedingungen hergestellt werden kann, und die ein Protein mit Pyruvatoxidase-Aktivität codiert.

## Revendications

1. Méthode de production d'acide succinique, comprenant les étapes suivantes :
laisser une bactérie corynéforme ayant la capacité de produire de l'acide succinique ou des cellules immobilisées ou des cellules désintégrées de celle-ci agir sur une matière première organique dans un liquide réactionnel contenant l'ion carbonate, l'ion bicarbonate ou du dioxyde de carbone pour produire et
accumuler de l'acide succinique dans le liquide réactionnel ; et
recueillir l'acide succinique à partir du liquide réactionnel,
dans laquelle ladite bactérie a été modifiée de manière à ce qu'une activité de pyruvate oxydase soit diminuée et que la génération d'acide acétique en tant que sous-produit soit réduite.

2. Méthode selon la revendication 1, dans laquelle la pyruvate oxydase est une protéine telle que décrite en (A) ou (B) qui suivent :
(A) une protéine ayant une séquence d'acides aminés de SEQ ID NO : 49 ; ou
(B) une protéine ayant une séquence d'acides aminés de SEQ ID NO : 49 comprenant la substitution, la délétion, l'insertion, ou l'addition d'un ou plusieurs acides aminés, et ayant une activité pyruvate oxydase.

3. Méthode selon la revendication 1 ou 2, dans laquelle l'activité pyruvate oxydase est diminuée par la rupture d'un gène de pyruvate oxydase sur un chromosome.

4. Méthode selon la revendication 3, dans laquelle le gène pyruvate oxydase est un ADN tel que décrit en (a) ou (b) qui suivent :
(a) un ADN comprenant la séquence nucléotidique des nucléotides numéros 996 à 2732 dans SEQ ID NO : 48 ; ou
(b) un ADN qui s'hybride à la séquence nucléotidique des nucléotides numéros 996 à 2732 dans SEQ ID NO : 48 ou à une sonde qui peut être préparée à partir de la séquence nucléotidique dans des conditions stringentes, et qui code pour une protéine ayant une activité pyruvate oxydase.

5. Méthode selon l'une quelconque des revendications 1 à 4, dans laquelle ladite bactérie a été en outre modifiée de manière à ce que l'activité de l'une ou de plusieurs de la phosphotransacétylase, de l'acétate kinase et l'acétyl-CoA hydrolase soit diminuée.

6. Méthode selon l'une quelconque des revendications 1 à 5, dans laquelle ladite bactérie a été en outre modifiée de manière à ce qu'une activité de lactate déshydrogénase soit diminuée.

7. Méthode selon l'une quelconque des revendications 1 à 6, dans laquelle ladite bactérie a été en outre modifiée de manière à ce qu'une activité de pyruvate carboxylase soit augmentée.

8. Méthode selon l'une quelconque des revendications 1 à 7, dans laquelle la bactérie ou les cellules immobilisées ou les cellules désintégrées de celle-ci sont laissées agir sur la matière première organique dans des conditions anaérobies.

9. Méthode de production d'un polymère contenant de l'acide succinique, comprenant les étapes consistant à produire de l'acide succinique par la méthode selon l'une quelconque des revendications 1 à 8 et à polymériser l'acide succinique obtenu.

10. Bactérie corynéforme ayant une capacité de produire de l'acide succinique, ladite bactérie ayant été modifiée de manière à ce qu'une activité de pyruvate oxydase soit diminuée par rapport à *Brevibacterium lactofermentum* ATCC 13869 ou à la souche Δldh de *Brevibacterium lactofermentum* et, ladite bactérie comportant en outre une ou plusieurs modifications choisies dans le groupe constitué par :
(a) la diminution de l'activité de l'une ou de plusieurs de la phosphotransacétylase, de l'acétate kinase et l'acétyl-CoA hydrolase ;
(b) la diminution d'une activité de lactate déshydrogénase ; et
(c) l'augmentation d'une activité de pyruvate carboxylase.

11. Bactérie corynéforme selon la revendication 10, dans laquelle la pyruvate oxydase est une protéine telle que décrite en (A) ou (B) qui suivent :
(A) une protéine ayant une séquence d'acides aminés de SEQ ID NO : 49 ; ou
(B) une protéine ayant une séquence d'acides aminés de SEQ ID NO : 49 comprenant la substitution, la délétion, l'insertion, ou l'addition d'un ou plusieurs acides aminés, et ayant une activité pyruvate oxydase.

12. Bactérie corynéforme selon la revendication 10 ou 11, dans laquelle l'activité pyruvate oxydase est diminuée par la rupture d'un gène de pyruvate oxydase sur un chromosome.

13. Bactérie corynéforme selon la revendication 12, dans laquelle le gène de pyruvate oxydase est un ADN tel que décrit en (a) ou (b) qui suivent :
(a) un ADN comprenant la séquence nucléotidique des nucléotides numéros 996 à 2732 dans SEQ ID NO : 48 ; ou
(b) un ADN qui s'hybride à la séquence nucléotidique des nucléotides numéros 996 à 2732 dans SEQ ID NO : 48 ou à une sonde qui peut être préparée à partir de la séquence nucléotidique dans des conditions stringentes, et qui code pour une protéine ayant une activité pyruvate oxydase.


### SEQUENCE LISTING

<110> Ajinomoto Co., Inc
   Mitsubishi Chemical Corporation
<120> SUCCINIC ACID-PRODUCING BACTERIUM AND PROCESS FOR PRODUCING SUCCINIC ACID
<130> C438-C5047
<150> JP 2004-150672
   <151> 2004-05-20
<160> 61
<110>PatentIn version 3. 1
<210> 1
   <211> 24
   <212> DNA
   <213> Artificial
<220>
   <223> primer
<400> 1
   cgggatcctt tttaacccat caca 24
<210> 2
   <211> 29
   <212> DNA
   <213> Artificial
<220>
   <223> primer
<400> 2
   gaagatcttc aaaaggttag gaatacggt 29
<210> 3
   <211> 23
   <212> DNA
   <213> Artificial
<220>
   <223> primer
<400> 3 23
   ccttttgaag atcgaccagt tgg 23
<210> 4
   <211> 44
   <212> DNA
   <213> Artificial
<220>
   <223> primer
<400> 4 44
   tacctggaat gctgttttcc cagggatcgc agtggtgagt aacc 44
<210> 5
   <211> 28
   <212> DNA
   <213> Artificial
<220>
   <223> primer
<400> 5
   cctgggaaaa cagcattcca ggtattag 28
<210> 6
   <211> 23
   <212> DNA
   <213> Artificial
<220>
   <223> primer
<400> 6 23
   tgcaggtcga ctctagagga tcc 23
<210> 7
   <211> 20
   <212> DNA
   <213> Artificial
<220>
   <223> primer
<400> 7
   cactgcacgg ccctgcgaac 20
<210> 8
   <211> 42
   <212> DNA
   <213> Artificial
<220>
   <223> primer
<400> 8
   cgccaactag gcgccaaaaa ttcctgattt ccctaaccgg ac 42
<210> 9
   <211> 42
   <212> DNA
   <213> Artificial
<220>
   <223> primer
<400> 9
   gtccggttag ggaaatcagg aatttttggc gcctagttgg cg 42
<210> 10
   <211> 20
   <212> DNA
   <213> Artificial
<220>
   <223> primer
<400> 10
   tgtgggcctt cggcgaggac 20
<210> 11
   <211> 26
   <212> DNA
   <213> Artificial
<220>
   <223> primer
<400> 11 26
   gagtcgaccg caccccattt ttcata 26
<210> 12
   <211> 28
   <212> DNA
   <213> Artificial
<220>
   <223> primer
<400> 12 28
   tggtcgacgt gaatgctcgg cgggatcc 28
<210> 13
   <211> 24
   <212> DNA
   <213> Artificial
<220>
   <223> primer
<400> 13 24
   cttccatctt cctcatggtg ctgc 24
<210> 14
   <211> 44
   <212> DNA
   <213> Artificial
<220>
   <223> primer
<400> 14
   ccaggagagc taagcgaact ccattagctg cgtcctcctg cctg 44
<210> 15
   <211> 44
   <212> DNA
   <213> Artificial
<220>
   <223> primer
<400> 15
   caggcaggag gacgcagcta atggagttcg cttagctctc ctgg 44
<210> 16
   <211> 31
   <212> DNA
   <213> Artificial
<220>
   <223> primer
<400> 16
   gcgtctagac ctttaggagt gcgatgtccc c 31
<210> 17
   <211> 33
   <212> DNA
   <213> Artificial
<220>
   <223> primer
<400> 17
   gcgtctagac gactgtgctg ttaacccgaa ccc 33
<210> 18
   <211> 33
   <212> DNA
   <213> Artificial
<220>
   <223> primer
<400> 18 33
   gcgtctagag agttaggccc ttagaagcga ttc 33
<210> 19
   <211> 24
   <212> DNA
   <213> Artificial
<220>
   <223> primer
<400> 19 24
   gctcaaagcg tggaattgag atcg 24
<210> 20
   <211> 42
   <212> DNA
   <213> Artificial
<220>
   <223> primer
<400> 20 42
   ccaggagagc taagcgaact ttcggcgctc atgactggtt cg 42
<210> 21
   <211> 42
   <212> DNA
   <213> Artificial
<220>
   <223> primer
<400> 21 42
   cgaaccagtc atgagcgccg aaagttcgct tagctctcct gg 42
<210> 22
   <211> 30
   <212> DNA
   <213> Artificial
<220>
   <223> primer
<400> 22 30
   gcgtctagag tacgcaaggc ggacgaacgc 30
<210> 23
   <211> 23
   <212> DNA
   <213> Artificial
<220>
   <223> primer
<400> 23 23
   gccttgatat cttcccgcaa acc 23
<210> 24
   <211> 47
   <212> DNA
   <213> Artificial
<220>
   <223> primer
<400> 24 47
   cttgtggtcc tggaaacaca caccgaagtg aatttcgcag agattgc 47
<210> 25
   <211> 48
   <212> DNA
   <213> Artificial
<220>
   <223> primer
<400> 25
   cgcaatctct gcgaaattca cttcggtgtg tgtttccagg accacaag 48
<210> 26
   <211> 22
   <212> DNA
   <213> Artificial
<220>
   <223> primer
<400> 26
   ggtttctcgg ggtctaaacc gg 22
<210> 27
   <211> 33
   <212> DNA
   <213> Artificial
<220>
   <223> primer
<400> 27
   gggaatctag accacgccaa tggaaatttc tcc 33
<210> 28
   <211> 35
   <212> DNA
   <213> Artificial
<220>
   <223> primer
<400> 28
   gggaatctag acgtgacaag atctggcgaa atcgc 35
<210> 29
   <211> 23
   <212> DNA
   <213> Artificial
<220>
   <223> primer
<400> 29
   gccttgatat cttcccgcaa ace 23
<210> 30
   <211> 47
   <212> DNA
   <213> Artificial
<220>
   <223> primer
<400> 30
   cttgtggtcc tggaaacaca caccgaagtg aatttcgcag agattgc 47
<210> 31
   <211> 48
   <212> DNA
   <213> Artificial
<220>
   <223> primer
<400> 31
   cgcaatctct gcgaaattca cttcggtgtg tgtttccagg accacaag 48
<210> 32
   <211> 22
   <212> DNA
   <213> Artificial
<220>
   <223> primer
<400> 32
   ggtttctcgg ggtctaaacc gg 22
<210> 33
   <211> 33
   <212> DNA
   <213> Artificial
<220>
   <223> primer
<400> 33
   gggaatctag accacgccaa tggaaatttc tcc 33
<210> 34
   <211> 35
   <212> DNA
   <213> Artificial
<220>
   <223> primer
<400> 34
   gggaatctag acgtgacaag atctggcgaa atcgc 35
<210> 35
   <211> 24
   <212> DNA
   <213> Artificial
<220>
   <223> primer
<400> 35
   gcttctgcgc aaagcaagcc tccg 24
<210> 36
   <211> 50
   <212> DNA
   <213> Artificial
<220>
   <223> primer
<400> 36
   gtccgattac ctgaggaggt attcccatga aggcataagt tttttcttgg 50
<210> 37
   <211> 50
   <212> DNA
   <213> Artificial
<220>
   <223> primer
<400> 37
   ccaagaaaaa acttatgcct tcatgggaat acctcctcag gtaatcggac 50
<210> 38
   <211> 26
   <212> DNA
   <213> Artificial
<220>
   <223> primer
<400> 38 26
   ggtcatgtgc atggttttct cattgc 26
<210> 39
   <211> 33
   <212> DNA
   <213> Artificial
<220>
   <223> primer
<400> 39 33
   ggcctctaga cctgcaccga tcaggatgag tgg 33
<210> 40
   <211> 35
   <212> DNA
   <213> Artificial
<220>
   <223> primer
<400> 40 35
   gcgctctaga ctcaacaaga gcacgcgcag tcacc 35
<210> 41
   <211> 2014
   <212> DNA
   <213> Bacillus subtilis
<220>
   <221> CDS
   <222> (464).. (1885)
   <223>
<400> 41
   gatccttttt aacccatcac atatacctgc cgttcactat tatttagtga aatgagatat    60
   tatgatattt tctgaattgt gattaaaaag gcaactttat gcccatgcaa cagaaactat   120
   aaaaaataca gagaatgaaa agaaacagat agatttttta gttctttagg cccgtagtct   180
   gcaaatcctt ttatgatttt ctatcaaaca aaagaggaaa atagaccagt tgcaatccaa   240
   acgagagtct aatagaatga ggtcgaaaag taaatcgcgc gggtttgtta ctgataaagc   300
   aggcaagacc taaaatgtgt aaagggcaaa gtgtatactt tggcgtcacc ccttacatat   360
   tttaggtctt tttttattgt gcgtaactaa cttgccatct tcaaacagga gggctggaag   420
   aagcagaccg ctaacacagt acataaaaaa ggagacatga acg atg aac atc aaa     475
                                                   Met Asn Ile Lys
                                                   1
   aag ttt gca aaa caa gca aca gta tta acc ttt act acc gca ctg ctg     523
   Lys Phe Ala Lys Gln Ala Thr Val Leu Thr Phe Thr Thr Ala Leu Leu
   5                   10                  15                  20
   gca gga ggc gca act caa gcg ttt gcg aaa gaa acg aac caa aag cca     571
   Ala Gly Gly Ala Thr Gln Ala Phe Ala Lys Glu Thr Asn Gln Lys Pro
                   25                  30                  35
   tat aag gaa aca tac ggc att tcc cat att aca cgc cat gat atg ctg     619
   Tyr Lys Glu Thr Tyr Gly Ile Ser His Ile Thr Arg His Asp Met Leu
               40                  45                  50
   caa atc cct gaa cag caa aaa aat gaa aaa tat caa gtt cct gaa ttc     667
   Gln Ile Pro Glu Gln Gln Lys Asn Glu Lys Tyr Gln Val Pro Glu Phe
           55                  60                  65
   gat tcg tcc aca att aaa aat atc tct tct gca aaa ggc ctg gac gtt     715
   Asp Ser Ser Thr Ile Lys Asn Ile Ser Ser Ala Lys Gly Leu Asp Val
       70                  75                  80
   tgg gac agc tgg cca tta caa aac gct gac ggc act gtc gca aac tat     763
   Trp Asp Ser Trp Pro Leu Gln Asn Ala Asp Gly Thr Val Ala Asn Tyr
   85                  90                  95                  100
   cac ggc tac cac atc gtc ttt gca tta gcc gga gat cct aaa aat gcg     811
   His Gly Tyr His Ile Val Phe Ala Leu Ala Gly Asp Pro Lys Asn Ala
                   105                 110                 115
   gat gac aca tcg att tac atg ttc tat caa aaa gtc ggc gaa act tct     859
   Asp Asp Thr Ser Ile Tyr Met Phe Tyr Gln Lys Val Gly Glu Thr Ser
   120 125 130
   att gac agc tgg aaa aac gct ggc cgc gtc ttt aaa gac agc gac aaa 907
   Ile Asp Ser Trp Lys Asn Ala Gly Arg Val Phe Lys Asp Ser Asp Lys
   135 140 145
   ttc gat gca aat gat tct atc cta aaa gac caa aca caa gaa tgg tca 955
   Phe Asp Ala Asn Asp Ser Ile Leu Lys Asp Gln Thr Gln Glu Trp Ser
   150 155 160
   ggt tca gcc aca ttt aca tct gac gga aaa atc cgt tta ttc tac act 1003
   Gly Ser Ala Thr Phe Thr Ser Asp Gly Lys Ile Arg Leu Phe Tyr Thr
   165 170 175 180
   gat ttc tcc ggt aaa cat tac ggc aaa caa aca ctg aca act gca caa 1051
   Asp Phe Ser Gly Lys His Tyr Gly Lys Gln Thr Leu Thr Thr Ala Gln
   185 190 195
   gtt aac gta tca gca tca gac agc tct ttg aac atc aac ggt gta gag 1099
   Val Asn Val Ser Ala Ser Asp Ser Ser Leu Asn Ile Asn Gly Val Glu
   200 205 210
   gat tat aaa tca atc ttt gac ggt gac gga aaa acg tat caa aat gta 1147
   Asp Tyr Lys Ser Ile Phe Asp Gly Asp Gly Lys Thr Tyr Gln Asn Val
   215 220 225
   cag cag ttc atc gat gaa ggc aac tac agc tca ggc gac aac cat acg 1195
   Gln Gln Phe Ile Asp Glu Gly Asn Tyr Ser Ser Gly Asp Asn His Thr
   230 235 240
   ctg aga gat cct cac tac gta gaa gat aaa ggc cac aaa tac tta gta 1243
   Leu Arg Asp Pro His Tyr Val Glu Asp Lys Gly His Lys Tyr Leu Val
   245 250 255 260
   ttt gaa gca aac act gga act gaa gat ggc tac caa ggc gaa gaa tct 1291
   Phe Glu Ala Asn Thr Gly Thr Glu Asp Gly Tyr Gln Gly Glu Glu Ser
   265 270 275
   tta ttt aac aaa gca tac tat ggc aaa agc aca tca ttc ttc cgt caa 1339
   Leu Phe Asn Lys Ala Tyr Tyr Gly Lys Ser Thr Ser Phe Phe Arg Gln
   280 285 290
   gaa agt caa aaa ctt ctg caa agc gat aaa aaa cgc acg gct gag tta 1387
   Glu Ser Gln Lys Leu Leu Gln Ser Asp Lys Lys Arg Thr Ala Glu Leu
   295 300 305
   gca aac ggc gct ctc ggt atg att gag cta aac gat gat tac aca ctg 1435
   Ala Asn Gly Ala Leu Gly Met Ile Glu Leu Asn Asp Asp Tyr Thr Leu
   310 315 320
   aaa aaa gtg atg aaa ccg ctg att gca tct aac aca gta aca gat gaa 1483
   Lys Lys Val Met Lys Pro Leu Ile Ala Ser Asn Thr Val Thr Asp Glu
   325 330 335 340
   att gaa cgc gcg aac gtc ttt aaa atg aac ggc aaa tgg tac ctg ttc 1531
   Ile Glu Arg Ala Asn Val Phe Lys Met Asn Gly Lys Trp Tyr Leu Phe
   345 350 355
   act gac tcc cgc gga tca aaa atg acg att gac ggc att acg tct aac 1579
   Thr Asp Ser Arg Gly Ser Lys Met Thr Ile Asp Gly Ile Thr Ser Asn
                   360                 365                 370
   gat att tac atg ctt ggt tat gtt tct aat tct tta act ggc cca tac      1627
   Asp Ile Tyr Met Leu Gly Tyr Val Ser Asn Ser Leu Thr Gly Pro Tyr
               375                 380                 385
   aag ccg ctg aac aaa act ggc ctt gtg tta aaa atg gat ctt gat cct      1675
   Lys Pro Leu Asn Lys Thr Gly Leu Val Leu Lys Met Asp Leu Asp Pro
           390                 395                 400
   aac gat gta acc ttt act tac tca cac ttc gct gta cct caa gcg aaa      1723
   Asn Asp Val Thr Phe Thr Tyr Ser His Phe Ala Val Pro Gln Ala Lys
   405                 410                 415                 420
   gga aac aat gtc gtg att aca agc tat atg aca aac aga gga ttc tac      1771
   Gly Asn Asn Val Val Ile Thr Ser Tyr Met Thr Asn Arg Gly Phe Tyr
                   425                 430                 435
   gca gac aaa caa tca acg ttt gcg cca agc ttc ctg ctg aac atc aaa      1819
   Ala Asp Lys Gln Ser Thr Phe Ala Pro Ser Phe Leu Leu Asn Ile Lys
               440                 445                 450
   ggc aag aaa aca tct gtt gtc aaa gac agc atc ctt gaa caa gga caa      1867
   Gly Lys Lys Thr Ser Val Val Lys Asp Ser Ile Leu Glu Gln Gly Gln
           455                 460                 465
   tta aca gtt aac aaa taa aaacgcaaaa gaaaatgccg atatcctatt             1915
   Leu Thr Val Asn Lys
       470
   ggcattttct tttatttctt atcaacataa aggtgaatcc catatgaact atataaaagc   1975
   aggcaaatgg ctaaccgtat tcctaacctt ttgaagatc                           2014
<210> 42
   <211> 473
   <212> PRT
   <213> Bacillus subtilis
<400> 42
   Met Asn Ile Lys Lys Phe Ala Lys Gln Ala Thr Val Leu Thr Phe Thr
   1               5                   10                  15
   Thr Ala Leu Leu Ala Gly Gly Ala Thr Gln Ala Phe Ala Lys Glu Thr
               20                  25                  30
   Asn Gln Lys Pro Tyr Lys Glu Thr Tyr Gly Ile Ser His Ile Thr Arg
           35                  40                  45
   His Asp Met Leu Gln Ile Pro Glu Gln Gln Lys Asn Glu Lys Tyr Gln
       50                  55                  60
   Val Pro Glu Phe Asp Ser Ser Thr Ile Lys Asn Ile Ser Ser Ala Lys
   65                  70                  75                  80
   Gly Leu Asp Val Trp Asp Ser Trp Pro Leu Gln Asn Ala Asp Gly Thr
                   85                  90                  95
   Val Ala Asn Tyr His Gly Tyr His Ile Val Phe Ala Leu Ala Gly Asp
   100 105 110
   Pro Lys Asn Ala Asp Asp Thr Ser Ile Tyr Met Phe Tyr Gln Lys Val
   115 120 125
   Gly Glu Thr Ser Ile Asp Ser Trp Lys Asn Ala Gly Arg Val Phe Lys
   130 135 140
   Asp Ser Asp Lys Phe Asp Ala Asn Asp Ser Ile Leu Lys Asp Gln Thr
   145 150 155 160
   Gln Glu Trp Ser Gly Ser Ala Thr Phe Thr Ser Asp Gly Lys Ile Arg
   165 170 175
   Leu Phe Tyr Thr Asp Phe Ser Gly Lys His Tyr Gly Lys Gln Thr Leu
   180 185 190
   Thr Thr Ala Gln Val Asn Val Ser Ala Ser Asp Ser Ser Leu Asn Ile
   195 200 205
   Asn Gly Val Glu Asp Tyr Lys Ser Ile Phe Asp Gly Asp Gly Lys Thr
   210 215 220
   Tyr Gln Asn Val Gln Gln Phe Ile Asp Glu Gly Asn Tyr Ser Ser Gly
   225 230 235 240
   Asp Asn His Thr Leu Arg Asp Pro His Tyr Val Glu Asp Lys Gly His
   245 250 255
   Lys Tyr Leu Val Phe Glu Ala Asn Thr Gly Thr Glu Asp Gly Tyr Gln
   260 265 270
   Gly Glu Glu Ser Leu Phe Asn Lys Ala Tyr Tyr Gly Lys Ser Thr Ser
   275 280 285
   Phe Phe Arg Gln Glu Ser Gln Lys Leu Leu Gln Ser Asp Lys Lys Arg
   290 295 300
   Thr Ala Glu Leu Ala Asn Gly Ala Leu Gly Met Ile Glu Leu Asn Asp
   305 310 315 320
   Asp Tyr Thr Leu Lys Lys Val Met Lys Pro Leu Ile Ala Ser Asn Thr
   325 330 335
   Val Thr Asp Glu Ile Glu Arg Ala Asn Val Phe Lys Met Asn Gly Lys
   340 345 350
   Trp Tyr Leu Phe Thr Asp Ser Arg Gly Ser Lys Met Thr Ile Asp Gly
   355 360 365
   Ile Thr Ser Asn Asp Ile Tyr Met Leu Gly Tyr Val Ser Asn Ser Leu
   370 375 380
   Thr Gly Pro Tyr Lys Pro Leu Asn Lys Thr Gly Leu Val Leu Lys Met
   385 390 395 400
   Asp Leu Asp Pro Asn Asp Val Thr Phe Thr Tyr Ser His Phe Ala Val
   405 410 415
   Pro Gln Ala Lys Gly Asn Asn Val Val Ile Thr Ser Tyr Met Thr Asn
   420 425 430
   Arg Gly Phe Tyr Ala Asp Lys Gln Ser Thr Phe Ala Pro Ser Phe Leu
   435 440 445
   Leu Asn Ile Lys Gly Lys Lys Thr Ser Val Val Lys Asp Ser Ile Leu
   450 455 460
   Glu Gln Gly Gln Leu Thr Val Asn Lys
   465 470
<210> 43
   <211> 2820
   <212> DNA
   <213> Corynebacterium glutamicum
<220>
   <221> CDS
   <222> (898).. (1854)
   <223>
<400> 43
   tgcagaatta tgcaagatgc gccgcaacaa aacgcgatcg gccaaggtca aagtggtcaa      60
   tgtaatgacc gaaaccgctg cgatgaaact tatccacggc ggtaaaaacc tctcaattag     120
   gagcttgacc tcattaatac tgtgctgggt taattcgccg gtgatcagca gcgcgccgta     180
   ccccaaggtg ccgacactaa tgcccgcgat cgtctccttc ggtccaaaat tcttctgccc     240
   aatcagccgg atttgggtgc gatgcctgat caatcccaca accgtggtgg tcaacgtgat     300
   ggcaccagtt gcgatgtggg tggcgttgta aattttcctg gatacccgcc ggttggttct     360
   ggggaggatc gagtggattc ccgtcgctgc cgcatgcccc accgcttgta aaacagccag     420
   gttagcagcc gtaacccacc acggtttcgg caacaatgac ggcgagagag cccaccacat     480
   tgcgatttcc gctccgataa agccagcgcc catatttgca gggaggattc gcctgcggtt     540
   tggcgacatt cggatccccg gaactagctc tgcaatgacc tgcgcgccga gggaggcgag     600
   gtgggtggca ggttttagtg cgggtttaag cgttgccagg cgagtggtga gcagagacgc     660
   tagtctgggg agcgaaacca tattgagtca tcttggcaga gcatgcacaa ttctgcaggg     720
   cataggttgg ttttgctcga tttacaatgt gattttttca acaaaaataa cacttggtct     780
   gaccacattt tcggacataa tcgggcataa ttaaaggtgt aacaaaggaa tccgggcaca     840
   agctcttgct gattttctga gctgctttgt gggttgtccg gttagggaaa tcaggaa        897
   gtg gga tcg aaa atg aaa gaa acc gtc ggt aac aag att gtc ctc att       945
   Val Gly Ser Lys Met Lys Glu Thr Val Gly Asn Lys Ile Val Leu Ile
   1               5                   10                  15
   ggc gca gga gat gtt gga gtt gca tac gca tac gca ctg atc aac cag       993
   Gly Ala Gly Asp Val Gly Val Ala Tyr Ala Tyr Ala Leu Ile Asn Gln
               20                  25                  30
   ggc atg gca gat cac ctt gcg atc atc gac atc gat gaa aag aaa ctc      1041
   Gly Met Ala Asp His Leu Ala Ile Ile Asp Ile Asp Glu Lys Lys Leu
           35                  40                  45
   gaa ggc aac gtc atg gac tta aac cat ggt gtt gtg tgg gcc gat tcc      1089
   Glu Gly Asn Val Met Asp Leu Asn His Gly Val Val Trp Ala Asp Ser
       50                  55                  60
   cgc acc cgc gtc acc aag ggc acc tac gct gac tgc gaa gac gca gcc      1137
   Arg Thr Arg Val Thr Lys Gly Thr Tyr Ala Asp Cys Glu Asp Ala Ala
   65                  70                  75                  80
   atg gtt gtc att tgt gcc ggc gca gcc caa aag cca ggc gag acc cgc     1185
   Met Val Val Ile Cys Ala Gly Ala Ala Gln Lys Pro Gly Glu Thr Arg
                   85                  90                  95
   ctc cag ctg gtg gac aaa aac gtc aag att atg aaa tcc atc gtc ggc     1233
   Leu Gln Leu Val Asp Lys Asn Val Lys Ile Met Lys Ser Ile Val Gly
               100                 105                 110
   gat gtc atg gac agc gga ttc gac ggc atc ttc ctc gtg gcg tcc aac     1281
   Asp Val Met Asp Ser Gly Phe Asp Gly Ile Phe Leu Val Ala Ser Asn
           115                 120                 125
   cca gtg gat atc ctg acc tac gca gtg tgg aaa ttc tcc ggc ttg gaa     1329
   Pro Val Asp Ile Leu Thr Tyr Ala Val Trp Lys Phe Ser Gly Leu Glu
       130                 135                 140
   tgg aac cgc gtg atc ggc tcc gga act gtc ctg gac tcc gct cga ttc     1377
   Trp Asn Arg Val Ile Gly Ser Gly Thr Val Leu Asp Ser Ala Arg Phe
   145                 150                 155                 160
   cgc tac atg ctg ggc gaa ctc tac gaa gtg gca cca agc tcc gtc cac     1425
   Arg Tyr Met Leu Gly Glu Leu Tyr Glu Val Ala Pro Ser Ser Val His
                   165                 170                 175
   gcc tac atc atc ggc gaa cac ggc gac act gaa ctt cca gtc ctg tcc     1473
   Ala Tyr Ile Ile Gly Glu His Gly Asp Thr Glu Leu Pro Val Leu Ser
               180                 185                 190
   tcc gcg acc atc gca ggc gta tcg ctt agc cga atg ctg gac aaa gac     1521
   Ser Ala Thr Ile Ala Gly Val Ser Leu Ser Arg Met Leu Asp Lys Asp
           195                 200                 205
   cca gag ctt gag ggc cgt cta gag aaa att ttc gaa gac acc cgc gac     1569
   Pro Glu Leu Glu Gly Arg Leu Glu Lys Ile Phe Glu Asp Thr Arg Asp
       210                 215                 220
   gct gcc tat cac att atc gac gcc aag ggc tcc act tcc tac ggc atc     1617
   Ala Ala Tyr His Ile Ile Asp Ala Lys Gly Ser Thr Ser Tyr Gly Ile
   225                 230                 235                 240
   ggc atg ggt ctt gct cgc atc acc cgc gca atc ctg cag aac caa gac     1665
   Gly Met Gly Leu Ala Arg Ile Thr Arg Ala Ile Leu Gln Asn Gln Asp
                   245                 250                 255
   gtt gca gtc cca gtc tct gca ctg ctc cac ggt gaa tac ggt gag gaa     1713
   Val Ala Val Pro Val Ser Ala Leu Leu His Gly Glu Tyr Gly Glu Glu
               260                 265                 270
   gac atc tac atc ggc acc cca gct gtg gtg aac cgc cga ggc atc cgc     1761
   Asp Ile Tyr Ile Gly Thr Pro Ala Val Val Asn Arg Arg Gly Ile Arg
           275                 280                 285
   cgc gtt gtc gaa cta gaa atc acc gac cac gag atg gaa cgc ttc aag     1809
   Arg Val Val Glu Leu Glu Ile Thr Asp His Glu Met Glu Arg Phe Lys
       290                 295                 300
   cat tcc gca aat acc ctg cgc gaa att cag aag cag ttc ttc taa         1854
   His Ser Ala Asn Thr Leu Arg Glu Ile Gln Lys Gln Phe Phe
   305                 310                 315
   atctttggcg cctagttggc gacgcaagtg tttcattgga acacttgcgc tgccaacttt   1914
   ttggtttacg ggcacaatga aactgttgga tggaatttag agtgtttgta gcttaaggag   1974
   ctcaaatgaa tgagtttgac caggacattc tccaggagat caagactgaa ctcgacgagt   2034
   taattctaga acttgatgag gtgacacaaa ctcacagcga ggccatcggg caggtctccc   2094
   caacccatta cgttggtgcc cgcaacctca tgcattacgc gcatcttcgc accaaagacc   2154
   tccgtggcct gcagcaacgc ctctcctctg tgggagctac ccgcttgact accaccgaac   2214
   cagcagtgca ggcccgcctc aaggccgccc gcaatgttat cggagctttc gcaggtgaag   2274
   gcccacttta tccaccctca gatgtcgtcg atgccttcga agatgccgat gagattctcg   2334
   acgagcacgc cgaaattctc cttggcgaac ccctaccgga tactccatcc tgcatcatgg   2394
   tcaccctgcc caccgaagcc gccaccgaca ttgaacttgt ccgtggcttc gccaaaagcg   2454
   gcatgaatct agctcgcatc aactgtgcac acgacgatga aaccgtctgg aagcagatga   2514
   tcgacaacgt ccacaccgtt gcagaagaag ttggccggga aatccgcgtc agcatggacc   2574
   tcgccggacc aaaagtacgc accggcgaaa tcgccccagg cgcagaagta ggtcgcgcac   2634
   gagtaacccg cgacgaaacc ggaaaagtac tgacgcccgc aaaactgtgg atcaccgccc   2694
   acggctccga accagtccca gcccccgaaa gcctgcccgg tcgccccgct ctgccgattg   2754
   aagtcacccc agaatggttc gacaaactag aaatcggcag cgtcatcaac gtcccagaca   2814
   cccgcg                                                              2820
<210> 44
   <211> 318
   <212> PRT
   <213> Corynebacterium glutamicum
<400> 44
   Val Gly Ser Lys Met Lys Glu Thr Val Gly Asn Lys Ile Val Leu Ile
   1               5                   10                  15
   Gly Ala Gly Asp Val Gly Val Ala Tyr Ala Tyr Ala Leu Ile Asn Gln
               20                  25                  30
   Gly Met Ala Asp His Leu Ala Ile Ile Asp Ile Asp Glu Lys Lys Leu
           35                  40                  45
   Glu Gly Asn Val Met Asp Leu Asn His Gly Val Val Trp Ala Asp Ser
       50                  55                  60
   Arg Thr Arg Val Thr Lys Gly Thr Tyr Ala Asp Cys Glu Asp Ala Ala
   65                  70                  75                  80
   Met Val Val Ile Cys Ala Gly Ala Ala Gln Lys Pro Gly Glu Thr Arg
                   85                  90                  95
   Leu Gln Leu Val Asp Lys Asn Val Lys Ile Met Lys Ser Ile Val Gly
               100                 105                 110
   Asp Val Met Asp Ser Gly Phe Asp Gly Ile Phe Leu Val Ala Ser Asn
           115                 120                 125
   Pro Val Asp Ile Leu Thr Tyr Ala Val Trp Lys Phe Ser Gly Leu Glu
       130                 135                 140
   Trp Asn Arg Val Ile Gly Ser Gly Thr Val Leu Asp Ser Ala Arg Phe
   145                 150                 155                 160
   Arg Tyr Met Leu Gly Glu Leu Tyr Glu Val Ala Pro Ser Ser Val His
   165 170 175
   Ala Tyr Ile Ile Gly Glu His Gly Asp Thr Glu Leu Pro Val Leu Ser
   180 185 190
   Ser Ala Thr Ile Ala Gly Val Ser Leu Ser Arg Met Leu Asp Lys Asp
   195 200 205
   Pro Glu Leu Glu Gly Arg Leu Glu Lys Ile Phe Glu Asp Thr Arg Asp
   210 215 220
   Ala Ala Tyr His Ile Ile Asp Ala Lys Gly Ser Thr Ser Tyr Gly Ile
   225 230 235 240
   Gly Met Gly Leu Ala Arg Ile Thr Arg Ala Ile Leu Gln Asn Gln Asp
   245 250 255
   Val Ala Val Pro Val Ser Ala Leu Leu His Gly Glu Tyr Gly Glu Glu
   260 265 270
   Asp Ile Tyr Ile Gly Thr Pro Ala Val Val Asn Arg Arg Gly Ile Arg
   275 280 285
   Arg Val Val Glu Leu Glu Ile Thr Asp His Glu Met Glu Arg Phe Lys
   290 295 300
   His Ser Ala Asn Thr Leu Arg Glu Ile Gln Lys Gln Phe Phe
   305 310 315
<210> 45
   <211> 4200
   <212> DNA
   <213> Corynebacterium glutamicum
<220>
   <221> CDS
   <222> (956)..(1942)
   <223>
<220>
   <221> CDS
   <222> (1945).. (3135)
   <223>
<400> 45
   cctgctggac ctgcaccgac aacggcaaca cgcaaagggc gagacatata aagttcgatt 60
   ccttaaaggg gttctaaaaa atgtggagta tgtgagcggg ggttccactt gtagattcga 120
   ctcctatcgg ggtgcgactg ctaatggtgc cctgctatca accctccatg atacgtggta 180
   agtgcagact aataaaggcc agtcggggag tattgggggc tttgctgggg tcagatttgt 240
   cacgctgcgc gctttcatag accccattaa tggggggtga agagctgtaa agtaccgcta 300
   aaaactttgc aaagggtgct tcgcaacttg taaccgctcc gtattgtttt ctacggcaat 360
   aagcatttgt gctgctcaaa gcgtggaatt gagatcggtt tgaaaattac aaaataaaac 420
   tttgcaaacc gggctgtacg caaggcggac gaacgctaaa ctatgtaaga aatcacaacc    480
   tcccctcatt agtgccagga ggcacaagcc tgaagtgtca tcaatgagaa ggttcaggct    540
   gaaattagaa aggcgatgta tgtctgacac accgacctca gctctgatca ccacggtcaa    600
   ccgcagcttc gatggattcg atttggaaga agtagcagca gaccttggag ttcggctcac    660
   ctacctgccc gacgaagaac tagaagtatc caaagttctc gcggcggacc tcctcgctga    720
   ggggccagct ctcatcatcg gtgtaggaaa cacgtttttc gacgcccagg tcgccgctgc    780
   cctcggcgtc ccagtgctac tgctggtaga caagcaaggc aagcacgttg ctcttgctcg    840
   cacccaggta aacaatgccg gcgcagttgt tgcagcagca tttaccgctg aacaagagcc    900
   aatgccggat aagctgcgca aggctgtgcg caaccacagc aacctcgaac cagtc atg     958
                                                                Met
                                                                1
   agc gcc gaa ctc ttt gaa aac tgg ctg ctc aag cgc gca cgc gca gag     1006
   Ser Ala Glu Leu Phe Glu Asn Trp Leu Leu Lys Arg Ala Arg Ala Glu
               5                  10                  15
   cac tcc cac att gtg ctg cca gaa ggt gac gac gac cgc atc ttg atg     1054
   His Ser His Ile Val Leu Pro Glu Gly Asp Asp Asp Arg Ile Leu Met
           20                  25                  30
   gct gcc cac cag ctg ctt gat caa gac atc tgt gac atc acg atc ctg     1102
   Ala Ala His Gln Leu Leu Asp Gln Asp Ile Cys Asp Ile Thr Ile Leu
       35                  40                  45
   ggc gat cca gta aag atc aag gag cgc gct acc gaa ctt ggc ctg cac     1150
   Gly Asp Pro Val Lys Ile Lys Glu Arg Ala Thr Glu Leu Gly Leu His
   50                  55                  60                  65
   ctt aac act gca tac ctg gtc aat ccg ctg aca gat cct cgc ctg gag     1198
   Leu Asn Thr Ala Tyr Leu Val Asn Pro Leu Thr Asp Pro Arg Leu Glu
                   70                  75                  80
   gaa ttc gcc gaa caa ttc gcg gag ctg cgc aag tca aag agc gtc act     1246
   Glu Phe Ala Glu Gln Phe Ala Glu Leu Arg Lys Ser Lys Ser Val Thr
               85                  90                  95
   atc gat gaa gcc cgc gaa atc atg aag gat att tcc tac ttc ggc acc     1294
   Ile Asp Glu Ala Arg Glu Ile Met Lys Asp Ile Ser Tyr Phe Gly Thr
           100                 105                 110
   atg atg gtc cac aac ggc gac gcc gac gga atg gta tcc ggt gca gca     1342
   Met Met Val His Asn Gly Asp Ala Asp Gly Met Val Ser Gly Ala Ala
       115                 120                 125
   aac acc acc gca cac acc att aag cca agc ttc cag atc atc aaa act     1390
   Asn Thr Thr Ala His Thr Ile Lys Pro Ser Phe Gln Ile Ile Lys Thr
   130                 135                 140                 145
   gtt cca gaa gca tcc gtc gtt tct tcc atc ttc ctc atg gtg ctg cgc     1438
   Val Pro Glu Ala Ser Val Val Ser Ser Ile Phe Leu Met Val Leu Arg
                   150                 155                 160
   ggg cga ctg tgg gca ttc ggc gac tgt gct gtt aac ccg aac cca act     1486
   Gly Arg Leu Trp Ala Phe Gly Asp Cys Ala Val Asn Pro Asn Pro Thr
               165                 170                 175
   gct gaa cag ctt ggt gaa atc gcc gtt gtg tca gca aaa act gca gca     1534
   Ala Glu Gln Leu Gly Glu Ile Ala Val Val Ser Ala Lys Thr Ala Ala
           180                 185                 190
   caa ttt ggc att gat cct cgc gta gcc atc ttg tcc tac tcc act ggc     1582
   Gln Phe Gly Ile Asp Pro Arg Val Ala Ile Leu Ser Tyr Ser Thr Gly
       195                 200                 205
   aac tcc ggc gga ggc tca gat gtg gat cgc gcc atc gac gct ctt gca     1630
   Asn Ser Gly Gly Gly Ser Asp Val Asp Arg Ala Ile Asp Ala Leu Ala
   210                 215                 220                 225
   gaa gca cgc cga ctt aac cca gaa cta tgc gtc gat gga cca ctt cag     1678
   Glu Ala Arg Arg Leu Asn Pro Glu Leu Cys Val Asp Gly Pro Leu Gln
                   230                 235                 240
   ttc gac gcc gcc gtc gac ccg ggt gtg gcg cgc aag aag atg cca gac     1726
   Phe Asp Ala Ala Val Asp Pro Gly Val Ala Arg Lys Lys Met Pro Asp
               245                 250                 255
   tct gac gtc gct ggc cag gca aat gtg ttt atc ttc cct gac ctg gaa     1774
   Ser Asp Val Ala Gly Gln Ala Asn Val Phe Ile Phe Pro Asp Leu Glu
           260                 265                 270
   gcc gga aac atc ggc tac aaa act gca caa cgc acc ggt cac gcc ctg     1822
   Ala Gly Asn Ile Gly Tyr Lys Thr Ala Gln Arg Thr Gly His Ala Leu
       275                 280                 285
   gca gtt ggt ccg att ctg cag ggc cta aac aaa cca gtc aac gac ctt     1870
   Ala Val Gly Pro Ile Leu Gln Gly Leu Asn Lys Pro Val Asn Asp Leu
   290                 295                 300                 305
   tcc cgt ggc gca aca gtc cct gac atc gtc aac aca gta gcc atc aca     1918
   Ser Arg Gly Ala Thr Val Pro Asp Ile Val Asn Thr Val Ala Ile Thr
                   310                 315                 320
   gca att cag gca gga gga cgc agc ta atg gca ttg gca ctt gtt ttg      1965
   Ala Ile Gln Ala Gly Gly Arg Ser    Met Ala Leu Ala Leu Val Leu
               325                    330                 335
   aac tcc ggt tca tct tcc atc aaa ttc cag ctg gtc aac ccc gaa aac     2013
   Asn Ser Gly Ser Ser Ser Ile Lys Phe Gln Leu Val Asn Pro Glu Asn
               340                 345                 350
   tct gcc atc gac gag cca tat gtt tct ggt ctt gtg gag cag att ggt     2061
   Ser Ala Ile Asp Glu Pro Tyr Val Ser Gly Leu Val Glu Gln Ile Gly
           355                 360                 365
   gag cca aac ggc cgc atc gta ctc aaa ata gag ggt gaa aaa tat acc     2109
   Glu Pro Asn Gly Arg Ile Val Leu Lys Ile Glu Gly Glu Lys Tyr Thr
       370                 375                 380
   cta gag aca ccc atc gca gat cac tcc gaa ggc cta aac ctg gcg ttc     2157
   Leu Glu Thr Pro Ile Ala Asp His Ser Glu Gly Leu Asn Leu Ala Phe
   385                 390                 395                 400
   gat ctc atg gac cag cac aac tgt ggt cct tcc caa ctg gaa atc acc     2205
   Asp Leu Met Asp Gln His Asn Cys Gly Pro Ser Gln Leu Glu Ile Thr
                   405                 410                 415
   gca gtt gga cac cgc gtg gtc cac ggc gga atc ttg ttc tcc gca ccg     2253
   Ala Val Gly His Arg Val Val His Gly Gly Ile Leu Phe Ser Ala Pro
   420 425 430
   gaa ctt atc act gat gaa atc gtg gaa atg atc cgc gat ctc att cca 2301
   Glu Leu Ile Thr Asp Glu Ile Val Glu Met Ile Arg Asp Leu Ile Pro
   435 440 445
   ctc gca cca ctg cac aac cct gca aac gtt gac ggc att gat gtt gct 2349
   Leu Ala Pro Leu His Asn Pro Ala Asn Val Asp Gly Ile Asp Val Ala
   450 455 460
   cga aaa att ctc ccc gat gtc cca cac gta gct gtc ttt gac acc ggt 2397
   Arg Lys Ile Leu Pro Asp Val Pro His Val Ala Val Phe Asp Thr Gly
   465 470 475 480
   ttc ttc cac tca ctt cca cca gca gct gcg ctg tat gcc atc aac aag 2445
   Phe Phe His Ser Leu Pro Pro Ala Ala Ala Leu Tyr Ala Ile Asn Lys
   485 490 495
   gat gtc gca gct gaa cac gga atc agg cgc tat ggt ttc cac ggc acc 2493
   Asp Val Ala Ala Glu His Gly Ile Arg Arg Tyr Gly Phe His Gly Thr
   500 505 510
   tcc cat gaa ttt gtg tcc aag cgc gtg gtg gaa att ctg gaa aag ccc 2541
   Ser His Glu Phe Val Ser Lys Arg Val Val Glu Ile Leu Glu Lys Pro
   515 520 525
   acc gaa gac atc aac acc atc acc ttc cac ctg ggc aac ggc gca tcc 2589
   Thr Glu Asp Ile Asn Thr Ile Thr Phe His Leu Gly Asn Gly Ala Ser
   530 535 540
   atg gct gct gtt caa ggt ggc cgt gcg gta gat act tcc atg ggt atg 2637
   Met Ala Ala Val Gln Gly Gly Arg Ala Val Asp Thr Ser Met Gly Met
   545 550 555 560
   aca cct ctc gcg ggc ctt gtc atg ggt acc cga agc ggt gac att gat 2685
   Thr Pro Leu Ala Gly Leu Val Met Gly Thr Arg Ser Gly Asp Ile Asp
   565 570 575
   cca ggt atc gtc ttc cac ctt tcc cgc acc gct ggc atg agc atc gat 2733
   Pro Gly Ile Val Phe His Leu Ser Arg Thr Ala Gly Met Ser Ile Asp
   580 585 590
   gag atc gat aat ctg ctg aac aaa aag tcg ggt gta aag gga ctt tcc 2781
   Glu Ile Asp Asn Leu Leu Asn Lys Lys Ser Gly Val Lys Gly Leu Ser
   595 600 605
   ggt gtt aat gat ttc cgt gaa ctg cgg gaa atg atc gac aac aat gat 2829
   Gly Val Asn Asp Phe Arg Glu Leu Arg Glu Met Ile Asp Asn Asn Asp
   610 615 620
   caa gat gcc tgg tcc gcg tac aac att tac ata cac caa ctc cgc cgc 2877
   Gln Asp Ala Trp Ser Ala Tyr Asn Ile Tyr Ile His Gln Leu Arg Arg
   625 630 635 640
   tac ctc ggt tcc tac atg gtg gca ctg gga cgg gta gac acc atc gtg 2925
   Tyr Leu Gly Ser Tyr Met Val Ala Leu Gly Arg Val Asp Thr Ile Val
   645 650 655
   ttc acc gcc ggt gtc ggt gaa aat gcc cag ttt gtc cgt gag gat gcc 2973
   Phe Thr Ala Gly Val Gly Glu Asn Ala Gln Phe Val Arg Glu Asp Ala
   660 665 670
   ttg gca ggt ttg gaa atg tac gga att gag atc gat cca gag cgt aac 3021
   Leu Ala Gly Leu Glu Met Tyr Gly Ile Glu Ile Asp Pro Glu Arg Asn
   675 680 685
   gca ttg cca aac gat ggt cct cga ttg att tcc acc gat gcc tcc aag 3069
   Ala Leu Pro Asn Asp Gly Pro Arg Leu Ile Ser Thr Asp Ala Ser Lys
   690 695 700
   gtg aag gtg ttt gtt att cca act aat gaa gag tta gct atc gct agg 3117
   Val Lys Val Phe Val Ile Pro Thr Asn Glu Glu Leu Ala Ile Ala Arg
   705 710 715 720
   tac gcg gtg aag ttc gct tagctctcct ggttaggatc caccacaaat 3165
   Tyr Ala Val Lys Phe Ala
   725
   cgctctgatc agcggttttg tggtggattt ttgcgttttt aaggggtgaa actgcacgga 3225
   tccaccacag atcccagttt tcctttggaa cgtggtggat ccttgccctg gagcttcaca 3285
   ggaatcgctt gttggcccct agacctcttg gggttgcgaa ttttcgtccc caccgaacat 3345
   taaaaggccg gttttggtcg aaaatttgct ctaacacctt gctattatgc gaatattcgt 3405
   tccatttcat cgaattccag caacccgtaa cgagaagttg aacaggaaac ctgcagtaac 3465
   cccgcagaaa tcacatcagc cccaattgtc ccaaaagtaa ctcccccaga atcgcttcta 3525
   agggcctaac tcgcccaaag tcaaactagg ggacatcgca ctcctaaagg cccttaaatc 3585
   gccacctacc aaatagcccc aagtcaaaac agctagaacc aactcagtgg ccgcacggca 3645
   ttcgccatat ccacaagtgc gtaacggtgg tgcgggaacg gtgcagaacg tgcctgaatg 3705
   cggagtgcct cggagatgcc ggtgcgcagg cctttttggg agaacgggta ttcaaacaaa 3765
   gggttcgcgg aggcggaagc tttgagtttt cgctctcgaa gccagctgag gcctgccgac 3825
   atgatggcaa ttttgatttg gttgaagcgg ggttcgtttg tggggatttc ggtgagtcgg 3885
   cgggcagccc gtcggatgcg ggattcactc aaattggagc tgaccagcaa caagatggtg 3945
   gtgagggtgg ccattcggta gtgggtggat gattggggga gtttgtctag ggcttggact 4005
   gcgagttcga tttggttttc ggccatgagt tggcgggcga gcccgaacgc ggaggacacg 4065
   gtggtggggt tggttgccca gacaagtgcg tagaggcgca gtgaatggaa gcggactacg 4125
   tgtgggtcgc tggagatgtg ggaccaggtg tcgctgagtg attcgaaggc ggaggcgtcg 4185
   aggtcttcga aatct 4200
<210> 46
   <211> 329
   <212> PRT
   <213> Corynebacterium glutamicum
<400> 46
   Met Ser Ala Glu Leu Phe Glu Asn Trp Leu Leu Lys Arg Ala Arg Ala
   1 5 10 15
   Glu His Ser His Ile Val Leu Pro Glu Gly Asp Asp Asp Arg Ile Leu
   20 25 30
   Met Ala Ala His Gln Leu Leu Asp Gln Asp Ile Cys Asp Ile Thr Ile
   35 40 45
   Leu Gly Asp Pro Val Lys Ile Lys Glu Arg Ala Thr Glu Leu Gly Leu
   50 55 60
   His Leu Asn Thr Ala Tyr Leu Val Asn Pro Leu Thr Asp Pro Arg Leu
   65 70 75 80
   Glu Glu Phe Ala Glu Gln Phe Ala Glu Leu Arg Lys Ser Lys Ser Val
   85 90 95
   Thr Ile Asp Glu Ala Arg Glu Ile Met Lys Asp Ile Ser Tyr Phe Gly
   100 105 110
   Thr Met Met Val His Asn Gly Asp Ala Asp Gly Met Val Ser Gly Ala
   115 120 125
   Ala Asn Thr Thr Ala His Thr Ile Lys Pro Ser Phe Gln Ile Ile Lys
   130 135 140
   Thr Val Pro Glu Ala Ser Val Val Ser Ser Ile Phe Leu Met Val Leu
   145 150 155 160
   Arg Gly Arg Leu Trp Ala Phe Gly Asp Cys Ala Val Asn Pro Asn Pro
   165 170 175
   Thr Ala Glu Gln Leu Gly Glu Ile Ala Val Val Ser Ala Lys Thr Ala
   180 185 190
   Ala Gln Phe Gly Ile Asp Pro Arg Val Ala Ile Leu Ser Tyr Ser Thr
   195 200 205
   Gly Asn Ser Gly Gly Gly Ser Asp Val Asp Arg Ala Ile Asp Ala Leu
   210 215 220
   Ala Glu Ala Arg Arg Leu Asn Pro Glu Leu Cys Val Asp Gly Pro Leu
   225 230 235 240
   Gln Phe Asp Ala Ala Val Asp Pro Gly Val Ala Arg Lys Lys Met Pro
   245 250 255
   Asp Ser Asp Val Ala Gly Gln Ala Asn Val Phe Ile Phe Pro Asp Leu
   260 265 270
   Glu Ala Gly Asn Ile Gly Tyr Lys Thr Ala Gln Arg Thr Gly His Ala
   275 280 285
   Leu Ala Val Gly Pro Ile Leu Gln Gly Leu Asn Lys Pro Val Asn Asp
   290 295 300
   Leu Ser Arg Gly Ala Thr Val Pro Asp Ile Val Asn Thr Val Ala Ile
   305 310 315 320
   Thr Ala Ile Gln Ala Gly Gly Arg Ser
   325
<210> 47
   <211> 397
   <212> PRT
   <213> Corynebacterium glutamicum
<400> 47
   Met Ala Leu Ala Leu Val Leu Asn Ser Gly Ser Ser Ser Ile Lys Phe
   1 5 10 15
   Gln Leu Val Asn Pro Glu Asn Ser Ala Ile Asp Glu Pro Tyr Val Ser
   20 25 30
   Gly Leu Val Glu Gln Ile Gly Glu Pro Asn Gly Arg Ile Val Leu Lys
   35 40 45
   Ile Glu Gly Glu Lys Tyr Thr Leu Glu Thr Pro Ile Ala Asp His Ser
   50 55 60
   Glu Gly Leu Asn Leu Ala Phe Asp Leu Met Asp Gln His Asn Cys Gly
   65 70 75 80
   Pro Ser Gln Leu Glu Ile Thr Ala Val Gly His Arg Val Val His Gly
   85 90 95
   Gly Ile Leu Phe Ser Ala Pro Glu Leu Ile Thr Asp Glu Ile Val Glu
   100 105 110
   Met Ile Arg Asp Leu Ile Pro Leu Ala Pro Leu His Asn Pro Ala Asn
   115 120 125
   Val Asp Gly Ile Asp Val Ala Arg Lys Ile Leu Pro Asp Val Pro His
   130 135 140
   Val Ala Val Phe Asp Thr Gly Phe Phe His Ser Leu Pro Pro Ala Ala
   145 150 155 160
   Ala Leu Tyr Ala Ile Asn Lys Asp Val Ala Ala Glu His Gly Ile Arg
   165 170 175
   Arg Tyr Gly Phe His Gly Thr Ser His Glu Phe Val Ser Lys Arg Val
   180 185 190
   Val Glu Ile Leu Glu Lys Pro Thr Glu Asp Ile Asn Thr Ile Thr Phe
   195 200 205
   His Leu Gly Asn Gly Ala Ser Met Ala Ala Val Gln Gly Gly Arg Ala
   210 215 220
   Val Asp Thr Ser Met Gly Met Thr Pro Leu Ala Gly Leu Val Met Gly
   225 230 235 240
   Thr Arg Ser Gly Asp Ile Asp Pro Gly Ile Val Phe His Leu Ser Arg
   245 250 255
   Thr Ala Gly Met Ser Ile Asp Glu Ile Asp Asn Leu Leu Asn Lys Lys
   260 265 270
   Ser Gly Val Lys Gly Leu Ser Gly Val Asn Asp Phe Arg Glu Leu Arg
   275 280 285
   Glu Met Ile Asp Asn Asn Asp Gln Asp Ala Trp Ser Ala Tyr Asn Ile
   290 295 300
   Tyr Ile His Gln Leu Arg Arg Tyr Leu Gly Ser Tyr Met Val Ala Leu
   305 310 315 320
   Gly Arg Val Asp Thr Ile Val Phe Thr Ala Gly Val Gly Glu Asn Ala
   325 330 335
   Gln Phe Val Arg Glu Asp Ala Leu Ala Gly Leu Glu Met Tyr Gly Ile
   340 345 350
   Glu Ile Asp Pro Glu Arg Asn Ala Leu Pro Asn Asp Gly Pro Arg Leu
   355 360 365
   Ile Ser Thr Asp Ala Ser Lys Val Lys Val Phe Val Ile Pro Thr Asn
   370 375 380
   Glu Glu Leu Ala Ile Ala Arg Tyr Ala Val Lys Phe Ala
   385 390 395
<210> 48
   <211> 3780
   <212> DNA
   <213> Corynebacterium glutamicum
<220>
   <221> CDS
   <222> (996)..(2735)
   <223>
<400> 48
   taatgaggaa aaccgaaccc caccagaaga attccaacag cgcaccacca atgatcgggc    60
   ctgccgcagc gccaagaatt gccacggaac cccaaatacc aattgcagtg ttgcgctcac   120
   gctcatcctc aaacgtaatg cggatcagag ccaaggttgc aggcatcatc gttgccgcac   180
   cgatgccaag gaaagctctc gcagcaacaa gagcccacgc agttggagca aacgcagcac   240
   caagtgaagc gattccgaaa atgctcaagc ccatgaggaa catccggcgg tggccgattt   300
   tgtcacccaa agtgccggta cccaaaagaa ggcccgccat gagcagggga tatgcgttga   360
   tgatccacaa cgcttgggtt tcggtggctg cgagctgttc acgcagcaga gggagtgcgg   420
   tgtagagaat cgagttgtct acaccgatca gaaagagacc accgctgata acggcgagga   480
   aagcccaacg ttgggttttc gtaggcgctt gcgcctgtaa ggtttctgaa gtcatggatc   540
   gtaactgtaa cgaatggtcg gtacagttac aactcttttg ttggtgtttt agaccacggc   600
   gctgtgtggc gatttaagac gtcggaaatc gtaggggact gtcagcgtgg gtcgggttct   660
   ttgaggcgct tagaggcgat tctgtgaggt cactttttgt ggggtcgggg tctaaatttg   720
   gccagttttc gaggcgacca gacaggcgtg cccacgatgt ttaaataggc gatcggtggg   780
   catctgtgtt tggtttcgac gggctgaaac caaaccagac tgcccagcaa cgacggaaat   840
   cccaaaagtg ggcatccctg tttggtaccg agtacccacc cgggcctgaa actccctggc   900
   aggcgggcga agcgtggcaa caactggaat ttaagagcac aattgaagtc gcaccaagtt   960
   aggcaacaca atagccataa cgttgaggag ttcag atg gca cac agc tac gca     1013
                                          Met Ala His Ser Tyr Ala
                                          1               5
   gaa caa tta att gac act ttg gaa gct caa ggt gtg aag cga att tat    1061
   Glu Gln Leu Ile Asp Thr Leu Glu Ala Gln Gly Val Lys Arg Ile Tyr
                   10                  15                  20
   ggt ttg gtg ggt gac agc ctt aat ccg atc gtg gat gct gtc cgc caa    1109
   Gly Leu Val Gly Asp Ser Leu Asn Pro Ile Val Asp Ala Val Arg Gln
               25                  30                  35
   tca gat att gag tgg gtg cac gtt cga aat gag gaa gcg gcg gcg ttt    1157
   Ser Asp Ile Glu Trp Val His Val Arg Asn Glu Glu Ala Ala Ala Phe
   40 45 50
   gca gcc ggt gcg gaa tcg ttg atc act ggg gag ctg gca gta tgt gct 1205
   Ala Ala Gly Ala Glu Ser Leu Ile Thr Gly Glu Leu Ala Val Cys Ala
   55 60 65 70
   gct tct tgt ggt cct gga aac aca cac ctg att cag ggt ctt tat gat 1253
   Ala Ser Cys Gly Pro Gly Asn Thr His Leu Ile Gln Gly Leu Tyr Asp
   75 80 85
   tcg cat cga aat ggt gcg aag gtg ttg gcc atc gct agc cat att ccg 1301
   Ser His Arg Asn Gly Ala Lys Val Leu Ala Ile Ala Ser His Ile Pro
   90 95 100
   agt gcc cag att ggt tcg acg ttc ttc cag gaa acg cat ccg gag att 1349
   Ser Ala Gln Ile Gly Ser Thr Phe Phe Gln Glu Thr His Pro Glu Ile
   105 110 115
   ttg ttt aag gaa tgc tct ggt tac tgc gag atg gtg aat ggt ggt gag 1397
   Leu Phe Lys Glu Cys Ser Gly Tyr Cys Glu Met Val Asn Gly Gly Glu
   120 125 130
   cag ggt gaa cgc att ttg cat cac gcg att cag tcc acc atg gcg ggt 1445
   Gln Gly Glu Arg Ile Leu His His Ala Ile Gln Ser Thr Met Ala Gly
   135 140 145 150
   aaa ggt gtg tcg gtg gta gtg att cct ggt gat atc gct aag gaa gac 1493
   Lys Gly Val Ser Val Val Val Ile Pro Gly Asp Ile Ala Lys Glu Asp
   155 160 165
   gca ggt gac ggt act tat tcc aat tcc act att tct tct ggc act cct 1541
   Ala Gly Asp Gly Thr Tyr Ser Asn Ser Thr Ile Ser Ser Gly Thr Pro
   170 175 180
   gtg gtg ttc ccg gat cct act gag gct gca gcg ctg gtg gag gcg att 1589
   Val Val Phe Pro Asp Pro Thr Glu Ala Ala Ala Leu Val Glu Ala Ile
   185 190 195
   aac aac gct aag tct gtc act ttg ttc tgc ggt gcg ggc gtg aag aat 1637
   Asn Asn Ala Lys Ser Val Thr Leu Phe Cys Gly Ala Gly Val Lys Asn
   200 205 210
   gct cgc gcg cag gtg ttg gag ttg gcg gag aag att aaa tca ccg atc 1685
   Ala Arg Ala Gln Val Leu Glu Leu Ala Glu Lys Ile Lys Ser Pro Ile
   215 220 225 230
   ggg cat gcg ctg ggt ggt aag cag tac atc cag cat gag aat ccg ttt 1733
   Gly His Ala Leu Gly Gly Lys Gln Tyr Ile Gln His Glu Asn Pro Phe
   235 240 245
   gag gtc ggc atg tct ggc ctg ctt ggt tac ggc gcc tgc gtg gat gcg 1781
   Glu Val Gly Met Ser Gly Leu Leu Gly Tyr Gly Ala Cys Val Asp Ala
   250 255 260
   tcc aat gag gcg gat ctg ctg att cta ttg ggt acg gat ttc cct tat 1829
   Ser Asn Glu Ala Asp Leu Leu Ile Leu Leu Gly Thr Asp Phe Pro Tyr
   265 270 275
   tct gat ttc ctt cct aaa gac aac gtt gcc cag gtg gat atc aac ggt 1877
   Ser Asp Phe Leu Pro Lys Asp Asn Val Ala Gln Val Asp Ile Asn Gly
       280                 285                 290
   gcg cac att ggt cga cgt acc acg gtg aag tat ccg gtg acc ggt gat      1925
   Ala His Ile Gly Arg Arg Thr Thr Val Lys Tyr Pro Val Thr Gly Asp
   295                 300                 305                 310
   gtt gct gca aca atc gaa aat att ttg cct cat gtg aag gaa aaa aca      1973
   Val Ala Ala Thr Ile Glu Asn Ile Leu Pro His Val Lys Glu Lys Thr
                   315                 320                 325
   gat cgt tcc ttc ctt gat cgg atg ctc aag gca cac gag cgt aag ttg      2021
   Asp Arg Ser Phe Leu Asp Arg Met Leu Lys Ala His Glu Arg Lys Leu
               330                 335                 340
   agc tcg gtg gta gag acg tac aca cat aac gtc gag aag cat gtg cct      2069
   Ser Ser Val Val Glu Thr Tyr Thr His Asn Val Glu Lys His Val Pro
           345                 350                 355
   att cac cct gaa tac gtt gcc tct att ttg aac gag ctg gcg gat aag      2117
   Ile His Pro Glu Tyr Val Ala Ser Ile Leu Asn Glu Leu Ala Asp Lys
       360                 365                 370
   gat gcg gtg ttt act gtg gat acc ggc atg tgc aat gtg tgg cat gcg      2165
   Asp Ala Val Phe Thr Val Asp Thr Gly Met Cys Asn Val Trp His Ala
   375                 380                 385                 390
   agg tac atc gag aat ccg gag gga acg cgc gac ttt gtg ggt tca ttc      2213
   Arg Tyr Ile Glu Asn Pro Glu Gly Thr Arg Asp Phe Val Gly Ser Phe
                   395                 400                 405
   cgc cac ggc acg atg gct aat gcg ttg cct cat gcg att ggt gcg caa      2261
   Arg His Gly Thr Met Ala Asn Ala Leu Pro His Ala Ile Gly Ala Gln
               410                 415                 420
   agt gtt gat cga aac cgc cag gtg atc gcg atg tgt ggc gat ggt ggt      2309
   Ser Val Asp Arg Asn Arg Gln Val Ile Ala Met Cys Gly Asp Gly Gly
           425                 430                 435
   ttg ggc atg ctg ctg ggt gag ctt ctg acc gtt aag ctg cac caa ctt      2357
   Leu Gly Met Leu Leu Gly Glu Leu Leu Thr Val Lys Leu His Gln Leu
       440                 445                 450
   ccg ctg aag gct gtg gtg ttt aac aac agt tct ttg ggc atg gtg aag      2405
   Pro Leu Lys Ala Val Val Phe Asn Asn Ser Ser Leu Gly Met Val Lys
   455                 460                 465                 470
   ttg gag atg ctc gtg gag gga cag cca gaa ttt ggt act gac cat gag      2453
   Leu Glu Met Leu Val Glu Gly Gln Pro Glu Phe Gly Thr Asp His Glu
                   475                 480                 485
   gaa gtg aat ttc gca gag att gcg gcg gct gcg ggt atc aaa tcg gta      2501
   Glu Val Asn Phe Ala Glu Ile Ala Ala Ala Ala Gly Ile Lys Ser Val
               490                 495                 500
   cgc atc acc gat ccg aag aaa gtt cgc gag cag cta gct gag gca ttg      2549
   Arg Ile Thr Asp Pro Lys Lys Val Arg Glu Gln Leu Ala Glu Ala Leu
           505                 510                 515
   gca tat cct gga cct gta ctg atc gat atc gtc acg gat cct aat gcg      2597
   Ala Tyr Pro Gly Pro Val Leu Ile Asp Ile Val Thr Asp Pro Asn Ala
   520                    525                    530
   ctg tcg atc cca cca acc atc acg tgg gaa cag gtc atg gga ttc agc    2645
   Leu Ser Ile Pro Pro Thr Ile Thr Trp Glu Gln Val Met Gly Phe Ser
   535                 540                 545                 550
   aag gcg gcc acc cga acc gtc ttt ggt gga gga gta gga gcg atg atc    2693
   Lys Ala Ala Thr Arg Thr Val Phe Gly Gly Gly Val Gly Ala Met Ile
                   555                 560                 565
   gat ctg gcc cgt tcg aac ata agg aat att cct act cca tga            2735
   Asp Leu Ala Arg Ser Asn Ile Arg Asn Ile Pro Thr Pro
               570                 575
   tgattgatac acctgctgtt ctcattgacc gcgagcgctt aactgccaac atttccagga  2795
   tggcagctca cgccggtgcc catgagattg ccctgcgtcc gcatgtgaaa acgcacaaaa  2855
   tcattgaaat tgcgcagatg caggtcgacg ccggtgcccg agggatcacc tgcgcaacca  2915
   ttggcgaggc ggaaattttt gccggcgcag gttttacgga catctttatt gcatatccgc  2975
   tgtatctaac cgatcatgca gtgcaacgcc tgaacgcgat ccccggagaa atttccattg  3035
   gcgtggattc ggtagagatg gcacaggcga cggcgggttt gcgggaagat atcaaggctc  3095
   tgattgaagt ggattcggga catcgtagaa gtggagtcac ggcgactgct tcagaattga  3155
   gtcagatccg cgaggcgctg ggcagcaggt atgcaggagt gtttactttt cctgggcatt  3215
   cttatggccc gggaaatggt gagcaggcag cagctgatga gcttcaggct ctaaacaaca  3275
   gcgtccagcg acttgctggc ggcctgactt ctggcggttc ctcgccgtct gcgcagttta  3335
   cagacgcaat cgatgagatg cgaccaggcg tgtatgtgtt taacgattcc cagcagatca  3395
   cctcgggagc atgcactgag aagcaggtgg caatgacggt gctgtctact gtggtcagcc  3455
   gaaatgtgtc agatcgtcgg atcattttgg atgcgggatc caaaatcctc agcactgata  3515
   aaccagcatg gattgatggc aatggttttg ttctggggaa tcctgaagcc cgaatctctg  3575
   ctttgtcgga gcatcacgca accattttct ggccagataa agtgctactt ccagtaatcg  3635
   gggagcagct caacatcgtg cccaaccatg cctgcaacgt gattaatttg gtggatgagg  3695
   tctacgttcg ggaagccgat ggcactttcc gtacctggaa ggtagttgcc cgcggcagaa  3755
   acaattaggg aaacctcttg acctt                                        3780
<210> 49
   <211> 579
   <212> PRT
   <213> Corynebacterium glutamicum
<400> 49
   Met Ala His Ser Tyr Ala Glu Gln Leu Ile Asp Thr Leu Glu Ala Gln
   1               5                   10                  15
   Gly Val Lys Arg Ile Tyr Gly Leu Val Gly Asp Ser Leu Asn Pro Ile
               20                  25                  30
   Val Asp Ala Val Arg Gln Ser Asp Ile Glu Trp Val His Val Arg Asn
           35                  40                  45
   Glu Glu Ala Ala Ala Phe Ala Ala Gly Ala Glu Ser Leu Ile Thr Gly
       50                  55                  60
   Glu Leu Ala Val Cys Ala Ala Ser Cys Gly Pro Gly Asn Thr His Leu
   65 70 75 80
   Ile Gln Gly Leu Tyr Asp Ser His Arg Asn Gly Ala Lys Val Leu Ala
   85 90 95
   Ile Ala Ser His Ile Pro Ser Ala Gln Ile Gly Ser Thr Phe Phe Gln
   100 105 110
   Glu Thr His Pro Glu Ile Leu Phe Lys Glu Cys Ser Gly Tyr Cys Glu
   115 120 125
   Met Val Asn Gly Gly Glu Gln Gly Glu Arg Ile Leu His His Ala Ile
   130 135 140
   Gln Ser Thr Met Ala Gly Lys Gly Val Ser Val Val Val Ile Pro Gly
   145 150 155 160
   Asp Ile Ala Lys Glu Asp Ala Gly Asp Gly Thr Tyr Ser Asn Ser Thr
   165 170 175
   Ile Ser Ser Gly Thr Pro Val Val Phe Pro Asp Pro Thr Glu Ala Ala
   180 185 190
   Ala Leu Val Glu Ala Ile Asn Asn Ala Lys Ser Val Thr Leu Phe Cys
   195 200 205
   Gly Ala Gly Val Lys Asn Ala Arg Ala Gln Val Leu Glu Leu Ala Glu
   210 215 220
   Lys Ile Lys Ser Pro Ile Gly His Ala Leu Gly Gly Lys Gln Tyr Ile
   225 230 235 240
   Gln His Glu Asn Pro Phe Glu Val Gly Met Ser Gly Leu Leu Gly Tyr
   245 250 255
   Gly Ala Cys Val Asp Ala Ser Asn Glu Ala Asp Leu Leu Ile Leu Leu
   260 265 270
   Gly Thr Asp Phe Pro Tyr Ser Asp Phe Leu Pro Lys Asp Asn Val Ala
   275 280 285
   Gln Val Asp Ile Asn Gly Ala His Ile Gly Arg Arg Thr Thr Val Lys
   290 295 300
   Tyr Pro Val Thr Gly Asp Val Ala Ala Thr Ile Glu Asn Ile Leu Pro
   305 310 315 320
   His Val Lys Glu Lys Thr Asp Arg Ser Phe Leu Asp Arg Met Leu Lys
   325 330 335
   Ala His Glu Arg Lys Leu Ser Ser Val Val Glu Thr Tyr Thr His Asn
   340 345 350
   Val Glu Lys His Val Pro Ile His Pro Glu Tyr Val Ala Ser Ile Leu
   355 360 365
   Asn Glu Leu Ala Asp Lys Asp Ala Val Phe Thr Val Asp Thr Gly Met
   370 375 380
   Cys Asn Val Trp His Ala Arg Tyr Ile Glu Asn Pro Glu Gly Thr Arg
   385 390 395 400
   Asp Phe Val Gly Ser Phe Arg His Gly Thr Met Ala Asn Ala Leu Pro
   405 410 415
   His Ala Ile Gly Ala Gln Ser Val Asp Arg Asn Arg Gln Val Ile Ala
   420 425 430
   Met Cys Gly Asp Gly Gly Leu Gly Met Leu Leu Gly Glu Leu Leu Thr
   435 440 445
   Val Lys Leu His Gln Leu Pro Leu Lys Ala Val Val Phe Asn Asn Ser
   450 455 460
   Ser Leu Gly Met Val Lys Leu Glu Met Leu Val Glu Gly Gln Pro Glu
   465 470 475 480
   Phe Gly Thr Asp His Glu Glu Val Asn Phe Ala Glu Ile Ala Ala Ala
   485 490 495
   Ala Gly Ile Lys Ser Val Arg Ile Thr Asp Pro Lys Lys Val Arg Glu
   500 505 510
   Gln Leu Ala Glu Ala Leu Ala Tyr Pro Gly Pro Val Leu Ile Asp Ile
   515 520 525
   Val Thr Asp Pro Asn Ala Leu Ser Ile Pro Pro Thr Ile Thr Trp Glu
   530 535 540
   Gln Val Met Gly Phe Ser Lys Ala Ala Thr Arg Thr Val Phe Gly Gly
   545 550 555 560
   Gly Val Gly Ala Met Ile Asp Leu Ala Arg Ser Asn Ile Arg Asn Ile
   565 570 575
   Pro Thr Pro
<210> 50
   <211> 3600
   <212> DNA
   <213> Corynebacterium glutamicum
<220>
   <221> CDS
   <222> (1037) .. (2545)
   <223>
<400> 50
   gaagcgctac ggacttcgcg ccggcgtcga cagcaatgcg tccagcatcc aagtgagtat 60
   ggtgctcatc atcaatacca acgcggaact tcaccgtcac cggaatgtcc gtgccttccg 120
   tagccttcac agccgcggaa acgatgtttt caaacaaacg gcgcttgtaa ggaatcgcag 180
   aaccgccacc ccggcgcgtg acctttggaa ccgggcagcc aaagttcata tcaatatgat 240
   ccgccaagtt ttcatcaacg atcatcttcg ccgcttcgta ggtgtacttc gggtcaaccg 300
   tgtacagctg caagcttcgg ggattttcat ccggcgcgaa ggtggtcatg tgcatggttt 360
   tctcattgcg ctcaacaaga gcacgcgcag tcaccatttc acagacgtac agccccgaga 420
   ttgttcccgt gcgttgcatt tcctgttcac ggcacagcgt gcggaaagca acgttggtta 480
   caccagccat gggggctaga accacagggg aggcaaggtc aaaggggccg atttttaaag 540
   tcacctaact attgtccccc gtgaatcagg ttgggcaaaa tatttgaagc aaattgtgag 600
   cagggcgcaa ctaggaaagt ggtgtgcttt cactttttgg gggctggggt tgggttaagc 660
   ttcgcgggct ctagggttgg tctgagcttt attcctgggc tttgggaggc ttgcaaacag 720
   ggggcatgca aatttggggg taatgctggg ccttgaaatc ccactatcac agatagtatt   780
   cgggcatttc ctgtcacgat ggtttatcct tgggacacaa catcaaagtg gggtacatca   840
   tatgcttccg gttgaagtga cctatctgaa aagattggtc gaaccttgaa gcaatggtgt   900
   gaactgcgtt aacgaatttt gtcggacgtt aaaatggtcg cattctgctt gctgaagtgg   960
   cacacctatg tgttctgctt gggtatagca gtgcgggaaa aatttgaaaa agtccgatta  1020
   cctgaggagg tattca atg tct gat cgc att gct tca gaa aag ctg cgc tcc  1072
                     Met Ser Asp Arg Ile Ala Ser Glu Lys Leu Arg Ser
                     1               5                   10
   aag ctc atg tcc gcc gac gag gcg gca cag ttt gtt aac cac ggt gac    1120
   Lys Leu Met Ser Ala Asp Glu Ala Ala Gln Phe Val Asn His Gly Asp
           15                  20                  25
   aag gtt ggt ttc tcc ggc ttc acc ggc gct ggc tac cca aag gca ctg    1168
   Lys Val Gly Phe Ser Gly Phe Thr Gly Ala Gly Tyr Pro Lys Ala Leu
       30                  35                  40
   cct acg gca atc gct aac cgg gct aaa gaa gca cac ggt gca ggc aac    1216
   Pro Thr Ala Ile Ala Asn Arg Ala Lys Glu Ala His Gly Ala Gly Asn
   45                  50                  55                  60
   gac tac gca atc gac ctg ttc act ggc gca tcg acc gcc cct gac tgc    1264
   Asp Tyr Ala Ile Asp Leu Phe Thr Gly Ala Ser Thr Ala Pro Asp Cys
                   65                  70                  75
   gat ggc gta ctt gca gaa gct gac gct atc cgc tgg cgc atg cca tac    1312
   Asp Gly Val Leu Ala Glu Ala Asp Ala Ile Arg Trp Arg Met Pro Tyr
               80                  85                  90
   gca tct gat cca atc atg cgt aac aag atc aac tcc ggc tcc atg gga    1360
   Ala Ser Asp Pro Ile Met Arg Asn Lys Ile Asn Ser Gly Ser Met Gly
           95                  100                 105
   tac tcc gat atc cac ctg tcc cac tcc ggc cag cag gtt gaa gag ggc    1408
   Tyr Ser Asp Ile His Leu Ser His Ser Gly Gln Gln Val Glu Glu Gly
       110                 115                 120
   ttc ttc ggc cag ctc aac gta gct gtc att gaa atc acc cgc atc act    1456
   Phe Phe Gly Gln Leu Asn Val Ala Val Ile Glu Ile Thr Arg Ile Thr
   125                 130                 135                 140
   gaa gag ggc tac atc atc cct tct tcc tcc gtg ggt aac aac gtt gag    1504
   Glu Glu Gly Tyr Ile Ile Pro Ser Ser Ser Val Gly Asn Asn Val Glu
                   145                 150                 155
   tgg ctc aac gct gca gag aag gtc atc ctc gag gtt aac tct tgg cag    1552
   Trp Leu Asn Ala Ala Glu Lys Val Ile Leu Glu Val Asn Ser Trp Gln
               160                 165                 170
   tct gaa gac ctc gaa ggt atg cac gac atc tgg tct gtt cct gcc ctg    1600
   Ser Glu Asp Leu Glu Gly Met His Asp Ile Trp Ser Val Pro Ala Leu
           175                 180                 185
   cca aac cgc att gcc gtg cca atc aac aag cca ggc gac cgc atc ggt    1648
   Pro Asn Arg Ile Ala Val Pro Ile Asn Lys Pro Gly Asp Arg Ile Gly
       190                 195                 200
   aag acc tac atc gag ttc gac acc gac aag gtt gtt gct gtt gtt gag    1696
   Lys Thr Tyr Ile Glu Phe Asp Thr Asp Lys Val Val Ala Val Val Glu
   205 210 215 220
   acc aac acc gca gac cgc aac gca cca ttc aag cct gtc gac gac atc 1744
   Thr Asn Thr Ala Asp Arg Asn Ala Pro Phe Lys Pro Val Asp Asp Ile
   225 230 235
   tct aag aag atc gct ggc aac ttc ctc gac ttc ctg gaa agc gaa gtt 1792
   Ser Lys Lys Ile Ala Gly Asn Phe Leu Asp Phe Leu Glu Ser Glu Val
   240 245 250
   gct gca ggt cgc ctg tcc tac gac ggc tac atc atg cag tcc ggc gtg 1840
   Ala Ala Gly Arg Leu Ser Tyr Asp Gly Tyr Ile Met Gln Ser Gly Val
   255 260 265
   ggc aac gtg cca aac gcg gtg atg gca ggc ctg ctg gaa tcc aag ttt 1888
   Gly Asn Val Pro Asn Ala Val Met Ala Gly Leu Leu Glu Ser Lys Phe
   270 275 280
   gag aac atc cag gcc tac acc gaa gtt atc cag gac ggc atg gtg gac 1936
   Glu Asn Ile Gln Ala Tyr Thr Glu Val Ile Gln Asp Gly Met Val Asp
   285 290 295 300
   ctc atc gac gcc ggc aag atg acc gtt gca tcc gca act tcc ttc tcc 1984
   Leu Ile Asp Ala Gly Lys Met Thr Val Ala Ser Ala Thr Ser Phe Ser
   305 310 315
   ctg tct cct gag tac gca gag aag atg aac aac gag gct aag cgt tac 2032
   Leu Ser Pro Glu Tyr Ala Glu Lys Met Asn Asn Glu Ala Lys Arg Tyr
   320 325 330
   cgc gag tcc att atc ctg cgc cca cag cag atc tct aac cac cca gag 2080
   Arg Glu Ser Ile Ile Leu Arg Pro Gln Gln Ile Ser Asn His Pro Glu
   335 340 345
   gtc atc cgc cgc gtt ggc ctg atc gcc acc aac ggt ctc atc gag gct 2128
   Val Ile Arg Arg Val Gly Leu Ile Ala Thr Asn Gly Leu Ile Glu Ala
   350 355 360
   gac att tac ggc aac gtc aac tcc acc aac gtt tct ggc tcc cgc gtc 2176
   Asp Ile Tyr Gly Asn Val Asn Ser Thr Asn Val Ser Gly Ser Arg Val
   365 370 375 380
   atg aac ggc atc ggc ggc tcc ggc gac ttc acc cgt aac ggc tac atc 2224
   Met Asn Gly Ile Gly Gly Ser Gly Asp Phe Thr Arg Asn Gly Tyr Ile
   385 390 395
   tcc agc ttc atc acc cct tca gag gca aag ggc ggc gca atc tct gcg 2272
   Ser Ser Phe Ile Thr Pro Ser Glu Ala Lys Gly Gly Ala Ile Ser Ala
   400 405 410
   atc gtt cct ttc gca tcc cac atc gac cac acc gag cac gat gtc atg 2320
   Ile Val Pro Phe Ala Ser His Ile Asp His Thr Glu His Asp Val Met
   415 420 425
   gtt gtt atc tct gag tac ggt tac gca gac ctt cgt ggt ctg gct cca 2368
   Val Val Ile Ser Glu Tyr Gly Tyr Ala Asp Leu Arg Gly Leu Ala Pro
   430 435 440
   cgt gag cgc gtt gcc aag atg atc ggc ctg gct cac cct gat tac cgc 2416
   Arg Glu Arg Val Ala Lys Met Ile Gly Leu Ala His Pro Asp Tyr Arg
   445 450 455 460
   cca ctg ctc gag gag tac tac gct cgc gca acc tcc ggt gac aac aag 2464
   Pro Leu Leu Glu Glu Tyr Tyr Ala Arg Ala Thr Ser Gly Asp Asn Lys
   465 470 475
   tac atg cag acc cct cat gat ctt gca acc gcg ttt gat ttc cac atc 2512
   Tyr Met Gln Thr Pro His Asp Leu Ala Thr Ala Phe Asp Phe His Ile
   480 485 490
   aac ctg gct aag aac ggc tcc atg aag gca taa gttttttctt ggtttagaaa    2565
   Asn Leu Ala Lys Asn Gly Ser Met Lys Ala
           495                 500
   ccgccgcctc gacaacattt cgaggcggcg gtttctttta ttacctgggt tttgagcgtt 2625
   aaattagacc aggtcaggct agtgtttggt agctaattga gggcgatttt aataaggccg 2685
   gtgccatgta ctaatatggt ctgagttggg cctatagctc agttggtaga gctacggact 2745
   tttaatccgc aggtcttggg ttcgagtccc aatgggccca catcttaagt acccctgttt 2805
   tggagaatgc tccgagccag gggtactttt cttttcctca cacacagtag ctgctgagaa 2865
   aaatgaagac cttttgttag gttgggagta tgaccaaccc atacgaggcc ttcataccgc 2925
   tcaagcatcg tacggggatt gaacccgagc acaccttttg ggaatgggaa aacaaaaggg 2985
   ttcacattgc aaggagacgt cgagaagcgc ccgtccgcgt tatcgtggtg catgggctag 3045
   gcacccatag tggcgccctc tggcccctcg tcgcggccat tgagggcgcg gacctcgccg 3105
   cgatcgacct gcctaaaact ccgctttacg acgattggct gcgcctttta gaatctttca 3165
   tctcttccga agacgacggt cggccactca tcctgatcgg tgcaggcacc ggaggcttgc 3225
   tttgcgcaga agctgcacac cgcacaggac tggtcgcaca cgtcattgcc acctgcctgc 3285
   tcaacccctc cgaccagccg acgcgccggg cactgttcag gttttcaccg ctgactcggt 3345
   tgatccaagg ccgcttgcgc aaccgcgaaa ttcccgtgac cagagtgttg aacttcagca 3405
   aaatcagccg cagcccagcc ctgagcaaat tgtgcgcggc cgatgaattt agcggagcat 3465
   ccaaaataac ctggggtttc ctcgcgtcat atgtgcaaca caaggccaaa ctgggtgcag 3525
   ttcccgtcac tctgatgcac cctgaccacg accttctgac tcccgttgag ctcagtctgc 3585
   gtacgctttc gcgcc 3600
<210> 51
   <211> 502
   <212> PRT
   <213> Corynebacterium glutamicum
<400> 51
   Met Ser Asp Arg Ile Ala Ser Glu Lys Leu Arg Ser Lys Leu Met Ser
   1 5 10 15
   Ala Asp Glu Ala Ala Gln Phe Val Asn His Gly Asp Lys Val Gly Phe
   20 25 30
   Ser Gly Phe Thr Gly Ala Gly Tyr Pro Lys Ala Leu Pro Thr Ala Ile
   35 40 45
   Ala Asn Arg Ala Lys Glu Ala His Gly Ala Gly Asn Asp Tyr Ala Ile
   50 55 60
   Asp Leu Phe Thr Gly Ala Ser Thr Ala Pro Asp Cys Asp Gly Val Leu
   65 70 75 80
   Ala Glu Ala Asp Ala Ile Arg Trp Arg Met Pro Tyr Ala Ser Asp Pro
   85 90 95
   Ile Met Arg Asn Lys Ile Asn Ser Gly Ser Met Gly Tyr Ser Asp Ile
   100 105 110
   His Leu Ser His Ser Gly Gln Gln Val Glu Glu Gly Phe Phe Gly Gln
   115 120 125
   Leu Asn Val Ala Val Ile Glu Ile Thr Arg Ile Thr Glu Glu Gly Tyr
   130 135 140
   Ile Ile Pro Ser Ser Ser Val Gly Asn Asn Val Glu Trp Leu Asn Ala
   145 150 155 160
   Ala Glu Lys Val Ile Leu Glu Val Asn Ser Trp Gln Ser Glu Asp Leu
   165 170 175
   Glu Gly Met His Asp Ile Trp Ser Val Pro Ala Leu Pro Asn Arg Ile
   180 185 190
   Ala Val Pro Ile Asn Lys Pro Gly Asp Arg Ile Gly Lys Thr Tyr Ile
   195 200 205
   Glu Phe Asp Thr Asp Lys Val Val Ala Val Val Glu Thr Asn Thr Ala
   210 215 220
   Asp Arg Asn Ala Pro Phe Lys Pro Val Asp Asp Ile Ser Lys Lys Ile
   225 230 235 240
   Ala Gly Asn Phe Leu Asp Phe Leu Glu Ser Glu Val Ala Ala Gly Arg
   245 250 255
   Leu Ser Tyr Asp Gly Tyr Ile Met Gln Ser Gly Val Gly Asn Val Pro
   260 265 270
   Asn Ala Val Met Ala Gly Leu Leu Glu Ser Lys Phe Glu Asn Ile Gln
   275 280 285
   Ala Tyr Thr Glu Val Ile Gln Asp Gly Met Val Asp Leu Ile Asp Ala
   290 295 300
   Gly Lys Met Thr Val Ala Ser Ala Thr Ser Phe Ser Leu Ser Pro Glu
   305 310 315 320
   Tyr Ala Glu Lys Met Asn Asn Glu Ala Lys Arg Tyr Arg Glu Ser Ile
   325 330 335
   Ile Leu Arg Pro Gln Gln Ile Ser Asn His Pro Glu Val Ile Arg Arg
   340 345 350
   Val Gly Leu Ile Ala Thr Asn Gly Leu Ile Glu Ala Asp Ile Tyr Gly
   355 360 365
   Asn Val Asn Ser Thr Asn Val Ser Gly Ser Arg Val Met Asn Gly Ile
   370 375 380
   Gly Gly Ser Gly Asp Phe Thr Arg Asn Gly Tyr Ile Ser Ser Phe Ile
   385 390 395 400
   Thr Pro Ser Glu Ala Lys Gly Gly Ala Ile Ser Ala Ile Val Pro Phe
   405 410 415
   Ala Ser His Ile Asp His Thr Glu His Asp Val Met Val Val Ile Ser
               420                 425                 430
   Glu Tyr Gly Tyr Ala Asp Leu Arg Gly Leu Ala Pro Arg Glu Arg Val
           435                 440                 445
   Ala Lys Met Ile Gly Leu Ala His Pro Asp Tyr Arg Pro Leu Leu Glu
       450                 455                 460
   Glu Tyr Tyr Ala Arg Ala Thr Ser Gly Asp Asn Lys Tyr Met Gln Thr
   465                 470                 475                 480
   Pro His Asp Leu Ala Thr Ala Phe Asp Phe His Ile Asn Leu Ala Lys
                   485                 490                 495
   Asn Gly Ser Met Lys Ala
               500
<210> 52
   <211> 2100
   <212> DNA
   <213> Corynebacterium glutamicum
<220>
   <221> CDS
   <222> (850).. (1134)
   <223>
<400> 52
   ggccgattcc gaaggaaatc gcacgcctgg attttgataa cggtgatcca aacgcagcag     60
   ctcctgttcc aggtttgagc ccatttatgc ccaaagagca ggcaattgag cggcttttag    120
   agatcattgg acagaacctg ctgctgcctt ttgagatcga ggtgccggag aaaattcagc    180
   gggaagcact gagggatttc acggcggaaa ctcaattggg ttccaccgtg ggcgctgata    240
   tttttgatgc attgcaaatg gctgttgggg tggtgtcggg aagcgcgaag agcaattggc    300
   gcaaatgggg agcgtttggt gtgggggctg cagctttgac cgctgcaacg ggtggtttgg    360
   ctttggcggc tgtgccgact gttgctggag tagccactgt tgcctcgaca ctcgcagcat    420
   ttggtccagg tgggatgatg ggcggtttgg tcactgcagg aacactgctc acagttggtg    480
   gcggcagttt aaccgctggg gtgttgagct cggtgaacac cacggaagag atcgaagcgc    540
   tcgttgtaca gaagctaagt ttggctattt tgtggcagcg ccatgagata gatagaactc    600
   atgaggtgtg ggaagaattc gcggaggcag aacgtctgat tgtgcgggag cacacgcgtg    660
   tgaaaaacgt gtcggatagt tcttcgccca ttttgaaagc tttcgagcag cagcgttcga    720
   ctattgagcg ggcgttgaag tatttgagcg atcatgggat ggaacctggc tggtttgaag    780
   aactcgaacc accagcccca acaccgtttc taaaactgcg ggctaagaaa actgattagg    840
   agaaacaca atg gag aaa gtt cgt ctg act gct ttt gtt cat ggt cat gtc    891
             Met Glu Lys Val Arg Leu Thr Ala Phe Val His Gly His Val
             1               5                   10
   cag ggc gtg ggt ttt cga tgg tgg act acc tcg cag gca cga gaa tta      939
   Gln Gly Val Gly Phe Arg Trp Trp Thr Thr Ser Gln Ala Arg Glu Leu
   15                  20                  25                  30
   aaa ctt gca ggt tct gcc agt aat tta agt gac ggc cgg gtg tgc gtg      987
   Lys Leu Ala Gly Ser Ala Ser Asn Leu Ser Asp Gly Arg Val Cys Val
   35 40 45
   gtt gct gaa ggg cca caa aca cag tgc gaa gaa ctg ctg aga agg ttg 1035
   Val Ala Glu Gly Pro Gln Thr Gln Cys Glu Glu Leu Leu Arg Arg Leu
   50 55 60
   aag gaa aac ccc agc tcg tat cgc aga cca ggt cat gtg gac aca gtt 1083
   Lys Glu Asn Pro Ser Ser Tyr Arg Arg Pro Gly His Val Asp Thr Val
   65 70 75
   att gag caa tgg ggc gag ccg cgt gac gtt gaa ggc ttt gtg gag cgc 1131
   Ile Glu Gln Trp Gly Glu Pro Arg Asp Val Glu Gly Phe Val Glu Arg
   80 85 90
   tag actttaaccc cgttatgtat ttgaaatcgt tgacgctcaa ggggtttaag 1184
   tctttcgcgt ctgcgacgac cctgaaattt gagccaggca tttgtgccgt ggtgggtccg 1244
   aatggttcag gcaaatccaa tgtggttgat gcgctggcct gggtgatggg tgaaggttct 1304
   gcgaagacct tgcgtggcgg caaaatggaa gatgtcattt ttgctggcgc gggcgatcgt 1364
   aaaccgttgg gtcgcgcaga agtcacgctg accattgata actctgatgg cgcactgccc 1424
   attgagtaca ccgaagtgtc ggtgaccaga cggatgttcc gtgatggtgc aagtgaatat 1484
   gagatcaatg gggcgaaagc tcgattgatg gatatccagg agctgttgtc ggataccggt 1544
   attggccgtg aaatgcacat catggtgggg cagggaaagc tcgcagagat tttggagtcc 1604
   cgccccgaag agcgccgagc gtatatcgaa gaagctgcgg gtgtgctcaa gcaccggcgc 1664
   aggaaagaaa aggcgcagcg caaacttcag ggcatgcagg tcaatcttga tcgtttgcag 1724
   gatctgaccc atgagttggc caagcagctc aagccgttgg ctaggcaggc ggaagcagcg 1784
   cagcgtgcgg cgacggtgca ggctgatttg cgtgatgcgc gtttccagat tgctggcttt 1844
   gagatcgtga agctctcgga aaagctggaa acctctactg agcgcgagaa aatgattcgt 1904
   gagcaggcgg aagcagcaca agagcagctg gaagaagcca ccacaactca gatggaagtg 1964
   gagatggagt tggcggagat cactccgcag gctgaagctg cgcaacagtt gtggtttgat 2024
   ttgtcttcgc tggctgagcg ggtgtcggca acgatgcgta ttgctgcaga ccgtgcgagt 2084
   tcaggtgccg cggatg 2100
<210> 53
   <211> 94
   <212> PRT
   <213> Corynebacterium glutamicum
<400> 53
   Met Glu Lys Val Arg Leu Thr Ala Phe Val His Gly His Val Gln Gly
   1 5 10 15
   Val Gly Phe Arg Trp Trp Thr Thr Ser Gln Ala Arg Glu Leu Lys Leu
   20 25 30
   Ala Gly Ser Ala Ser Asn Leu Ser Asp Gly Arg Val Cys Val Val Ala
   35 40 45
   Glu Gly Pro Gln Thr Gln Cys Glu Glu Leu Leu Arg Arg Leu Lys Glu
   50 55 60
   Asn Pro Ser Ser Tyr Arg Arg Pro Gly His Val Asp Thr Val Ile Glu
   65 70 75 80
   Gln Trp Gly Glu Pro Arg Asp Val Glu Gly Phe Val Glu Arg
   85 90
<210> 54
   <211> 24
   <212> DNA
   <213> Artificial
<220>
   <223> primer
<400> 54 24
   cagcttcttc gatatacgct cgcc 24
<210> 55
   <211> 46
   <212> DNA
   <213> Artificial
<220>
   <223> primer
<400> 55 46
   ctgattagga gaaacacaat ggaggtggag cgctagactt taaccc 46
<210> 56
   <211> 46
   <212> DNA
   <213> Artificial
<220>
   <223> primer
<400> 56 46
   gggttaaagt ctagcgctcc acctccattg tgtttctcct aatcag 46
<210> 57
   <211> 25
   <212> DNA
   <213> Artificial
<220>
   <223> primer
<400> 57 25
   gctgcagctt tgaccgctgc aacgg 25
<210> 58
   <211> 33
   <212> DNA
   <213> Artificial
<220>
   <223> primer
<400> 58 33
   gggaatctag acccaccatg atgtgcattt cac 33
<210> 59
   <211> 34
   <212> DNA
   <213> Artificial
<220>
   <223> primer
<400> 59 34
   gggaatctag agttgctgga gtagccactg ttgc 34
<210> 60
   <211> 3423
   <212> DNA
   <213> Corynebacterium glutamicum
<220>
   <221> CDS
   <222> (1)..(3423)
   <223>
<400> 60
   gtg tcg act cac aca tct tca acg ctt cca gca ttc aaa aag atc ttg      48
   Val Ser Thr His Thr Ser Ser Thr Leu Pro Ala Phe Lys Lys Ile Leu
   1               5                   10                  15
   gta gca aac cgc ggc gaa atc gcg gtc cgt gct ttc cgt gca gca ctc      96
   Val Ala Asn Arg Gly Glu Ile Ala Val Arg Ala Phe Arg Ala Ala Leu
               20                  25                  30
   gaa acc ggt gca gcc acg gta gct att tac ccc cgt gaa gat cgg gga     144
   Glu Thr Gly Ala Ala Thr Val Ala Ile Tyr Pro Arg Glu Asp Arg Gly
               35                  40                  45
   tca ttc cac cgc tct ttt gct tct gaa gct gtc cgc att ggt acc gaa      192
   Ser Phe His Arg Ser Phe Ala Ser Glu Ala Val Arg Ile Gly Thr Glu
           50                  55                  60
   ggc tca cca gtc aag gcg tac ctg gac atc gat gaa att atc ggt gca      240
   Gly Ser Pro Val Lys Ala Tyr Leu Asp Ile Asp Glu Ile Ile Gly Ala
   65                  70                  75                  80
   gct aaa aaa gtt aaa gca gat gcc att tac ccg gga tac ggc ttc ctg      288
   Ala Lys Lys Val Lys Ala Asp Ala Ile Tyr Pro Gly Tyr Gly Phe Leu
                   85                  90                  95
   tct gaa aat gcc cag ctt gcc cgc gag tgt gcg gaa aac ggc att act      336
   Ser Glu Asn Ala Gln Leu Ala Arg Glu Cys Ala Glu Asn Gly Ile Thr
               100                 105                 110
   ttt att ggc cca acc cca gag gtt ctt gat ctc acc ggt gat aag tct      384
   Phe Ile Gly Pro Thr Pro Glu Val Leu Asp Leu Thr Gly Asp Lys Ser
           115                 120                 125
   cgc gcg gta acc gcc gcg aag aag gct ggt ctg cca gtt ttg gcg gaa      432
   Arg Ala Val Thr Ala Ala Lys Lys Ala Gly Leu Pro Val Leu Ala Glu
       130                 135                 140
   tcc acc ccg agc aaa aac atc gat gag atc gtt aaa agc gct gaa ggc      480
   Ser Thr Pro Ser Lys Asn Ile Asp Glu Ile Val Lys Ser Ala Glu Gly
   145                 150                 155                 160
   cag act tac ccc atc ttt gtg aag gca gtt gcc ggt ggt ggc gga cgc      528
   Gln Thr Tyr Pro Ile Phe Val Lys Ala Val Ala Gly Gly Gly Gly Arg
                   165                 170                 175
   ggt atg cgt ttt gtt gct tca cct gat gag ctt cgc aaa tta gca aca      576
   Gly Met Arg Phe Val Ala Ser Pro Asp Glu Leu Arg Lys Leu Ala Thr
               180                 185                 190
   gaa gca tct cgt gaa gct gaa gcg gct ttc ggc gat ggc gcg gta tat      624
   Glu Ala Ser Arg Glu Ala Glu Ala Ala Phe Gly Asp Gly Ala Val Tyr
           195                 200                 205
   gtc gaa cgt gct gtg att aac cct cag cat att gaa gtg cag atc ctt      672
   Val Glu Arg Ala Val Ile Asn Pro Gln His Ile Glu Val Gln Ile Leu
       210                 215                 220
   ggc gat cac act gga gaa gtt gta cac ctt tat gaa cgt gac tgc tca      720
   Gly Asp His Thr Gly Glu Val Val His Leu Tyr Glu Arg Asp Cys Ser
   225                 230                 235                 240
   ctg cag cgt cgt cac caa aaa gtt gtc gaa att gcg cca gca cag cat      768
   Leu Gln Arg Arg His Gln Lys Val Val Glu Ile Ala Pro Ala Gln His
                   245                 250                 255
   ttg gat cca gaa ctg cgt gat cgc att tgt gcg gat gca gta aag ttc      816
   Leu Asp Pro Glu Leu Arg Asp Arg Ile Cys Ala Asp Ala Val Lys Phe
               260                 265                 270
   tgc cgc tcc att ggt tac cag ggc gcg gga acc gtg gaa ttc ttg gtc      864
   Cys Arg Ser Ile Gly Tyr Gln Gly Ala Gly Thr Val Glu Phe Leu Val
               275                 280                 285
   gat gaa aag ggc aac cac gtc ttc atc gaa atg aac cca cgt atc cag      912
   Asp Glu Lys Gly Asn His Val Phe Ile Glu Met Asn Pro Arg Ile Gln
       290                 295                 300
   gtt gag cac acc gtg act gaa gaa gtc acc gag gtg gac ctg gtg aag      960
   Val Glu His Thr Val Thr Glu Glu Val Thr Glu Val Asp Leu Val Lys
   305                 310                 315                 320
   gcg cag atg cgc ttg gct gct ggt gca acc ttg aag gaa ttg ggt ctg     1008
   Ala Gln Met Arg Leu Ala Ala Gly Ala Thr Leu Lys Glu Leu Gly Leu
                   325                 330                 335
   acc caa gat aag atc aag acc cac ggt gca gca ctg cag tgc cgc atc     1056
   Thr Gln Asp Lys Ile Lys Thr His Gly Ala Ala Leu Gln Cys Arg Ile
               340                 345                 350
   acc acg gaa gat cca aac aac ggc ttc cgc cca gat acc gga act atc     1104
   Thr Thr Glu Asp Pro Asn Asn Gly Phe Arg Pro Asp Thr Gly Thr Ile
           355                 360                 365
   acc gcg tac cgc tca cca ggc gga gct ggc gtt cgt ctt gac ggt gca     1152
   Thr Ala Tyr Arg Ser Pro Gly Gly Ala Gly Val Arg Leu Asp Gly Ala
       370                 375                 380
   gct cag ctc ggt ggc gaa atc acc gca cac ttt gac tcc atg ctg gtg     1200
   Ala Gln Leu Gly Gly Glu Ile Thr Ala His Phe Asp Ser Met Leu Val
   385                 390                 395                 400
   aaa atg acc tgc cgt ggt tcc gac ttt gaa act gct gtt gct cgt gca     1248
   Lys Met Thr Cys Arg Gly Ser Asp Phe Glu Thr Ala Val Ala Arg Ala
                   405                 410                 415
   cag cgc gcg ttg gct gag ttc acc gtg tct ggt gtt gca acc aac att     1296
   Gln Arg Ala Leu Ala Glu Phe Thr Val Ser Gly Val Ala Thr Asn Ile
               420                 425                 430
   ggt ttc ttg cgt gcg ttg ctg cgg gaa gag gac ttc act tcc aag cgc     1344
   Gly Phe Leu Arg Ala Leu Leu Arg Glu Glu Asp Phe Thr Ser Lys Arg
           435                 440                 445
   atc gcc acc gga ttc att gcc gat cac ccg cac ctc ctt cag gct cca     1392
   Ile Ala Thr Gly Phe Ile Ala Asp His Pro His Leu Leu Gln Ala Pro
       450                 455                 460
   cct gct gat gat gag cag gga cgc atc ctg gat tac ttg gca gat gtc     1440
   Pro Ala Asp Asp Glu Gln Gly Arg Ile Leu Asp Tyr Leu Ala Asp Val
   465                 470                 475                 480
   acc gtg aac aag cct cat ggt gtg cgt cca aag gat gtt gca gct cct     1488
   Thr Val Asn Lys Pro His Gly Val Arg Pro Lys Asp Val Ala Ala Pro
                   485                 490                 495
   atc gat aag ctg cct aac atc aag gat ctg cca ctg cca cgc ggt tcc     1536
   Ile Asp Lys Leu Pro Asn Ile Lys Asp Leu Pro Leu Pro Arg Gly Ser
               500                 505                 510
   cgt gac cgc ctg aag cag ctt ggc cca gcc gcg ttt gct cgt gat ctc     1584
   Arg Asp Arg Leu Lys Gln Leu Gly Pro Ala Ala Phe Ala Arg Asp Leu
   515 520 525
   cgt gag cag gac gca ctg gca gtt act gat acc acc ttc cgc gat gca 1632
   Arg Glu Gln Asp Ala Leu Ala Val Thr Asp Thr Thr Phe Arg Asp Ala
   530 535 540
   cac cag tct ttg ctt gcg acc cga gtc cgc tca ttc gca ctg aag cct 1680
   His Gln Ser Leu Leu Ala Thr Arg Val Arg Ser Phe Ala Leu Lys Pro
   545 550 555 560
   gcg gca gag gcc gtc gca aag ctg act cct gag ctt ttg tcc gtg gag 1728
   Ala Ala Glu Ala Val Ala Lys Leu Thr Pro Glu Leu Leu Ser Val Glu
   565 570 575
   gcc tgg ggc ggc gcg acc tac gat gtg gcg atg cgt ttc ctc ttt gag 1776
   Ala Trp Gly Gly Ala Thr Tyr Asp Val Ala Met Arg Phe Leu Phe Glu
   580 585 590
   gat ccg tgg gac agg ctc gac gag ctg cgc gag gcg atg ccg aat gta 1824
   Asp Pro Trp Asp Arg Leu Asp Glu Leu Arg Glu Ala Met Pro Asn Val
   595 600 605
   aac att cag atg ctg ctt cgc ggc cgc aac acc gtg gga tac acc ccg 1872
   Asn Ile Gln Met Leu Leu Arg Gly Arg Asn Thr Val Gly Tyr Thr Pro
   610 615 620
   tac cca gac tcc gtc tgc cgc gcg ttt gtt aag gaa gct gcc agc tcc 1920
   Tyr Pro Asp Ser Val Cys Arg Ala Phe Val Lys Glu Ala Ala Ser Ser
   625 630 635 640
   ggc gtg gac atc ttc cgc atc ttc gac gcg ctt aac gac gtc tcc cag 1968
   Gly Val Asp Ile Phe Arg Ile Phe Asp Ala Leu Asn Asp Val Ser Gln
   645 650 655
   atg cgt cca gca atc gac gca gtc ctg gag acc aac acc gcg gta gcc 2016
   Met Arg Pro Ala Ile Asp Ala Val Leu Glu Thr Asn Thr Ala Val Ala
   660 665 670
   gag gtg gct atg gct tat tct ggt gat ctc tct gat cca aat gaa aag 2064
   Glu Val Ala Met Ala Tyr Ser Gly Asp Leu Ser Asp Pro Asn Glu Lys
   675 680 685
   ctc tac acc ctg gat tac tac cta aag atg gca gag gag atc gtc aag 2112
   Leu Tyr Thr Leu Asp Tyr Tyr Leu Lys Met Ala Glu Glu Ile Val Lys
   690 695 700
   tct ggc gct cac atc ttg gcc att aag gat atg gct ggt ctg ctt cgc 2160
   Ser Gly Ala His Ile Leu Ala Ile Lys Asp Met Ala Gly Leu Leu Arg
   705 710 715 720
   cca gct gcg gta acc aag ctg gtc acc gca ctg cgc cgt gaa ttc gat 2208
   Pro Ala Ala Val Thr Lys Leu Val Thr Ala Leu Arg Arg Glu Phe Asp
   725 730 735
   ctg cca gtg cac gtg cac acc cac gac act gcg ggt ggc cag ctg gca 2256
   Leu Pro Val His Val His Thr His Asp Thr Ala Gly Gly Gln Leu Ala
   740 745 750
   acc tac ttt gct gca gct caa gct ggt gca gat gct gtt gac ggt gct 2304
   Thr Tyr Phe Ala Ala Ala Gln Ala Gly Ala Asp Ala Val Asp Gly Ala
               755                 760                 765
   tcc gca cca ctg tct ggc acc acc tcc cag cca tcc ctg tct gcc att     2352
   Ser Ala Pro Leu Ser Gly Thr Thr Ser Gln Pro Ser Leu Ser Ala Ile
           770                 775                 780
   gtt gct gca ttc gcg cac acc cgt cgc gat acc ggt ttg agc ctc gag     2400
   Val Ala Ala Phe Ala His Thr Arg Arg Asp Thr Gly Leu Ser Leu Glu
   785                 790                 795                 800
   gct gtt tct gac ctc gag ccg tac tgg gaa gca gtg cgc gga ctg tac     2448
   Ala Val Ser Asp Leu Glu Pro Tyr Trp Glu Ala Val Arg Gly Leu Tyr
                   805                 810                 815
   ctg cca ttt gag tct gga acc cca ggc cca acc ggt cgc gtc tac cgc     2496
   Leu Pro Phe Glu Ser Gly Thr Pro Gly Pro Thr Gly Arg Val Tyr Arg
               820                 825                 830
   cac gaa atc cca ggc gga cag ttg tcc aac ctg cgt gca cag gcc acc     2544
   His Glu Ile Pro Gly Gly Gln Leu Ser Asn Leu Arg Ala Gln Ala Thr
           835                 840                 845
   gca ctg ggc ctt gcg gat cgt ttc gaa ctc atc gaa gac aac tac gca     2592
   Ala Leu Gly Leu Ala Asp Arg Phe Glu Leu Ile Glu Asp Asn Tyr Ala
       850                 855                 860
   gcc gtt aat gag atg ctg gga cgc cca acc aag gtc acc cca tcc tcc     2640
   Ala Val Asn Glu Met Leu Gly Arg Pro Thr Lys Val Thr Pro Ser Ser
   865                 870                 875                 880
   aag gtt gtt ggc gac ctc gca ctc cac ctc gtt ggt gcg ggt gtg gat     2688
   Lys Val Val Gly Asp Leu Ala Leu His Leu Val Gly Ala Gly Val Asp
                   885                 890                 895
   cca gca gac ttt gct gcc gat cca caa aag tac gac atc cca gac tct     2736
   Pro Ala Asp Phe Ala Ala Asp Pro Gln Lys Tyr Asp Ile Pro Asp Ser
               900                 905                 910
   gtc atc gcg ttc ctg cgc ggc gag ctt ggt aac cct cca ggt ggc tgg     2784
   Val Ile Ala Phe Leu Arg Gly Glu Leu Gly Asn Pro Pro Gly Gly Trp
           915                 920                 925
   cca gag cca ctg cgc acc cgc gca ctg gaa ggc cgc tcc gaa ggc aag     2832
   Pro Glu Pro Leu Arg Thr Arg Ala Leu Glu Gly Arg Ser Glu Gly Lys
       930                 935                 940
   gca cct ctg acg gaa gtt cct gag gaa gag cag gcg cac ctc gac gct     2880
   Ala Pro Leu Thr Glu Val Pro Glu Glu Glu Gln Ala His Leu Asp Ala
   945                 950                 955                 960
   gat gat tcc aag gaa cgt cgc aat agc ctc aac cgc ctg ctg ttc ccg     2928
   Asp Asp Ser Lys Glu Arg Arg Asn Ser Leu Asn Arg Leu Leu Phe Pro
                   965                 970                 975
   aag cca acc gaa gag ttc ctc gag cac cgt cgc cgc ttc ggc aac acc     2976
   Lys Pro Thr Glu Glu Phe Leu Glu His Arg Arg Arg Phe Gly Asn Thr
               980                 985                 990
   tct gcg ctg gat gat cgt gaa ttc ttc tac ggc ctg gtc gaa ggc cgc     3024
   Ser Ala Leu Asp Asp Arg Glu Phe Phe Tyr Gly Leu Val Glu Gly Arg
               995                     1000                    1005
   gag act  ttg atc cgc ctg cca  gat gtg cgc acc cca  ctg ctt gtt     3069
   Glu Thr  Leu Ile Arg Leu Pro  Asp Val Arg Thr Pro  Leu Leu Val
       1010                 1015                 1020
   cgc ctg  gat gcg atc tct gag  cca gac gat aag ggt  atg cgc aat     3114
   Arg Leu  Asp Ala Ile Ser Glu  Pro Asp Asp Lys Gly  Met Arg Asn
       1025                 1030                 1035
   gtt gtg  gcc aac gtc aac ggc  cag atc cgc cca atg  cgt gtg cgt     3159
   Val Val  Ala Asn Val Asn Gly  Gln Ile Arg Pro Met  Arg Val Arg
       1040                 1045                 1050
   gac cgc  tcc gtt gag tct gtc  acc gca acc gca gaa  aag gca gat     3204
   Asp Arg  Ser Val Glu Ser Val  Thr Ala Thr Ala Glu  Lys Ala Asp
       1055                 1060                 1065
   tcc tcc  aac aag ggc cat gtt  gct gca cca ttc gct  ggt gtt gtc     3249
   Ser Ser  Asn Lys Gly His Val  Ala Ala Pro Phe Ala  Gly Val Val
       1070                 1075                 1080
   acc gtg  act gtt gct gaa ggt  gat gag gtc aag gct  gga gat gca     3294
   Thr Val  Thr Val Ala Glu Gly  Asp Glu Val Lys Ala  Gly Asp Ala
       1085                 1090                 1095
   gtc gca  atc atc gag gct atg  aag atg gaa gca aca  atc act gct     3339
   Val Ala  Ile Ile Glu Ala Met  Lys Met Glu Ala Thr  Ile Thr Ala
       1100                 1105                 1110
   tct gtt  gac ggc aaa atc gat  cgc gtt gtg gtt cct  gct gca acg     3384
   Ser Val  Asp Gly Lys Ile Asp  Arg Val Val Val Pro  Ala Ala Thr
       1115                 1120                 1125
   aag gtg  gaa ggt ggc gac ttg  atc gtc gtc gtt tcc  taa             3423
   Lys Val  Glu Gly Gly Asp Leu  Ile Val Val Val Ser
       1130                 1135                 1140
<210> 61
   <211> 1140
   <212> PRT
   <213> Corynebacterium glutamicum
<400> 61
   Val Ser Thr His Thr Ser Ser Thr Leu Pro Ala Phe Lys Lys Ile Leu
   1               5                   10                  15
   Val Ala Asn Arg Gly Glu Ile Ala Val Arg Ala Phe Arg Ala Ala Leu
               20                  25                  30
   Glu Thr Gly Ala Ala Thr Val Ala Ile Tyr Pro Arg Glu Asp Arg Gly
           35                  40                  45
   Ser Phe His Arg Ser Phe Ala Ser Glu Ala Val Arg Ile Gly Thr Glu
       50                  55                  60
   Gly Ser Pro Val Lys Ala Tyr Leu Asp Ile Asp Glu Ile Ile Gly Ala
   65                  70                  75                  80
   Ala Lys Lys Val Lys Ala Asp Ala Ile Tyr Pro Gly Tyr Gly Phe Leu
                   85                  90                  95
   Ser Glu Asn Ala Gln Leu Ala Arg Glu Cys Ala Glu Asn Gly Ile Thr
               100                 105                 110
   Phe Ile Gly Pro Thr Pro Glu Val Leu Asp Leu Thr Gly Asp Lys Ser
           115                 120                 125
   Arg Ala Val Thr Ala Ala Lys Lys Ala Gly Leu Pro Val Leu Ala Glu
       130                 135                 140
   Ser Thr Pro Ser Lys Asn Ile Asp Glu Ile Val Lys Ser Ala Glu Gly
   145                 150                 155                 160
   Gln Thr Tyr Pro Ile Phe Val Lys Ala Val Ala Gly Gly Gly Gly Arg
                   165                 170                 175
   Gly Met Arg Phe Val Ala Ser Pro Asp Glu Leu Arg Lys Leu Ala Thr
               180                 185                 190
   Glu Ala Ser Arg Glu Ala Glu Ala Ala Phe Gly Asp Gly Ala Val Tyr
           195                 200                 205
   Val Glu Arg Ala Val Ile Asn Pro Gln His Ile Glu Val Gln Ile Leu
       210                 215                 220
   Gly Asp His Thr Gly Glu Val Val His Leu Tyr Glu Arg Asp Cys Ser
   225                 230                 235                 240
   Leu Gln Arg Arg His Gln Lys Val Val Glu Ile Ala Pro Ala Gln His
                   245                 250                 255
   Leu Asp Pro Glu Leu Arg Asp Arg Ile Cys Ala Asp Ala Val Lys Phe
               260                 265                 270
   Cys Arg Ser Ile Gly Tyr Gln Gly Ala Gly Thr Val Glu Phe Leu Val
           275                 280                 285
   Asp Glu Lys Gly Asn His Val Phe Ile Glu Met Asn Pro Arg Ile Gln
       290                 295                 300
   Val Glu His Thr Val Thr Glu Glu Val Thr Glu Val Asp Leu Val Lys
   305                 310                 315                 320
   Ala Gln Met Arg Leu Ala Ala Gly Ala Thr Leu Lys Glu Leu Gly Leu
                   325                 330                 335
   Thr Gln Asp Lys Ile Lys Thr His Gly Ala Ala Leu Gln Cys Arg Ile
               340                 345                 350
   Thr Thr Glu Asp Pro Asn Asn Gly Phe Arg Pro Asp Thr Gly Thr Ile
           355                 360                 365
   Thr Ala Tyr Arg Ser Pro Gly Gly Ala Gly Val Arg Leu Asp Gly Ala
       370                 375                 380
   Ala Gln Leu Gly Gly Glu Ile Thr Ala His Phe Asp Ser Met Leu Val
   385                 390                 395                 400
   Lys Met Thr Cys Arg Gly Ser Asp Phe Glu Thr Ala Val Ala Arg Ala
                   405                 410                 415
   Gln Arg Ala Leu Ala Glu Phe Thr Val Ser Gly Val Ala Thr Asn Ile
               420                 425                 430
   Gly Phe Leu Arg Ala Leu Leu Arg Glu Glu Asp Phe Thr Ser Lys Arg
                   435                 440                 445
   Ile Ala Thr Gly Phe Ile Ala Asp His Pro His Leu Leu Gln Ala Pro
               450                 455                 460
   Pro Ala Asp Asp Glu Gln Gly Arg Ile Leu Asp Tyr Leu Ala Asp Val
   465                 470                 475                 480
   Thr Val Asn Lys Pro His Gly Val Arg Pro Lys Asp Val Ala Ala Pro
                   485                 490                 495
   Ile Asp Lys Leu Pro Asn Ile Lys Asp Leu Pro Leu Pro Arg Gly Ser
               500                 505                 510
   Arg Asp Arg Leu Lys Gln Leu Gly Pro Ala Ala Phe Ala Arg Asp Leu
           515                 520                 525
   Arg Glu Gln Asp Ala Leu Ala Val Thr Asp Thr Thr Phe Arg Asp Ala
       530                 535                 540
   His Gln Ser Leu Leu Ala Thr Arg Val Arg Ser Phe Ala Leu Lys Pro
   545                 550                 555                 560
   Ala Ala Glu Ala Val Ala Lys Leu Thr Pro Glu Leu Leu Ser Val Glu
                   565                 570                 575
   Ala Trp Gly Gly Ala Thr Tyr Asp Val Ala Met Arg Phe Leu Phe Glu
               580                 585                 590
   Asp Pro Trp Asp Arg Leu Asp Glu Leu Arg Glu Ala Met Pro Asn Val
           595                 600                 605
   Asn Ile Gln Met Leu Leu Arg Gly Arg Asn Thr Val Gly Tyr Thr Pro
       610                 615                 620
   Tyr Pro Asp Ser Val Cys Arg Ala Phe Val Lys Glu Ala Ala Ser Ser
   625                 630                 635                 640
   Gly Val Asp Ile Phe Arg Ile Phe Asp Ala Leu Asn Asp Val Ser Gln
                   645                 650                 655
   Met Arg Pro Ala Ile Asp Ala Val Leu Glu Thr Asn Thr Ala Val Ala
               660                 665                 670
   Glu Val Ala Met Ala Tyr Ser Gly Asp Leu Ser Asp Pro Asn Glu Lys
           675                 680                 685
   Leu Tyr Thr Leu Asp Tyr Tyr Leu Lys Met Ala Glu Glu Ile Val Lys
       690                 695                 700
   Ser Gly Ala His Ile Leu Ala Ile Lys Asp Met Ala Gly Leu Leu Arg
   705                 710                 715                 720
   Pro Ala Ala Val Thr Lys Leu Val Thr Ala Leu Arg Arg Glu Phe Asp
                   725                 730                 735
   Leu Pro Val His Val His Thr His Asp Thr Ala Gly Gly Gln Leu Ala
               740                 745                 750
   Thr Tyr Phe Ala Ala Ala Gln Ala Gly Ala Asp Ala Val Asp Gly Ala
           755                 760                 765
   Ser Ala Pro Leu Ser Gly Thr Thr Ser Gln Pro Ser Leu Ser Ala Ile
       770                 775                 780
   Val Ala Ala Phe Ala His Thr Arg Arg Asp Thr Gly Leu Ser Leu Glu
   785                 790                 795                 800
   Ala Val Ser Asp Leu Glu Pro Tyr Trp Glu Ala Val Arg Gly Leu Tyr
                   805                 810                 815
   Leu Pro Phe Glu Ser Gly Thr Pro Gly Pro Thr Gly Arg Val Tyr Arg
               820                 825                 830
   His Glu Ile Pro Gly Gly Gln Leu Ser Asn Leu Arg Ala Gln Ala Thr
           835                 840                 845
   Ala Leu Gly Leu Ala Asp Arg Phe Glu Leu Ile Glu Asp Asn Tyr Ala
       850                 855                 860
   Ala Val Asn Glu Met Leu Gly Arg Pro Thr Lys Val Thr Pro Ser Ser
   865                 870                 875                 880
   Lys Val Val Gly Asp Leu Ala Leu His Leu Val Gly Ala Gly Val Asp
                   885                 890                 895
   Pro Ala Asp Phe Ala Ala Asp Pro Gln Lys Tyr Asp Ile Pro Asp Ser
               900                 905                 910
   Val Ile Ala Phe Leu Arg Gly Glu Leu Gly Asn Pro Pro Gly Gly Trp
           915                 920                 925
   Pro Glu Pro Leu Arg Thr Arg Ala Leu Glu Gly Arg Ser Glu Gly Lys
       930                 935                 940
   Ala Pro Leu Thr Glu Val Pro Glu Glu Glu Gln Ala His Leu Asp Ala
   945                 950                 955                 960
   Asp Asp Ser Lys Glu Arg Arg Asn Ser Leu Asn Arg Leu Leu Phe Pro
                   965                 970                 975
   Lys Pro Thr Glu Glu Phe Leu Glu His Arg Arg Arg Phe Gly Asn Thr
               980                 985                 990
   Ser Ala Leu Asp Asp Arg Glu Phe Phe Tyr Gly Leu Val Glu Gly Arg
           995                 1000                1005
   Glu Thr Leu Ile Arg Leu Pro Asp Val Arg Thr Pro Leu Leu Val
       1010                1015                1020
   Arg Leu Asp Ala Ile Ser Glu Pro Asp Asp Lys Gly Met Arg Asn
       1025                1030                1035
   Val Val Ala Asn Val Asn Gly Gln Ile Arg Pro Met Arg Val Arg
       1040                1045                1050
   Asp Arg Ser Val Glu Ser Val Thr Ala Thr Ala Glu Lys Ala Asp
       1055                1060                1065
   Ser Ser Asn Lys Gly His Val Ala Ala Pro Phe Ala Gly Val Val
       1070                1075                1080
   Thr Val Thr Val Ala Glu Gly Asp Glu Val Lys Ala Gly Asp Ala
       1085                1090                1095
   Val Ala Ile Ile Glu Ala Met Lys Met Glu Ala Thr Ile Thr Ala
       1100                1105                1110
   Ser Val Asp Gly Lys Ile Asp Arg Val Val Val Pro Ala Ala Thr
       1115                1120                1125
   Lys Val Glu Gly Gly Asp Leu Ile Val Val Val Ser
       1130                1135                1140